(19)

**Europäisches Patentamt**
**European Patent Office**
**Office européen des brevets**

(11) **EP 4 553 148 A1**

(12) **EUROPEAN PATENT APPLICATION**
published in accordance with Art. 153(4) EPC

(43) Date of publication:
**14.05.2025 Bulletin 2025/20**

(21) Application number: **23835551.5**

(22) Date of filing: **05.07.2023**

(51) International Patent Classification (IPC):
*C12N 5/071* (2010.01)        *C12N 5/077* (2010.01)
*C12Q 1/02* (2006.01)        *C12Q 1/06* (2006.01)
*C12M 1/00* (2006.01)

(52) Cooperative Patent Classification (CPC):
**C12M 1/00; C12N 5/06; C12Q 1/02; C12Q 1/06**

(86) International application number:
**PCT/JP2023/024866**

(87) International publication number:
**WO 2024/010020 (11.01.2024 Gazette 2024/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.07.2022 JP 2022110334**
              **14.03.2023 JP 2023040170**

(71) Applicant: **Oji Holdings Corporation**
**Chuo-ku**
**Tokyo 104-0061 (JP)**

(72) Inventors:
• **YATSUKA Yukiko**
  **Tokyo 113-8421 (JP)**
• **SAOTOME Hideo**
  **Tokyo 113-8421 (JP)**

• **MINOWA Osamu**
  **Tokyo 113-8421 (JP)**
• **TSUCHIDA Katsuharu**
  **Tokyo 113-8421 (JP)**
• **OKAZAKI Yasushi**
  **Tokyo 113-8421 (JP)**
• **TOKUNO Hisako**
  **Tokyo 104-0061 (JP)**
• **DAI Kotaro**
  **Tokyo 104-0061 (JP)**
• **SHINOTSUKA Kei**
  **Tokyo 104-0061 (JP)**

(74) Representative: **Godemeyer Blum Lenze**
**Patentanwälte**
**Partnerschaft mbB - werkpatent**
**An den Gärten 7**
**51491 Overath (DE)**

(54) **CULTURED CELL SHEET AND METHOD FOR PRODUCING SAME; METHOD FOR EVALUATING COMPOUND OR DRUG; AND METHOD FOR EVALUATING QUALITY OF CULTURED CELL SHEET**

(57)    An object of the present invention is to provide a matured cultured cell sheet comprising cardiomyocytes without using a specific drug and a method for producing the same. Another object of the present invention is to provide a method for evaluating a compound or drug using the cultured cell sheet and a method for evaluating the quality of the cultured cell sheet comprising cardiomyocytes. The cultured cell sheet of the present invention is a cultured cell sheet comprising cardiomyocytes, wherein the cardiomyocytes are arranged with alignment, and a degree of alignment determined according to the following formula (1) is 23% or more: degree of alignment (%) = (number of rod-shaped structures included within $\pm$ 15° of mode)/(total number of rod-shaped structures) $\times$ 100 (1), wherein in an image obtained by microscopy of the cultured cell sheet immunostained with an anti-$\alpha$-actinin antibody, where a screen horizontal direction is regarded as 0°, a degree of alignment is a frequency of rod-shaped structures included within $\pm15$° of the mode of angles which are measured in a longitudinal direction of the rod-shaped structures detected with an $\alpha$-actinin antibody within a measurement range of 71.6 $\mu$m $\times$ 71.6 $\mu$m.

EP 4 553 148 A1

[Fig. 1]

**Description**

Technical Field

The present invention relates to a cultured cell sheet composed of cardiomyocytes and a method for producing the same, a method for evaluating a compound or drug using the cultured cell sheet, and a method for evaluating the quality of the cultured cell sheet.

Background Art

From the viewpoint of reducing cost and time required for drug development and providing an alternative to animal experiments, cells induced to differentiate from stem cells, such as human iPS cells, are expected to be used for drug discovery. It is possible to conduct studies targeting these human cells from the initial stage of development, and the advantage is that studies conventionally conducted in animal experiments can be replaced by studies using stem cell-derived cells such as human iPS cells, eliminating differences in drug responsiveness due to species differences. In particular, there is a demand for the use of cardiomyocytes derived from stem cells such as human iPS cells in cardiomyocytes and the like, which are difficult to obtain from a living body.

However, currently available cardiomyocytes derived from stem cells such as human iPS cells are found to be immature, with physiological activities and motor functions close to those of fetal heart muscle. Therefore, it has been reported that there is also a discrepancy from the living body in terms of responsiveness to a drug, and a method for maturing cardiomyocytes derived from stem cells such as human iPS cells is required.

For example, Patent Literature 1 discloses an aqueous medium composition that is serum-free, the aqueous medium composition containing a thyroid hormone-like compound, a lipid mixture, and a carnitine compound, with the aim of overcoming a major limitation of the art by providing a chemically-defined culture medium composition that promotes maturation of fetal-like (immature) cardiomyocytes, which are themselves derived from pluripotent embryonic stem cell in *in vitro* culture, and a method for generating adult-like cardiomyocytes *in vitro* culture, which are more efficient and support large scale production of adult-like cardiomyocytes satisfying general requirements for cardiac research and clinical applications.

Citation List

Patent Literature

Patent Literature 1: JP 2020-022460 A

Summary of Invention

Technical Problem

In Patent Literature 1, adult-like cardiomyocytes are matured by using a specific medium, and the possibility that components contained in the medium have an influence on cardiomyocytes other than maturation cannot be denied.

An object of the present invention is to provide a matured cultured cell sheet comprising cardiomyocytes without using a specific drug and a method for producing the same. Another object of the present invention is to provide a method for evaluating a compound or drug (protein, peptide, antibody, DNA, RNA, etc.) using the cultured cell sheet. A further object of the present invention is to provide a method for evaluating the quality of a cultured cell sheet comprising cardiomyocytes.

Solution to Problem

The objects of the present invention can be attained by configurations <1> to <19> below.

<1> A cultured cell sheet comprising cardiomyocytes, wherein the cardiomyocytes are arranged with alignment, and a degree of alignment determined according to the following formula (1) is 23% or more:

Degree of alignment (%) = (number of rod-shaped structures included within $\pm 15°$ of mode)/(total number of rod-shaped structures) $\times$ 100     (1)

wherein in an image obtained by microscopy of the cultured cell sheet immunostained with an anti-$\alpha$-actinin antibody,

where a screen horizontal direction is regarded as 0°, the degree of alignment is a frequency of rod-shaped structures included within ±15° of a mode of angles which are measured in a longitudinal direction of the rod-shaped structures detected with an α-actinin antibody within a measurement range of 71.6 μm × 71.6 μm.

<2> The cultured cell sheet according to <1>, wherein in live cell imaging, number of vectors (degree of alignment) showing an angle of ±5° from an angle of a mode of motion vectors is 12% or more.

<3> The cultured cell sheet according to <1> or <2>, wherein the cardiomyocytes are stem cell-derived cardiomyocytes.

<4> The cultured cell sheet according to any one of <1> to <3>, which satisfies at least one of the following requirements A1 and A2:

Requirement A1: a ratio of an expression level of myosin light chain 3 (MYL3) gene to an expression level of actin beta (ACTB) gene, MYL3/ACTB, is 12.0 or more; and
Requirement A2: a ratio of an expression level of a myosin heavy chain 7 (MYH7) gene to an expression level of a myosin heavy chain 6 (MYH6) gene, MYH7/MYH6, is 6.0 or more.

<5> The cultured cell sheet according to any one of <1> to <4>, which satisfies at least one of the following requirements B1 and B2:

Requirement B1: a ratio of an expression level of creatine kinase M-type (CKM) gene to an expression level of actin beta (ACTB) gene, CKM/ACTB, is 1.80 or more; and
Requirement B2: a ratio of an expression level of a lactate dehydrogenase A subunit (LDHA) gene to an expression level of ACTB gene, LDHA/ACTB, is 0.80 or more.

<6> The cultured cell sheet according to any one of <1> to <5>, which satisfies at least one of the following requirements C1 to C4:

Requirement C1: a ratio of an expression level of calcium voltage-gated channel auxiliary subunit alpha2delta1 (CACNA2D1) gene to an expression level of ATPase Na+/K+ transporting subunit alpha 1 (ATP1A1) gene, CACNA2D1/ATP1A1, is 0.085 or more on any of days 15 to 30 of culture;
Requirement C2: a ratio of an expression level of potassium inwardly rectifying channel subfamily J member 2 (KCNJ2) gene to an expression level of ATPase Na+/K+ transporting subunit alpha 1 (ATP1A1) gene, KCNJ2/ATP1A1, is 0.038 or more on any of days 15 to 45 of culture;
Requirement C3: a ratio of an expression level of potassium voltage-gated channel subfamily E regulatory subunit 1 (KCNE1) gene to an expression level of ATPase Na+/K+ transporting subunit alpha 1 (ATP1A1) gene, KCNE1/ATP1A1, is 0.003 or more on any of days 15 to 45 of culture; and
Requirement C4: a ratio of an expression level of sodium voltage-gated channel alpha subunit 5 (SCN5A) gene to an expression level of ATPase Na+/K+ transporting subunit alpha 1 (ATP1A1) gene, SCN5A/ATP1A1, is 0.87 or more on any of days 15 to 45 of culture.

<7> The cultured cell sheet according to any one of <1> to <6>, which satisfies at least one of the following requirements D1 to D4:

Requirement D1: a ratio of an expression level of lipoprotein lipase (LPL) gene to an expression level of actin beta (ACTB) gene, LPL/ACTB, is 0.45 or more on any of days 15 to 45 of culture;
Requirement D2: a ratio of an expression level of acetyl-CoA acetyltransferase 1 (ACAT1) gene to an expression level of actin beta (ACTB) gene, ACAT1/ACTB, is 0.43 or more on any of days 15 to 30 of culture;
Requirement D3: a ratio of an expression level of hydroxyacyl-CoA dehydrogenase trifunctional multienzyme complex subunit alpha (HADHA) gene to an expression level of actin beta (ACTB) gene, HADHA/ACTB, is 0.75 or more on any of days 15 to 30 of culture; and
Requirement D4: a ratio of an expression level of hydroxyacyl-CoA dehydrogenase trifunctional multienzyme complex subunit beta (HADHB) gene to an expression level of actin beta (ACTB) gene, HADHB/ACTB, is 0.95 or more on any of days 15 to 30 of culture.

<8> The cultured cell sheet according to any one of <1> to <7>, which satisfies at least one of the following requirements E1 to E8:

Requirement E1: a ratio of an expression level of PPARG (peroxisome proliferator-activated receptor gamma) coactivator 1 alpha) (PPARGC1A) gene to an expression level of hypoxia inducible factor 1 alpha (HIF1A) gene, PPARGC1A/HIF1A, is 0.80 or more on any day of culture;

Requirement E2: a ratio of an expression level of estrogen related receptor alpha (ESRRA) gene to an expression level of hypoxia inducible factor 1 alpha (HIF1A) gene, ESRRA/HIF1A, is 0.55 or more on any day of culture;

Requirement E3: a ratio of an expression level of vascular endothelial growth factor A (VEGFA) gene to an expression level of hypoxia inducible factor 1 alpha (HIF1A) gene, VEGFA/HIF1A, is 0.65 or more on any day of culture;

Requirement E4: a ratio of an expression level of apelin (APLN) gene to an expression level of hypoxia inducible factor 1 alpha (HIF1A) gene, APLN/HIF1A, is 0.005 or more on any day of culture;

Requirement E5: a ratio of an expression level of fatty acid binding protein 3 (FABP3) gene to an expression level of hypoxia inducible factor 1 alpha (HIF1A) gene, FABP3/HIF1A, is 5.0 or more on any day of culture;

Requirement E6: a ratio of an expression level of endothelial cell specific molecule 1 (ESM1) gene to an expression level of hypoxia inducible factor 1 alpha (HIF1A) gene, ESM1/HIF1A, is 0.002 or more on any day of culture;

Requirement E7: a ratio of an expression level of endomucin (EMCN) gene to an expression level of hypoxia inducible factor 1 alpha (HIF1A) gene, EMCN/HIF1A, is 0.002 or more on any day after day 7 of culture; and

Requirement E8: a ratio of an expression level of BCL2 apoptosis regulator (BCL2) gene to an expression level of hypoxia inducible factor 1 alpha (HIF1A) gene, BCL2/HIF1A, is 0.013 or more on any day after day 7 of culture.

<9> The cultured cell sheet according to any one of <1> to <8>, wherein an area of mitochondria per cell is 200 $\mu m^2$ or more.

<10> The cultured cell sheet according to any one of <1> to <9>, which satisfies the following formula (2):

$$1.0 \leq CV/RV \leq 2.8 \qquad (2)$$

wherein CV (m/sec) and RV (m/sec) denote contractile velocity and relaxation velocity, respectively, detected in live cell imaging.

<11> The cultured cell sheet according to any one of <1> to <10>, which satisfies the following formula (3):

$$\text{Duration}/(\text{Interval})^{1/2} \geq 0.68 \qquad (3)$$

wherein in calcium imaging analysis, when a waveform peak height of a calcium transient is 100%, Duration (sec) denotes a waveform width at a height of 20%, and Interval (sec) denotes an interval between peaks.

<12> The cultured cell sheet according to any one of <1> to <11>, wherein a cell constituting the cultured cell sheet has an action potential duration at 80% repolarization of 600 msec or more and a maximum diastolic potential of -60 mV or less in a patch-clamp test in a solution at 25°C.

<13> The cultured cell sheet according to any one of <1> to <12>, wherein in an oxygen consumption rate measurement for measuring a rate at which a cell uses oxygen contained in a culture solution, an addition of a β-oxidation inhibitor that inhibits β-oxidation activity of a fatty acid decreases an oxygen consumption rate to 20% or less as compared with that before the addition of the β-oxidation inhibitor.

<14> The cultured cell sheet according to any one of <1> to <13>, which is for regenerative medicine.

<15> A method for producing the cultured cell sheet according to any one of <1> to <14>, comprising: culturing cardiomyocytes by using a cell sheet-forming member comprising a surface for forming a cultured cell sheet, wherein the surface comprises a plurality of flat portions and a plurality of recession and protrusion portions; each of the flat portions has a shape extending in a first direction, and a plurality of the flat portions are arrayed in a second direction intersecting the first direction over an entirety of the surface; each of the recession and protrusion portions comprises a plurality of stepped structures filling a gap between the flat portions adjacent to each other, and a pitch of the stepped structures is 100 nm or more and 10 $\mu m$ or less; the stepped structures each comprise a protrusion portion; the recession and protrusion portions each comprise a plurality of the protrusion portions in a bottom surface of a recession portion sandwiched between the flat portions adjacent to each other; and a difference between a height of a distal end surface of each of the recession and protrusion portions and a height of each of the flat portions is 0.5 $\mu m$ or less in a thickness direction of the cell sheet-forming member.

<16> A method for evaluating a compound or drug, comprising allowing a compound or drug to be evaluated to act on the cultured cell sheet according to any one of <1> to <14>.

<17> The method for evaluating a compound or drug according to <16>, comprising evaluating at least one change selected from the group consisting of a change in a physiological property and a change in motor function of the cultured cell sheet.

<18> The method for evaluating a compound or drug according to <16> or <17>, wherein the compound or drug to be evaluated is at least one existing compound selected from the group consisting of an INa blocker, an Ikr blocker, an Iks blocker, an Ica blocker, a 5-HT4 receptor agonist, an alpha-receptor blocker, a beta-receptor blocker, an alpha-receptor agonist, a beta-receptor agonist, a toxic deleterious substance, an anti-cancer drug, a lipid metabolic inhibitor, and another bioactive substance, or a candidate compound thereof.

<19> A method for evaluating quality of a cultured cell sheet comprising cardiomyocytes, comprising: evaluating any one of the following (i) to (xii):

(i) a degree of alignment determined according to the following formula (1) is 23% or more:

$$\text{Degree of alignment (\%)} = \text{(number of rod-shaped structures included within} \pm 15° \text{ of mode)/(total number of rod-shaped structures)} \times 100 \tag{1}$$

wherein in an image obtained by microscopy of the cultured cell sheet immunostained with an anti-$\alpha$-actinin antibody, where a screen horizontal direction is regarded as 0°, the degree of alignment is a frequency of rod-shaped structures included within $\pm 15°$ of the mode of angles which are measured in a longitudinal direction of the rod-shaped structures detected with an $\alpha$-actinin antibody within a measurement range of 71.6 $\mu$m $\times$ 71.6 $\mu$m,

(ii) the number of vectors (degree of alignment) showing an angle of $\pm 5°$ from an angle of a mode of motion vectors is 12% or more in live cell imaging,

(iii) the cultured cell sheet satisfies at least one of the following requirements A1 and A2:

Requirement A1: a ratio of an expression level of myosin light chain 3 (MYL3) gene to an expression level of actin beta (ACTB) gene, MYL3/ACTB, is 12.0 or more; and

Requirement A2: a ratio of an expression level of a myosin heavy chain 7 (MYH7) gene to an expression level of a myosin heavy chain 6 (MYH6) gene, MYH7/MYH6, is 6.0 or more,

(iv) the cultured cell sheet satisfies at least one of the following requirements B1 and B2:

Requirement B1: a ratio of an expression level of creatine kinase M-type (CKM) gene to an expression level of actin beta (ACTB) gene, CKM/ACTB, is 1.80 or more; and

Requirement B2: a ratio of an expression level of a lactate dehydrogenase A subunit (LDHA) gene to an expression level of ACTB gene, LDHA/ACTB, is 0.80 or more,

(v) the cultured cell sheet satisfies at least one of the following requirements C1 to C4:

Requirement C1: a ratio of an expression level of calcium voltage-gated channel auxiliary subunit alpha2-delta1 (CACNA2D1) gene to an expression level of ATPase Na+/K+ transporting subunit alpha 1 (ATP1A1) gene, CACNA2D1/ATP1A1, is 0.085 or more on any of days 15 to 30 of culture;

Requirement C2: a ratio of an expression level of potassium inwardly rectifying channel subfamily J member 2 (KCNJ2) gene to an expression level of ATPase Na+/K+ transporting subunit alpha 1 (ATP1A1) gene, KCNJ2/ATP1A1, is 0.038 or more on any of days 15 to 45 of culture;

Requirement C3: a ratio of an expression level of potassium voltage-gated channel subfamily E regulatory subunit 1 (KCNE1) gene to an expression level of ATPase Na+/K+ transporting subunit alpha 1 (ATP1A1) gene, KCNE1/ATP1A1, is 0.003 or more on any of days 15 to 45 of culture; and

Requirement C4: a ratio of an expression level of sodium voltage-gated channel alpha subunit 5 (SCN5A) gene to an expression level of ATPase Na+/K+ transporting subunit alpha 1 (ATP1A1) gene, SCN5A/ATP1A1, is 0.87 or more on any of days 15 to 45 of culture,

(vi) the cultured cell sheet satisfies at least one of the following requirements D1 to D4:

Requirement D1: a ratio of an expression level of lipoprotein lipase (LPL) gene to an expression level of actin beta (ACTB) gene, LPL/ACTB, is 0.45 or more on any of days 15 to 45 of culture;

Requirement D2: a ratio of an expression level of acetyl-CoA acetyltransferase 1 (ACAT1) gene to an expression level of actin beta (ACTB) gene, ACAT1/ACTB, is 0.43 or more on any of days 15 to 30 of culture;

Requirement D3: a ratio of an expression level of hydroxyacyl-CoA dehydrogenase trifunctional multienzyme complex subunit alpha (HADHA) gene to an expression level of actin beta (ACTB) gene, HADHA/ACTB, is 0.75 or more on any of days 15 to 30 of culture; and

Requirement D4: a ratio of an expression level of hydroxyacyl-CoA dehydrogenase trifunctional multienzyme complex subunit beta (HADHB) gene to an expression level of actin beta (ACTB) gene, HADH-

**EP 4 553 148 A1**

B/ACTB, is 0.95 or more on any of days 15 to 30 of culture,

(vii) the cultured cell sheet satisfies at least one of the following requirements E1 to E8:

Requirement E1: a ratio of an expression level of PPARG (peroxisome proliferator-activated receptor gamma) coactivator 1 alpha) (PPARGC1A) gene to an expression level of hypoxia inducible factor 1 alpha (HIF1A) gene, PPARGC1A/HIF1A, is 0.80 or more on any day of culture;
Requirement E2: a ratio of an expression level of estrogen related receptor alpha (ESRRA) gene to an expression level of hypoxia inducible factor 1 alpha (HIF1A) gene, ESRRA/HIF1A, is 0.55 or more on any day of culture;
Requirement E3: a ratio of an expression level of vascular endothelial growth factor A (VEGFA) gene to an expression level of hypoxia inducible factor 1 alpha (HIF1A) gene, VEGFA/HIF1A, is 0.65 or more on any day of culture;
Requirement E4: a ratio of an expression level of apelin (APLN) gene to an expression level of hypoxia inducible factor 1 alpha (HIF1A) gene, APLN/HIF1A, is 0.005 or more on any day of culture;
Requirement E5: a ratio of an expression level of fatty acid binding protein 3 (FABP3) gene to an expression level of hypoxia inducible factor 1 alpha (HIF1A) gene, FABP3/HIF1A, is 5.0 or more on any day of culture;
Requirement E6: a ratio of an expression level of endothelial cell specific molecule 1 (ESM1) gene to an expression level of hypoxia inducible factor 1 alpha (HIF1A) gene, ESM1/HIF1A, is 0.002 or more on any day of culture;
Requirement E7: a ratio of an expression level of endomucin (EMCN) gene to an expression level of hypoxia inducible factor 1 alpha (HIF1A) gene, EMCN/HIF1A, is 0.002 or more on any day after day 7 of culture; and
Requirement E8: a ratio of an expression level of BCL2 apoptosis regulator (BCL2) gene to an expression level of hypoxia inducible factor 1 alpha (HIF1A) gene, BCL2/HIF1A, is 0.013 or more on any day after day 7 of culture,

(viii) an area of mitochondria per cell is 200 $\mu m^2$ or more,
(ix) the cultured cell sheet satisfies the following formula (2):

$$1.0 \le CV/RV \le 2.8 \quad (2)$$

wherein CV (m/sec) and RV (m/sec) denote contractile velocity and relaxation velocity, respectively, detected in live cell imaging,
(x) the cultured cell sheet, which satisfies the following formula (3):

$$Duration/(Interval)^{1/2} \ge 0.68 \quad (3)$$

wherein in calcium imaging analysis, when a waveform peak height of a calcium transient is 100%, Duration (sec) denotes a waveform width at a height of 20%, and Interval (sec) denotes an interval between peaks,
(xi) a cell constituting the cultured cell sheet has an action potential duration at 80% repolarization of 600 msec or more and a maximum diastolic potential of -60 mV or less in a patch-clamp test in a solution at 25°C, and
<xii> in an oxygen consumption rate measurement for measuring a rate at which a cell uses oxygen contained in a culture solution, an addition of a β-oxidation inhibitor that inhibits β-oxidation activity of a fatty acid decreases an oxygen consumption rate to 20% or less as compared with that before the addition of the β-oxidation inhibitor.

Advantageous Effects of Invention

The present invention can provide a matured cultured cell sheet comprising cardiomyocytes without using a specific drug and a method for producing the same. The present invention can also provide a method for evaluating a compound or drug using the cultured cell sheet. Furthermore, the present invention can provide a method for evaluating the quality of a cultured cell sheet comprising cardiomyocytes.

Brief Description of Drawings

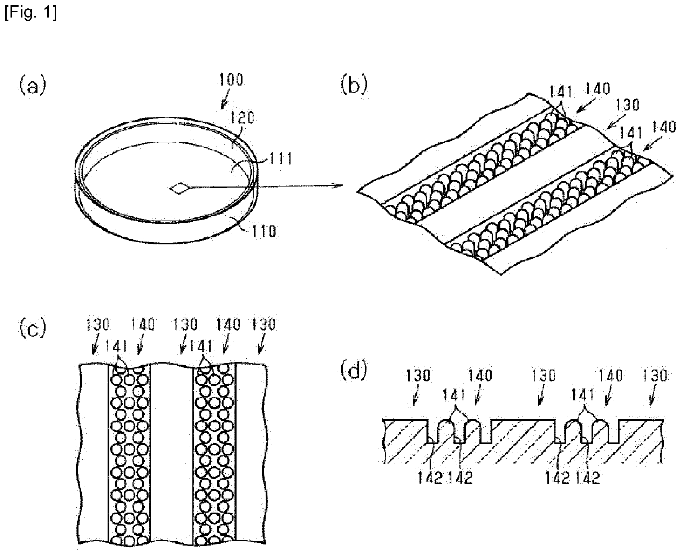

[Fig. 1] Fig. 1 is a diagram illustrating a configuration of a cell sheet-forming member in an embodiment of the cell

7

sheet-forming member, wherein (a) is a perspective view showing the structure of the cell sheet-forming member with a petri dish, (b) is a perspective view showing a part of the surface of the cell sheet-forming member enlarged, (c) is a plan view showing a part of the surface of the cell sheet-forming member enlarged, and (d) is a partial cross-sectional view showing a part of the cell sheet-forming member enlarged.

[Fig. 2] Fig. 2 is a process diagram to illustrate an example of a method for producing a cell sheet-forming member.

[Fig. 3] Figs. 3(a) to 3(c) are schematic diagrams to illustrate production processes of a cell sheet.

[Fig. 4] Fig. 4 is graphs showing a degree of alignment on day 15, day 30, day 45, and day 60 of the number of days in culture.

[Fig. 5] Fig. 5 is graphs showing a change over time in a ratio of an expression level of a myosin light chain 3 (MYL3) gene to an expression level of an actin beta (ACTB) gene, MYL3/ACTB.

[Fig. 6] Fig. 6 is graphs showing a change over time in a ratio of an expression level of a myosin heavy chain 7 (MYH7) gene to an expression level of a myosin heavy chain 6 (MYH6) gene, MYH7/MYH6.

[Fig. 7-1] Fig. 7-1 is graphs showing changes over time in expression level of a creatin kinase, M-type (CKM) gene, a cytochrome C oxidase subunit 6A2 (COX6A2) gene, a creatine kinase, mitochondrial 2 (CKMT2) gene, and a lactate dehydrogenase A subunit (LDHA) gene.

[Fig. 7-2] Fig. 7-2 is graphs showing changes over time in a ratio of the expression level of the CKM gene to the expression level of the actin beta (ACTB) gene, CKM/ACTB, a ratio of the expression level of the COX6A2 gene to the expression level of the ACTB gene, COX6A2/ACTB, a ratio of the expression level of the CKMT2 gene to the expression level of the ACTB gene, CKMT2/ACTB, and a ratio of the expression level of the LDHA gene to the expression level of the ACTB gene, LDHA/ACTB.

[Fig. 7-3] Fig. 7-3 is graphs showing changes over time in expression level of a calcium voltage-gated channel auxiliary subunit alpha2delta1 (CACNA2D1) gene, a potassium inwardly rectifying channel subfamily J member2 (KCNJ2) gene, a potassium voltage-gated channel subfamily E regulatory subunit 1 (KCNE1) gene, and a sodium voltage-gated channel alpha subunit 5 (SCN5A) gene.

[Fig. 7-4] Fig. 7-4 is graphs showing changes over time in a ratio of an expression level of the CACNA2D1 gene to an expression level of an ATPase Na+/K+ transporting subunit alpha 1 (ATP1A1) gene, CACNA2D1/ATP1A1, a ratio of an expression level of the KCNJ2 gene to the expression level of the ATP1A1 gene, KCNJ2/ATP1A1, a ratio of an expression level of the KCNE1 gene to the expression level of the ATP1A1 gene, KCNE1/ATP1A1, and a ratio of an expression level of the SCN5A gene to the expression level of the ATP1A1 gene, SCN5A/ATP1A1.

[Fig. 7-5] Fig. 7-5 is graphs showing changes over time in expression level of a lipoprotein lipase (LPL) gene, an acetyl-CoA acetyltransferase 1 (ACAT1) gene, a hydroxyacyl-CoA dehydrogenase trifunctional multienzyme complex subunit alpha (HADHA) gene, and a hydroxyacyl-CoA dehydrogenase trifunctional multienzyme complex subunit beta (HADHB) gene.

[Fig. 7-6] Fig. 7-6 is graphs showing changes over time in a ratio of an expression level of the LPL gene to an expression level of the actin beta (ACTB) gene, LPL/ACTB, a ratio of an expression level of the ACAT1 gene to the expression level of the ACTB gene, ACAT1/ACTB, a ratio of an expression level of the HADHA gene to the expression level of the ACTB gene, HADHA/ACTB, and a ratio of an expression level of the HADHB gene to the expression level of the ACTB gene, HADHB/ACTB.

[Fig. 7-7] Fig. 7-7 is graphs showing changes over time in expression level of a peroxisome proliferator-activated receptor gamma (PPARG) coactivator 1 alpha (PPARGC1A) gene, an estrogen related receptor alpha (ESRRA) gene, a vascular endothelial growth factor A (VEGFA) gene, and an apelin (APLN) gene.

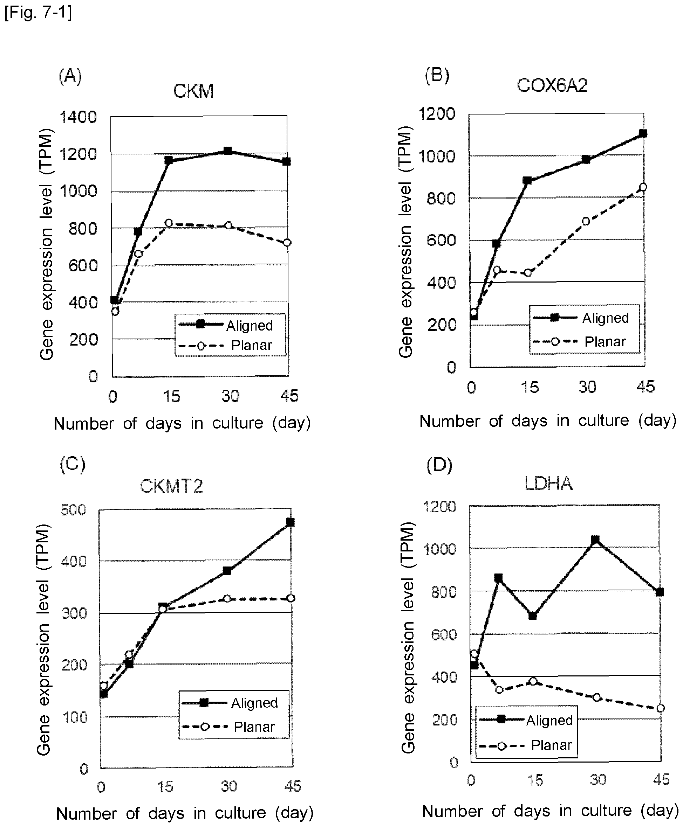

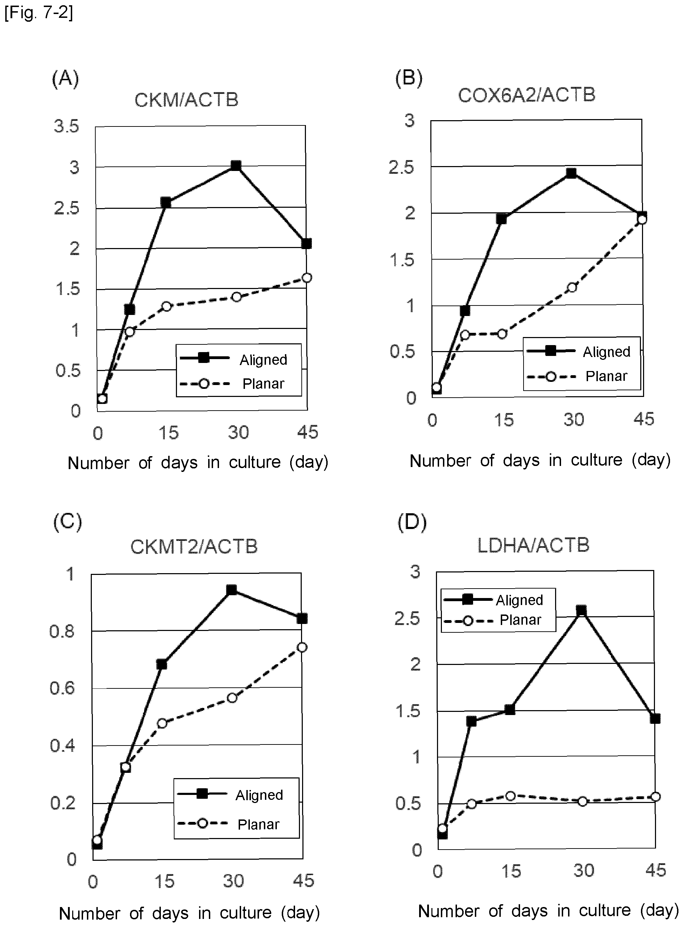

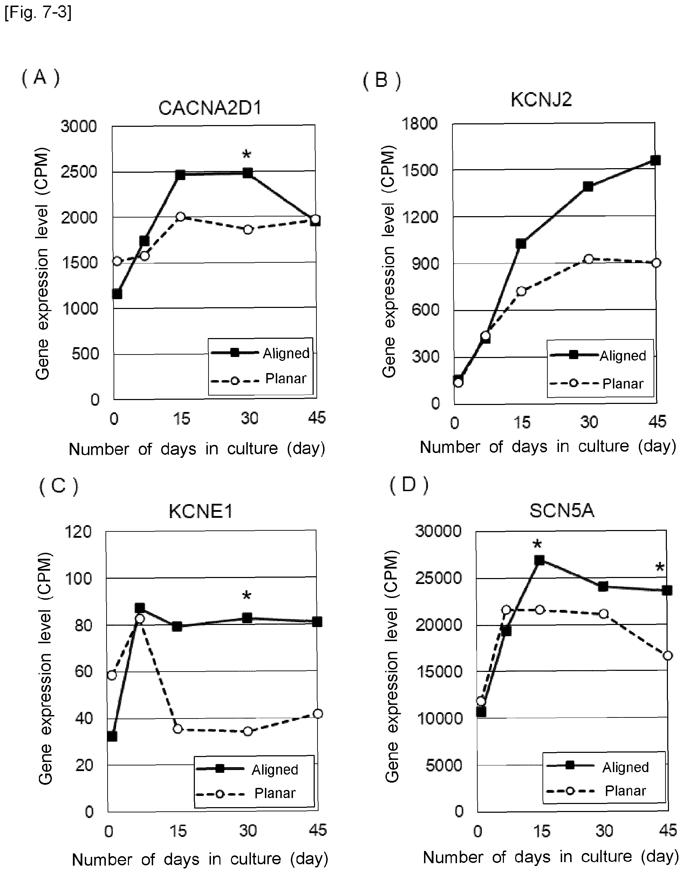

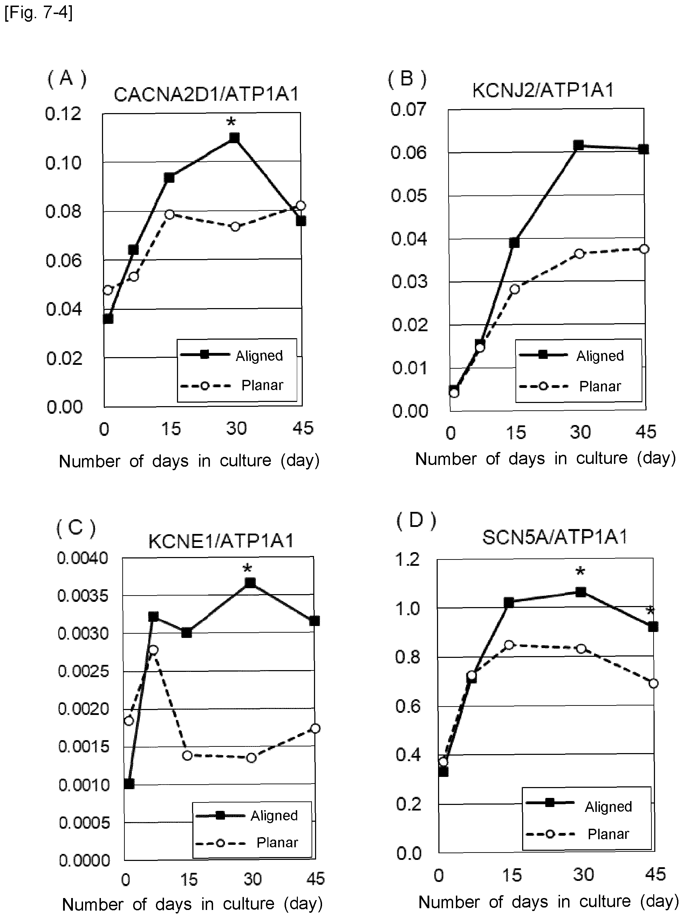

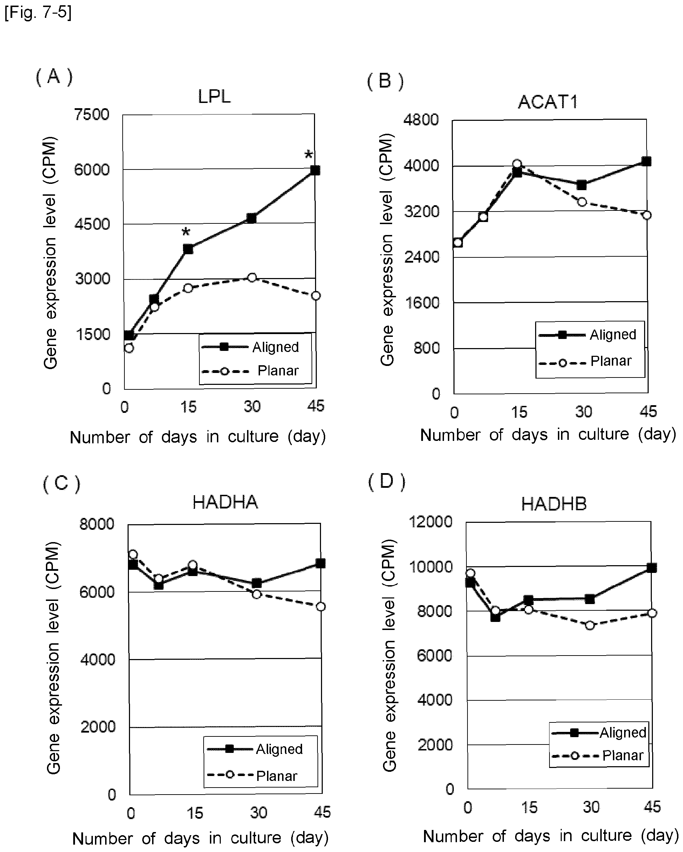

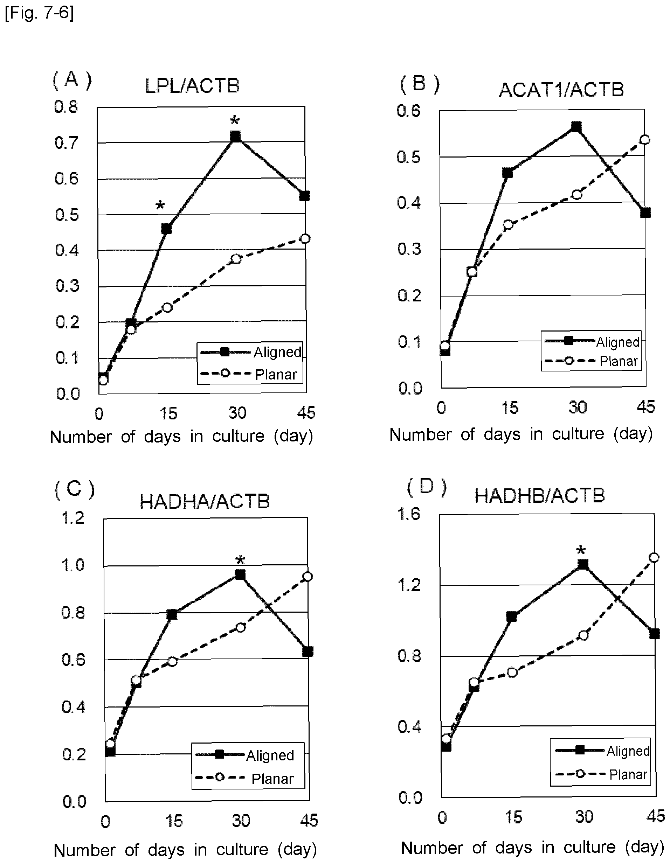

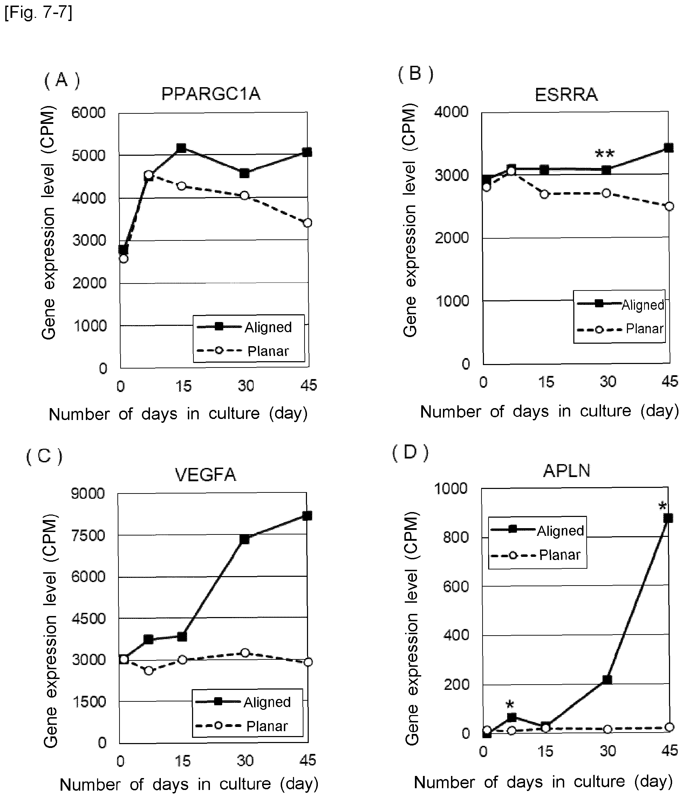

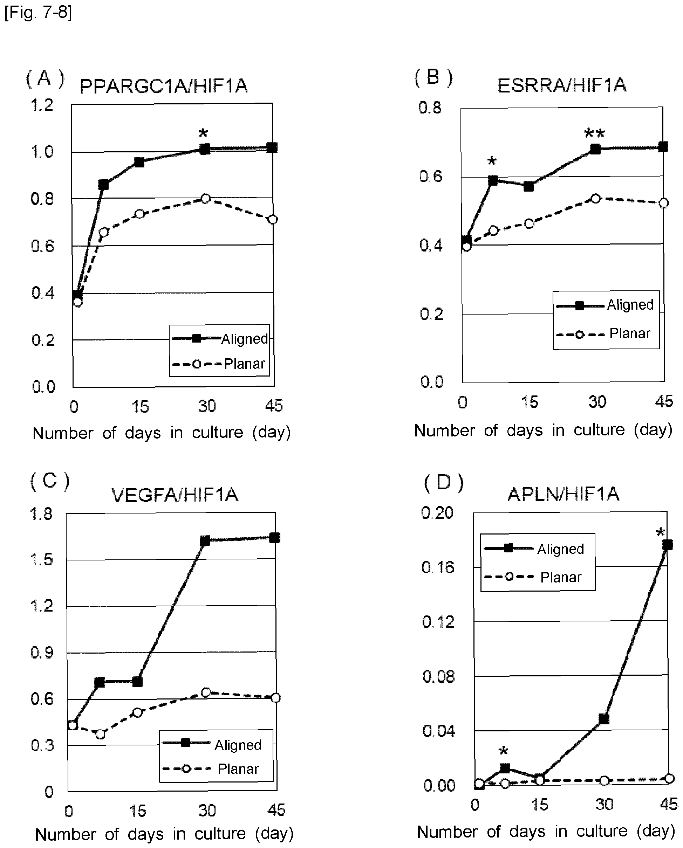

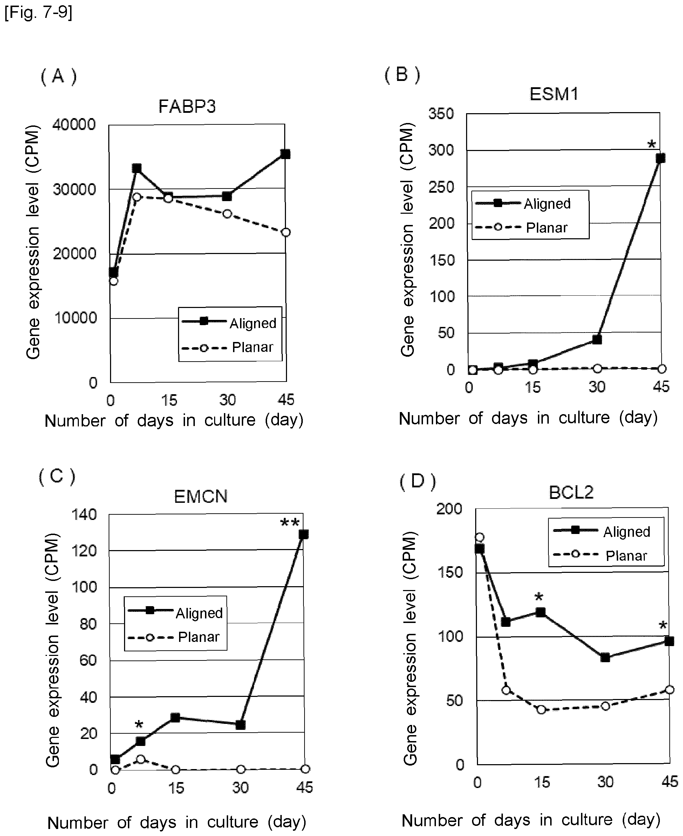

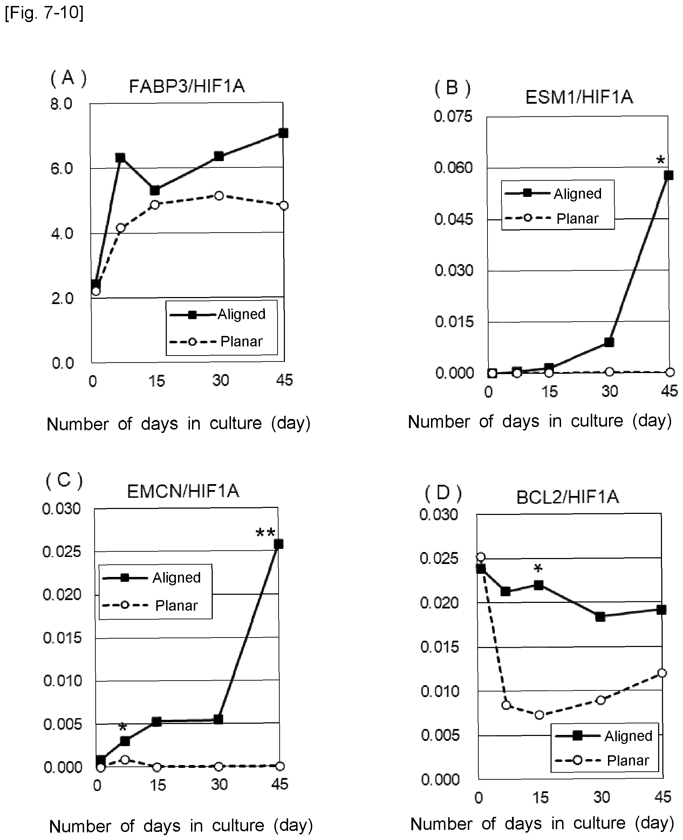

[Fig. 7-8] Fig. 7-8 is graphs showing changes over time in a ratio of an expression level of the PPARGC1A gene to an expression level of a hypoxia inducible factor 1 alpha (HIF1A) gene, PPARGC1A/HIF1A, a ratio of an expression level of the ESRRA gene to an expression level of the HIF1A gene, ESRRA/HIF1A, a ratio of an expression level of the VEGFA gene to an expression level of the HIF1A gene, VEGFA/HIF1A, and a ratio of the expression level of the APLN gene to the expression level of the HIF1A gene, APLN/HIF1A.

[Fig. 7-9] Fig. 7-9 is graphs showing changes over time in expression level of a fatty acid binding protein 3 (FABP3) gene, an endothelial cell specific molecule 1 (ESM1) gene, an endomucin (EMCN) gene, and a BCL2 apoptosis regulator (BCL2) gene.

[Fig. 7-10] Fig. 7-10 is graphs showing changes over time in a ratio of an expression level of the FABP3 gene to the expression level of the HIF1A gene, FABP3/HIF1A, a ratio of the expression level of the ESM1 gene to the expression level of the HIF1A gene, ESM1/HIF1A, a ratio of the expression level of the EMCN gene to the expression level of the HIF1A gene, EMCN/HIF1A, and a ratio of the expression level of the BCL2 gene to the expression level of the HIF1A gene, BCL2/HIF1A.

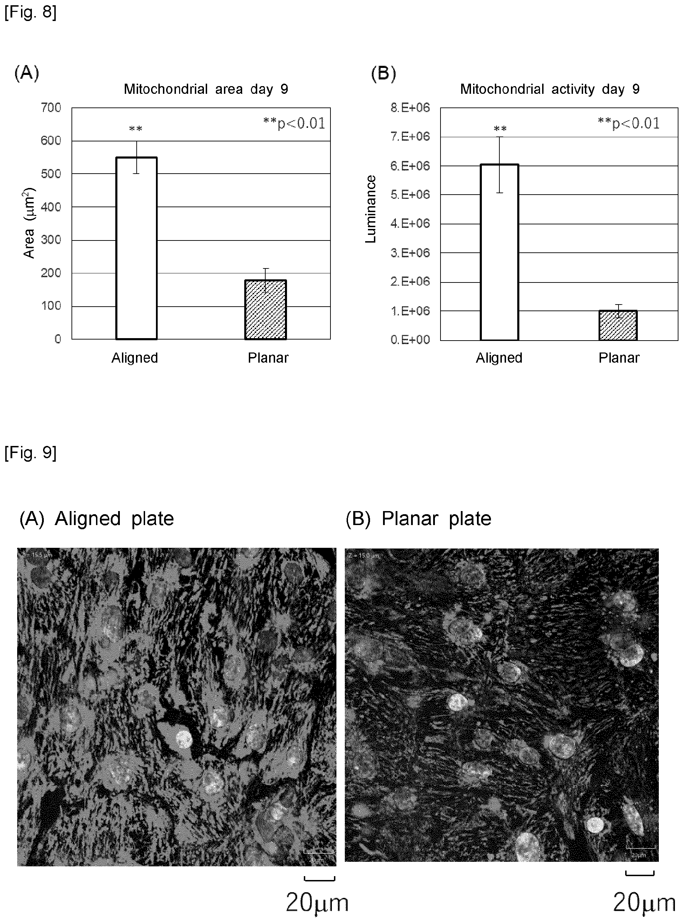

[Fig. 8] Fig. 8 is graphs showing a mitochondrial area and a mitochondrial activity per cell.

[Fig. 9] Fig. 9 is fluorescence images of stained nuclei and mitochondria.

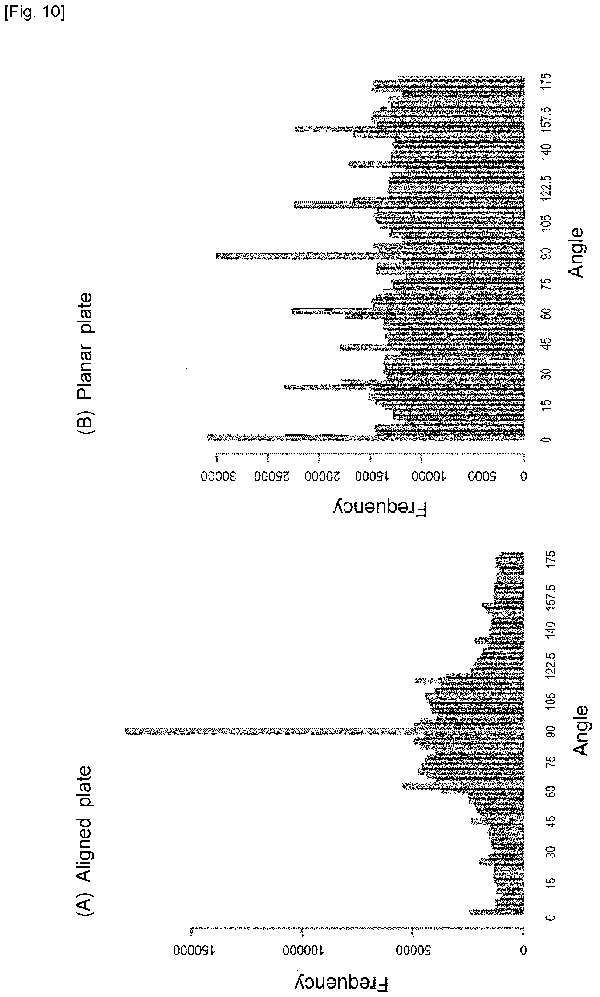

[Fig. 10] Fig. 10 is graphs showing frequency of angles indicated by motion vectors in a live cell imaging device.

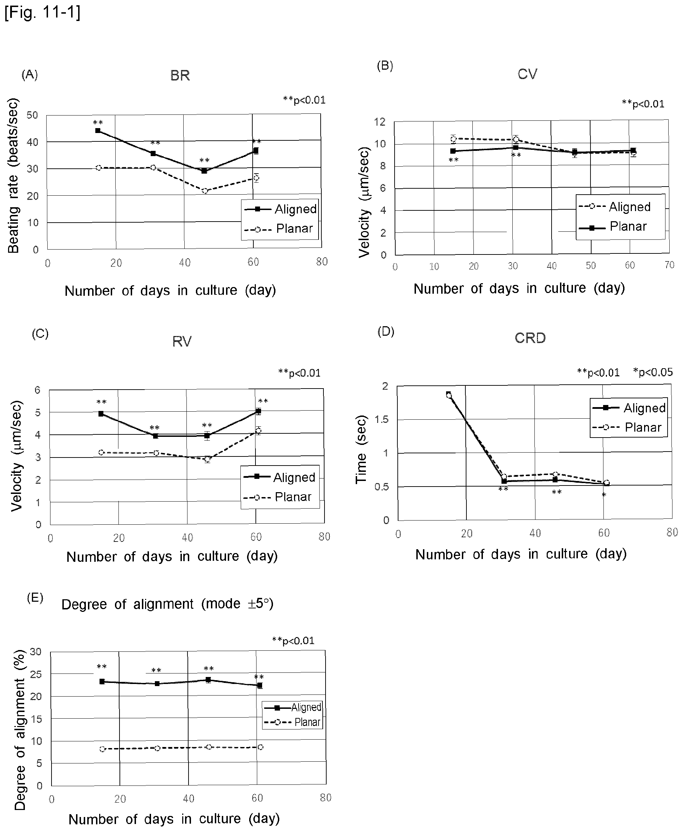

[Fig. 11-1] Fig. 11-1 is graphs showing changes over time in a beating rate (BR), a contractile velocity (CV), a relaxation velocity (RV), a contraction-relaxation duration (CRD), and a degree of alignment measured using the live cell imaging device.

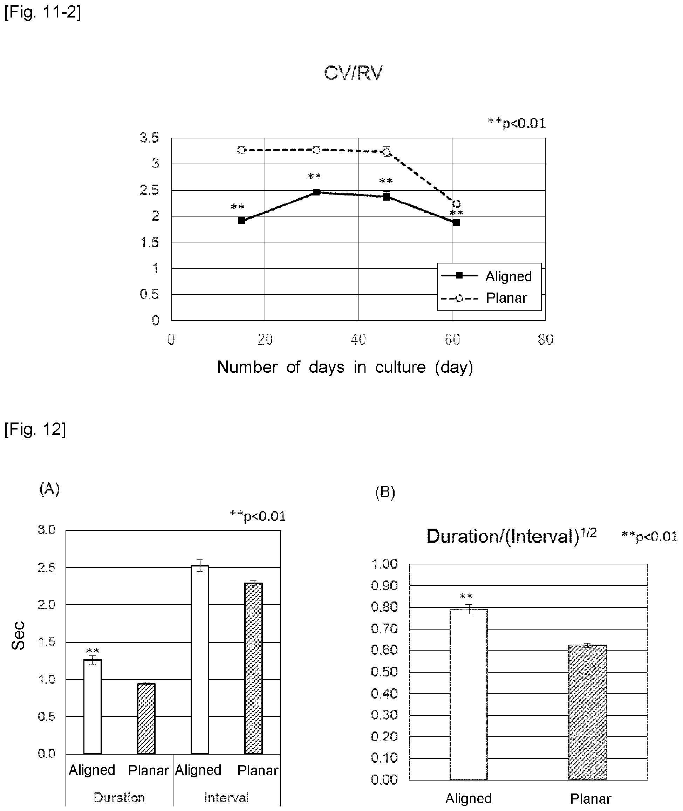

[Fig. 11-2] Fig. 11-2 is a graph showing changes over time in a ratio (CV/RV) of the contractile velocity (CV) to the relaxation velocity (RV) measured using the live cell imaging device.

[Fig. 12] Fig. 12 is graphs showing a duration, an interval, and duration/(interval)$^{1/2}$ on day 5 of culture as measured by a calcium imaging analysis.

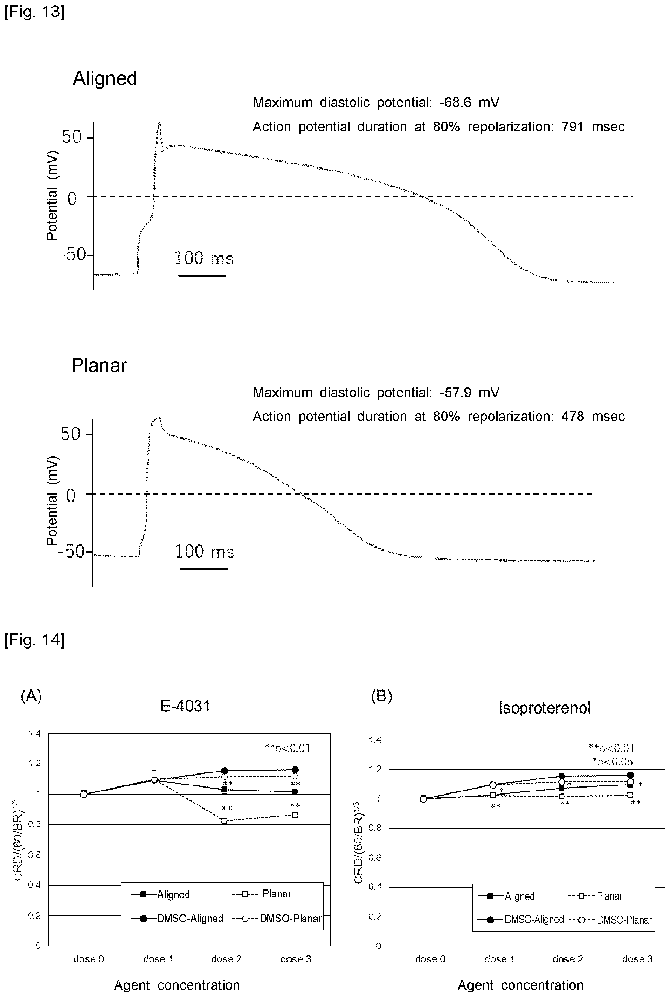

[Fig. 13] Fig. 13 is graphs showing results of a patch-clamp test.

[Fig. 14] Fig. 14 is graph showing an effect of each compound on a cultured cell sheet measured by a live cell imaging analysis.

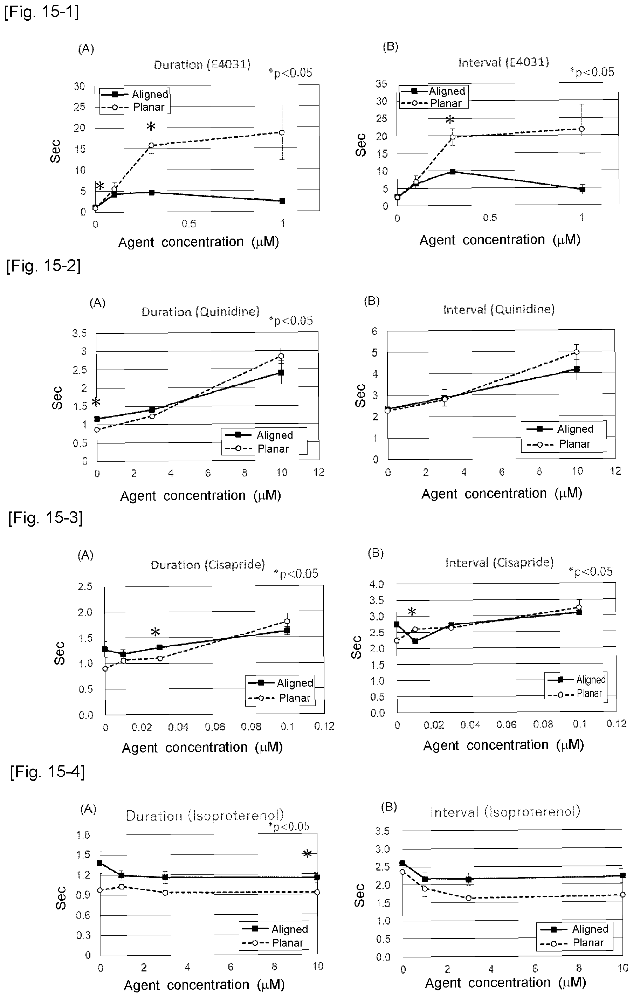

[Fig. 15-1] Fig. 15-1 is graphs showing effects of a compound (E-4031) on the cultured cell sheet measured by a calcium imaging analysis.

[Fig. 15-2] Fig. 15-2 is graphs showing effects of a compound (quinidine) on the cultured cell sheet measured by a calcium imaging analysis.

[Fig. 15-3] Fig. 15-3 is graphs showing effects of a compound (cisapride) on the cultured cell sheet measured by a calcium imaging analysis.

[Fig. 15-4] Fig. 15-4 is graphs showing effects of a compound (isoproterenol) on the cultured cell sheet measured by a calcium imaging analysis.

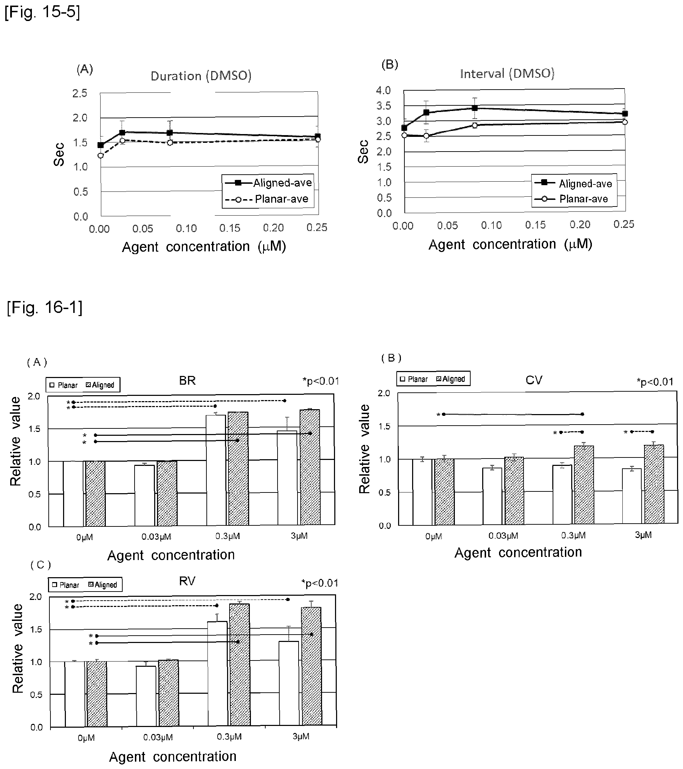

[Fig. 15-5] Fig. 15-5 is graphs showing effects of a compound (DMSO) on the cultured cell sheet measured by a calcium imaging analysis

[Fig. 16-1] Fig. 16-1 is graphs showing effects of a compound (isoproterenol) on the cultured cell sheet on day 15 of culture measured by a live cell imaging analysis.

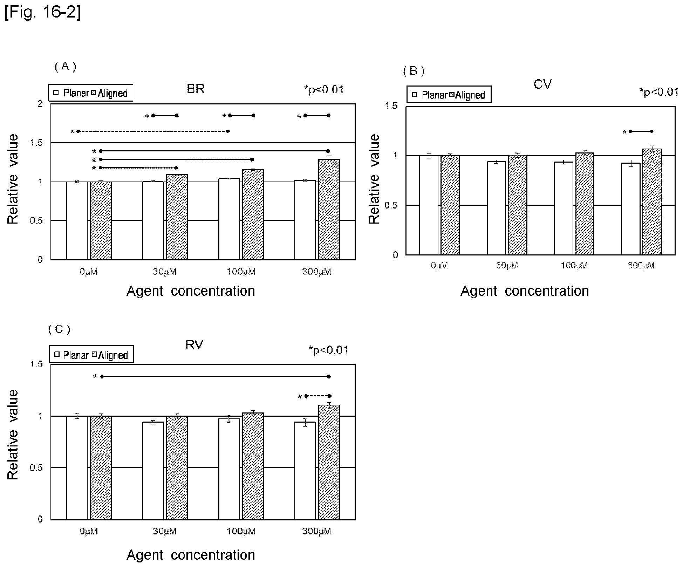

[Fig. 16-2] Fig. 16-2 is graphs showing effects of a compound (milrinone) on the cultured cell sheet on day 15 of culture measured by a live cell imaging analysis.

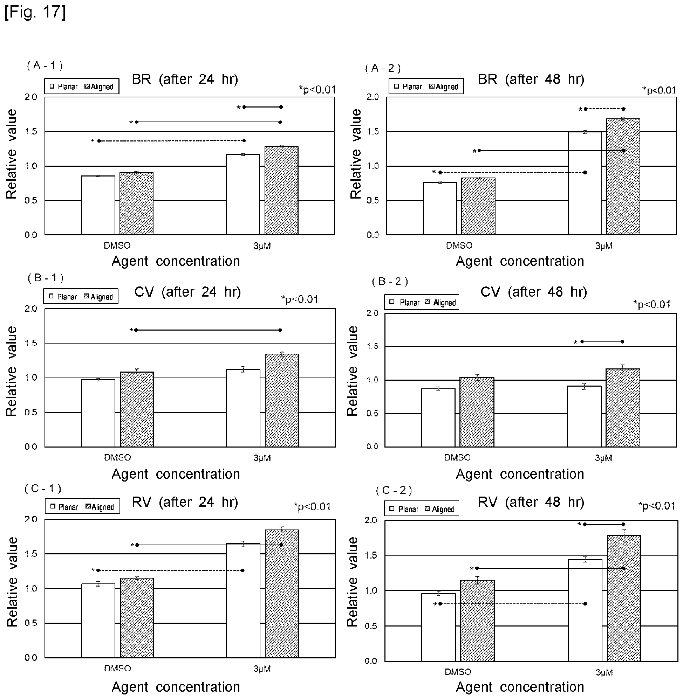

[Fig. 17] Fig. 17 is graphs showing effects of a compound (doxorubicin) on the cultured cell sheet on day 15 of culture measured by a live cell imaging analysis.

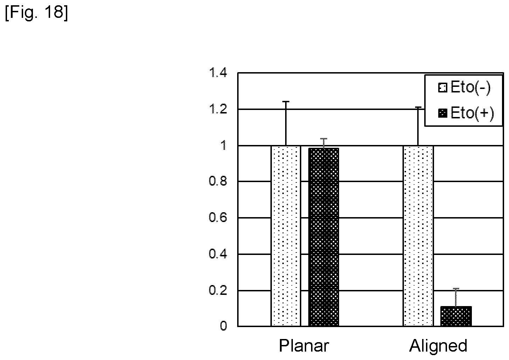

[Fig. 18] Fig. 18 is a graph showing changes in an oxygen consumption rate by addition of a β-oxidation inhibitor etomoxir.

Description of Embodiments

Hereinafter, preferred embodiments of the present invention will be described. Note that, in the present specification, "X to Y" indicating a range means "X or more and Y or less". When numerical ranges are listed stepwise, the upper and lower limits of each numerical range can be combined arbitrarily.

[Cultured cell sheet]

The cultured cell sheet of the present invention is a cultured cell sheet comprising cardiomyocytes, wherein the cardiomyocytes are arranged with alignment, and a degree of alignment determined according to the following formula (1) is 23% or more:

$$\text{Degree of alignment (\%)} = \frac{\text{number of rod-shaped structures included within} \pm 15° \text{ of mode}}{\text{total number of rod-shaped structures}} \times 100 \tag{1}$$

wherein in an image obtained by microscopy of the cultured cell sheet immunostained with an anti-α-actinin antibody, where a screen horizontal direction is regarded as 0°, the degree of alignment is a frequency of rod-shaped structures (Z-bands of myofibrils) included within ±15° of the mode of angles which are measured in a longitudinal direction of the rod-shaped structures detected with an α-actinin antibody within a measurement range of 71.6 μm × 71.6 μm.

The rod-shaped structures refer to the Z-bands of myofibrils as detected with α-actinin. The Z-bands lie orthogonal to the alignment of the myofibrils. When the cardiomyocytes are aligned in one direction with their long axes aligned, the Z-bands form an angle of 90° to the long axis of the cells.

For the image analysis of the rod-shaped structure, the acquired image was divided vertically into three equal parts and horizontally into three equal parts to obtain nine sections (the area of one section is 71.6 μm × 71.6 μm), and three sections (upper left, middle, and lower right sections) of them were used. When three or more images were acquired, one section in the upper left of each image was measured (n = 3 or more), and when two or less images were acquired, three sections (the upper left, middle, and lower right sections) from a representative one image were measured. On average, one section contains 10 cells. For the rod-shaped structure on the image, a rod-shaped structure detected with α-actinin can be detected by angular distribution analysis using an image analysis software (A-image-kun, manufactured by Asahi Kasei Engineering Corporation) or the like, and an angular distribution (0 to 180°) of the rod-shaped structure detected with α-actinin can be determined with the horizontal direction of the image regarded as 0°. It is also possible to mark the rod-shaped structure with a straight line using an image analysis software (for example, Photoshop, manufactured by Adobe) or the like, then save only the marked straight line as an image, and analyze the angular distribution of the straight line using an image analysis software (A-image-kun, manufactured by Asahi Kasei Engineering Corporation) or the like.

The angular distribution is expressed as a frequency of every 10° between 0° and 180°.

It should be noted that the cultured cell sheet means that the cell sheet is not present in vivo, but is produced in vitro.

As a method for maturing cardiomyocytes, culturing cells in a state close to a living body is considered effective. The myocardium in the living body is present in a unidirectional aligned state, in which electrical conduction between cells occurs via intercellular junctions, thereby performing functions such as contraction and relaxation.

The present embodiment has been found that cardiomyocyte maturation (improved physiological activity and motor function) was promoted by using an aligned culture substrate and culturing cardiomyocytes in an aligned state. It has been found that the use of cells cultured in this way enables the use of human cardiomyocytes derived from iPS cell or the like for toxicity and efficacy studies in the field of drug discovery. In addition, the cultured cell sheet is expected to be applied in regenerative medicine because the sheet has been matured and is in a state closer to *in vivo.*

The degree of alignment represented by the above formula (1) is 23% or more, preferably 24% or more, more preferably 25% or more, and even more preferably 26% or more from the viewpoint of promoting maturation of cardiomyocytes, and the upper limit is not particularly limited but is 50% or less from the viewpoint of ease of production.

It is sufficient that the degree of alignment expressed by the above formula (1) satisfies the above range at any number of days in culture.

<Cardiomyocyte>

The cultured cell sheet of the present embodiment is composed of cardiomyocytes.

Examples of the cardiomyocytes include cardiomyocytes of an invertebrate and a vertebrate including a human, and a non-human, and the vertebrate includes fish, amphibian, reptile, bird, and mammal. Specific examples of the mammal include rodents such as a mouse, a rat, a ferret, a hamster, a guinea pig, and a rabbit, a dog, a cat, a sheep, a pig, a cow, a horse, and a primate such as a rhesus monkey, a chimpanzee, an orangutan, a human, and the like. In addition to the mammal, the vertebrate includes fishes, birds including poultry, reptiles, and the like.

Among them, cardiomyocytes of mammals including humans and mice are preferred, and cardiomyocytes of humans are more preferred.

In the present embodiment, cardiomyocytes may be cardiomyocytes induced to differentiate from pluripotent stem cells (stem cells) by a method using various mesodermal lineage differentiation-inducing factor. In addition, in the present embodiment, the cardiomyocytes may be induced cardiomyocytes (iCMs, cardiomyocytes obtained by direct reprogramming) induced by introducing transcription factors into fibroblasts or blood cells of a patient or the like. In addition, the cardiomyocytes of the present embodiment may be primary cell culture cardiomyocytes obtained from the heart of a patient or the like.

Among them, the cardiomyocytes are preferably stem cell-derived cardiomyocytes.

Here, the stem cells include multipotential stem cells (stem cells) that can differentiate into various types of cells in a living organism such as a primate including a human and a mammal other than primate. It is preferable that the stem cells are capable of being passaged, maintain a state in which differentiation does not progress even if the stem cells are passaged, and have properties in which a karyotype or the like is hardly changed or an epigenetic phenotype is hardly changed. It is also preferable that the stem cells have sufficient proliferation capacity *in vitro* in this context. Specific examples of such a stem cell include an embryonic stem cell (hereinafter, referred to as an "ES cell"), an induced pluripotent stem cell (hereinafter, referred to as an "iPS cells"), and another artificially generated or selected stem cell having pluripotency. These stem cells may be stem cells generated by reprogramming somatic cells with various vectors such as retroviruses, adenoviruses, and plasmids containing specific genes, RNA, low molecular weight compounds, and the like.

Although the stem cell does not necessarily need to be a cell having pluripotency close to totipotency, but it is preferable to use a pluripotent cell having higher multipotency than a normal cell. In addition, the stem cell may be a cell generated from a cell obtained from a patient with a disease, a cell that serves as a model of another disease, a cell into which a reporter gene has been incorporated (reporter cell), a cell that can be conditionally knocked out, another genetically engineered cell, or the like. This genetic engineering includes addition, modification, or deletion of a gene in a chromosome, addition of a gene or the like by various vectors or artificial chromosomes, alteration of epigenetic control, addition of an artificial genetic material such as PNA, and other genetic engineering.

Among them, the cardiomyocytes in the present embodiment are preferably cardiomyocytes derived from ES cells or iPS cells from the viewpoint of availability and differentiation induction into cardiomyocytes.

<Gene expression>

The cultured cell sheet of the present embodiment preferably has a characteristic change in gene expression level associated with maturation of the cardiomyocytes. The characteristic change in gene expression level is described in, for example, Reference Literature 1 (Circ. Res. 2020 Apr 10: 126(8), 1086-1106) and Reference Literature 2 (Nat Rev Cardiol. 2020 Jun; 17(6):341-359).

In the method of the present invention, the gene expression level is measured according to the expression information of RNA, and examples of a method of obtaining the expression information of RNA include, but are not particularly limited to, converting RNA contained in a sample into cDNA by reverse transcription, followed by measurement of the cDNA or an amplified product thereof to obtain the expression information of RNA. Examples of a method of measuring the expression level include microarray, quantitative RT-PCR, and RNA-Seq, of which RNA-Seq is preferred.

The expression level of RNA is quantified by a signal intensity ratio when microarray analysis is used and by the number of sequence reads (read count value) mapped to the genome in RNA-seq analysis. The number of reads mapped to each transcript is used for analysis after a process called normalization in which the number of reads is corrected by the length of the transcript, the total number of reads obtained in the analysis, or the like. Specific examples of a normalization method include use of counts per million (CPM), in which the total number of reads is corrected to 1 million in order to correct a difference in the total number of reads between samples; and reads per kilobase of exon per million mapped sequence reads (RPKM) value, which is the number of reads corrected by the length of each gene when the length of the transcript is 1 kb and the total number of reads is 1 million. Similarly, fragments per kilobase of exon per Million mapped sequence reads (FPKM) value, which is the number of fragments corrected by the gene length of each gene when the total number of reads is 1 million, transcripts per million (TPM) value, which represents the number of transcripts when the number of reads of each transcript is corrected by the gene length and the total number of reads is 1 million, and the like are also commonly used. CPM and TPM are used, but the present embodiment is not limited thereto.

The cultured cell sheet of the present embodiment preferably has a higher expression level of myosin light chain 3 (MYL3) gene, and the ratio of an expression level of MYL3 gene to an expression level of the actin beta (ACTB) gene, which is assumed to be a constant expression level regardless of the tissue or culture period, MYL3/ACTB, is preferably 12.0 or more, more preferably 12.5 or more. Although depending on the number of days in culture, the ratio is even more preferably 15.0 or more. The upper limit is not particularly limited but is generally 30.0 or less, preferably 25.0 or less. Note that in the cultured cell sheet of the present embodiment, a change in the gene expression level is observed depending on the number of days in culture, but it is sufficient if the ratio of the gene expression levels described above may be achieved on any number of days in culture.

In addition, it has been reported that a change in isoform associated with maturation occurs in cardiomyocytes (see Reference Literature 1 above), and isoform switching from myosin heavy chain 6 (MYH6) to myosin heavy chain 7 (MYH7) is known as such a change in isoform.

In the cultured cell sheet of the present embodiment, the ratio of an expression level of the MYH7 gene to an expression level of the MYH6 gene, MYH7/MYH6, is preferably 6.0 or more and more preferably 7.0 or more. In addition, the upper limit is not particularly limited but is generally 15.0 or less, preferably 10.0 or less. Note that in the cultured cell sheet of the present embodiment, a change in the gene expression level is observed depending on the number of days in culture, but it is sufficient if the ratio of the gene expression levels described above may be achieved on any number of days in culture.

Furthermore, the cultured cell sheet preferably has an increased gene expression level of creatin kinase, M-type (CKM) associated with myocardial contraction. In the cultured cell sheet of the present embodiment, the expression level of the CKM gene is preferably 900 transcripts per kilobase million (TPM) or more, more preferably 1000 TPM or more, and even more preferably 1100 TPM or more on any number of days in culture. In addition, the upper limit thereof is not particularly limited but is generally 1500 TPM or less.

The ratio of the expression level of the CKM gene to that of the ACTB gene, CKM/ACTB, is preferably 1.80 or more, more preferably 2.00 or more, and even more preferably 2.10 or more for any number of days in culture, and the upper limit is not particularly limited, but is generally 5.0 or less, preferably 4.5 or less.

The cultured cell sheet preferably has an increased expression level of cytochrome C oxidase subunit 6A2 (COX6A2) gene, a protein localized in mitochondria. In the cultured cell sheet of the present embodiment, the expression level of the COX6A2 gene is preferably 850 TPM or more, more preferably 875 TPM or more, and even more preferably 900 TPM or more on any number of days in culture, and the upper limit is not particularly limited, but is generally 2000 TPM or less, preferably 1500 TPM or less.

The ratio of the expression level of the COX6A2 gene to that of the ACTB gene, COX6A2/ACTB, is preferably 1.95 or more, more preferably 2.20 or more, and even more preferably 2.40 or more for any number of days in culture, and the upper limit is not particularly limited, but is generally 5.00 or less, preferably 3.50 or less.

The cultured cell sheet preferably has an increased expression level of creatine kinase, mitochondrial 2 (CKMT2) gene, a protein localized in mitochondria. In the cultured cell sheet of the present embodiment, the expression level of the CKMT2 gene is preferably 330 TPM or more and more preferably 350 TPM or more on any number of days in culture, and the upper limit is not particularly limited, but is, for example, 1000 TPM or less, preferably 800 TPM or less.

The ratio of the expression level of the CKMT2 gene to that of the ACTB gene, CKMT2/ACTB, is preferably 0.60 or more, more preferably 0.65 or more, on days 15 to 30 of culture, and the upper limit is not particularly limited, but is generally 3.0 or less, preferably 1.5 or less. In addition, CKMT2/ACTB is preferably 0.80 or more on any number of days in culture.

The cultured cell sheet preferably has an increased expression level of lactate dehydrogenase A subunit (LDHA) gene, a glycolytic enzyme. Cellular activation is believed to be accompanied by increased energy production, leading to an increase in the expression level of LDHA gene.

In the cultured cell sheet of the present embodiment, the expression level of the LDHA gene is preferably 550 TPM or more, more preferably 600 TPM or more, and even more preferably 650 TPM or more on any number of days in culture, and the upper limit is not particularly limited, but is, for example, 2000 TPM or less, preferably 1500 TPM or less.

The ratio of the expression level of the LDHA gene to that of the ACTB gene, LDHA/ACTB, is preferably 0.80 or more, more preferably 1.00 or more, and even more preferably 1.20 or more, and the upper limit is not particularly limited, but is generally 5.0 or less, preferably 4.0 or less.

In the cultured cell sheet, it is preferable that the expression levels of calcium voltage-gated channel auxiliary subunit alpha2delta1 (CACNA2D1) related to the formation of calcium ion channels in myocardium; potassium inwardly rectifying channel subfamily J member 2 (KCNJ2) and potassium voltage-gated channel subfamily E regulatory subunit 1 (KCNE1) related to the formation of potassium ion channels in the myocardium; and sodium voltage-gated channel alpha subunit 5 (SCN5A) related to the formation of sodium ion channels in the myocardium increase over time. With the maturation of cardiomyocytes, the contractile relaxation movement of cardiomyocytes is activated, and along with which, it is believed that the expression levels of genes related to the formation of ion channels increase.

It should be noted that, for the CACNA2D1 gene, the KCNJ2 gene, the KCNE1 gene and the SCN5A gene, ATPase Na+/K+ transporting subunit alpha 1 (ATP1A1) involved in the generation of Na+ and K+ gradients inside and outside the cell membrane is used instead of the aforementioned ACTB. ATP1A1 is selected because that ATP1A1 is a cardiomyocyte membrane protein that is constantly expressed, and it has been reported that the expression level thereof varies little in iPS cell-derived cardiomyocytes, fetal heart muscle, and adult heart muscle (see Okai, et al., Video-based assessment of drug-induced effects on contractile motion properties using human induced pluripotent stem cell-derived cardiomyocytes, Journal of Pharmacological and Toxicological Methods, Volume 105, September 2020, 106893).

In the cultured cell sheet of the present embodiment, the expression level of the CACNA2D1 gene associated with calcium ion channel formation in the myocardium is preferably 2100 CPM or more, more preferably 2200 CPM or more, even more preferably 2300 CPM or more, and still more preferably 2400 CPM or more on any number of days in culture, and the upper limit is not particularly limited, but is, for example, 3500 CPM or less, preferably 3000 CPM or less.

The ratio of the expression level of the CACNA2D1 gene to that of the ATP1A1 gene, CACNA2D1/ATP1A1, is preferably 0.08 or more, more preferably 0.085 or more, even more preferably 0.09 or more, and still more preferably 0.095 or more on any of days 15 to 30 of culture, and the upper limit is not particularly limited, but is generally 0.50 or less, preferably 0.20 or less.

In the cultured cell sheet of the present embodiment, the expression level of the KCNJ2 gene is preferably 950 CPM or more, more preferably 1000 CPM or more, and even more preferably 1200 CPM or more on any number of days in culture, and the upper limit is not particularly limited, but is, for example, 3000 CPM or less, preferably 2000 CPM or less.

The ratio of the expression level of the KCNJ2 gene to that of the ATP1A1 gene, KCNJ2/ATP1A1, is preferably 0.038 or more, more preferably 0.040 or more, and even more preferably 0.045 or more on any of days 15 to 45 of culture, and the upper limit is not particularly limited, but is generally 0.15 or less, preferably 0.10 or less.

In the cultured cell sheet of the present embodiment, the expression level of the KCNE1 gene is preferably 50 CPM or more, more preferably 60 CPM or more, and even more preferably 70 CPM or more, still more preferably 75 CPM or more on any of days 15 to 45 of culture, and the upper limit is not particularly limited, but is, for example, 200 CPM or less, preferably 150 CPM or less.

The ratio of the expression level of the KCNE1 gene to that of the ATP1A1 gene, KCNE1/ATP1A1, is preferably 0.0025 or more, more preferably 0.003 or more, and even more preferably 0.0035 or more on any of days 15 to 45 of culture, and the upper limit is not particularly limited, but is generally 0.01 or less, preferably 0.007 or less.

In the cultured cell sheet of the present embodiment, the expression level of the SCN5A gene is preferably 22000 CPM or more, more preferably 23000 CPM or more, and even more preferably 23500 CPM or more on any of days 15 to 45 of culture, and the upper limit is not particularly limited, but is, for example, 50000 CPM or less, preferably 40000 CPM or less.

The ratio of the expression level of the SCN5A gene to that of the ATP1A1 gene, SCN5A/ATP1A1, is preferably 0.85 or more, more preferably 0.90 or more, and even more preferably 0.95 or more on any of days 15 to 45 of culture, and the upper limit is not particularly limited, but is generally 5.0 or less, preferably 2.0 or less.

Preferably, the expression levels of lipoprotein lipase (LPL), which is present on the cell surface and promotes the uptake of lipids into cells, and acetyl-CoA acetyltransferase 1 (ACAT1), hydroxyalkyl-CoA dehydrogenase trifunctional multienzyme complex subunit alpha (HADHA), and hydroxyalkyl-CoA dehydrogenase trifunctional multienzyme complex subunit beta (HADHB), which are localized in intracellular mitochondria and are associated with the β-oxidation of fatty acids, increase over time in the cultured cell sheet. With cardiomyocyte maturation, motor function improves and ATP consumption increases. Expression of these genes is thought to increase for more efficient ATP production.

In the cultured cell sheet of the present embodiment, the expression level of the LPL gene is preferably 3100 CPM or more, more preferably 3500 CPM or more, even more preferably 4000 CPM or more, and still more preferably 4500 CPM or more on any of days 15 to 45 of culture, and the upper limit is not particularly limited, but is, for example, 9000 CPM or less, preferably 7500 CPM or less.

The ratio of the expression level of the LPL gene to that of the ACTB gene, LPL/ACTB, is preferably 0.45 or more, more preferably 0.50 or more, and even more preferably 0.60 or more on any of days 15 to 45 of culture, and the upper limit is not particularly limited, but is generally 2.0 or less, preferably 1.5 or less, and more preferably 1.0 or less.

In the cultured cell sheet of the present embodiment, the expression level of the ACAT1 gene is preferably 3500 CPM or more and more preferably 3800 CPM or more on any of days 30 to 45 of culture, and the upper limit is not particularly limited, but is, for example, 8000 CPM or less, preferably 6000 CPM or less.

The ratio of the expression level of the ACAT1 gene to that of the ACTB gene, ACAT1/ACTB, is preferably 0.43 or more, more preferably 0.45 or more, and even more preferably 0.50 or more on any of days 15 to 30 of culture, and the upper limit is not particularly limited, but is generally 2.0 or less, preferably 1.5 or less, and more preferably 1.0 or less.

In the cultured cell sheet of the present embodiment, the expression level of the HADHA gene is preferably 6000 CPM or more, more preferably 6200 CPM or more, and even more preferably 6500 CPM or more on any of days 30 to 45 of culture, and the upper limit is not particularly limited, but is, for example, 12000 CPM or less, preferably 10000 CPM or less.

The ratio of the expression level of the HADHA gene to that of the ACTB gene, HADHA/ACTB, is preferably 0.75 or more, more preferably 0.80 or more, and even more preferably 0.90 or more on any of days 15 to 30 of culture, and the upper limit is not particularly limited, but is generally 2.5 or less, preferably 2.0 or less, and more preferably 1.5 or less.

In the cultured cell sheet of the present embodiment, the expression level of the HADHB gene is preferably 8200 CPM or more, more preferably 8300 CPM or more, and even more preferably 8500 CPM or more on any of days 15 to 45 of culture, and the upper limit is not particularly limited, but is, for example, 20000 CPM or less, preferably 15000 CPM or less.

The ratio of the expression level of the HADHB gene to that of the ACTB gene, HADHB/ACTB, is preferably 0.95 or more, more preferably 1.00 or more, even more preferably 1.10, and still more preferably 1.20 or more on any of days 15 to 30 of culture, and the upper limit is not particularly limited, but is generally 2.5 or less, preferably 2.0 or less, and more preferably 1.8 or less.

In the cultured cell sheet, it is preferable that expression levels of a peroxisome proliferator-activated receptor gamma (PPARG) coactivator 1 alpha (PPARGC1A) gene encoding a transcription factor PGC-1α that acts as a master regulator of mitochondrial biosynthesis; estrogen related receptor alpha (ESRRA) gene encoding estrogen-related receptor ERR-α related to the maturation of cardiomyocytes; a vascular endothelial growth factor A (VEGFA) gene encoding a vascular growth factor; an apelin (APLN) gene encoding myokine that stimulates cardiac contraction in myocardium; a fatty acid binding protein 3 (FABP3) gene encoding myokine that regulates the uptake of long-chain fatty acids in myocardium; an endothelial cell specific molecule 1 (ESM1) gene that is specifically expressed in endothelial cells; and an endomucin (EMCN) gene encoding mucin-like glycoprotein secreted from epithelial cells or the like increase over time. It is also preferable that an expression level of a BCL2 apoptosis regulator (BCL2) gene involved in the differentiation of cardiomyocytes is maintained.

It is believed that the expression levels of the PPARGC1A gene involved in mitochondrial functionalization and the VEGFA gene involved in angiogenesis are increased by the enhancement of motor function along with the maturation of cardiomyocytes. It is also believed that, along with this, the expression levels of genes encoding myokine such as APLN and FABP3 are increased. In addition, it is believed that the expression levels of genes such as ESM1 and EMCN specific for endothelial cells are increased. It is also believed that the expression of the BCL2 gene involved in the differentiation of cardiomyocytes is maintained.

In the cultured cell sheet of the present embodiment, the expression level of the PPARGC1A gene is preferably 4500 CPM or more, more preferably 4700 CPM or more, and even more preferably 5000 CPM or more on any of days 15 to 45 of culture, and the upper limit is not particularly limited, but is, for example, 20000 CPM or less, preferably 15000 CPM or less, and more preferably 8000 CPM or less.

The ratio of the expression level of the PPARGC1A gene to that of the HIF1A gene, PPARGC1A/HIF1A, is preferably 0.80 or more, more preferably 0.85 or more, and even more preferably 0.90 or more on any day of culture, and the upper limit is not particularly limited, but is generally 2.5 or less, preferably 2.0 or less, and more preferably 1.5 or less.

In the case of the PPARGC1A gene, ESRRA gene, VEGF gene, APLN gene, FABP3 gene, ESM1 gene, EMCN gene, and BCL2 gene, hypoxia inducible factor 1 alpha (HIF1A), a transcription factor induced under hypoxic conditions of cells, is used instead of ACTB as described above. The reason is that enhanced angiogenesis described in this section has been reported to be induced by hypoxia or exercise stimulation. Although both environments can be adopted under cell culture conditions, exercise-stimulated upregulation of each factor seems to occur in well-conditioned (functionally mature) cardiomyocytes (see Arany, et al., HIF-independent regulation of VEGF and angiogenesis by the transcriptional coactivator PGC-1a, Vol. 451, 21 February 2008).

In the cultured cell sheet of the present embodiment, the expression level of the ESRRA gene is preferably 2750 CPM or more, more preferably 2900 CPM or more, and even more preferably 3000 CPM or more on any of days 15 to 45 of culture, and the upper limit is not particularly limited, but is, for example, 10000 CPM or less, preferably 6000 CPM or less, and more preferably 4000 CPM or less.

The ratio of the expression level of the ESRRA gene to that of the HIF1A gene, ESRRA/HIF1A, is preferably 0.55 or more, more preferably 0.57 or more, and even more preferably 0.60 or more on any day of culture, and the upper limit is not particularly limited, but is generally 2.0 or less, preferably 1.5 or less, and more preferably 1.0 or less.

In the cultured cell sheet of the present embodiment, the expression level of the VEGFA gene is preferably 3300 CPM or more, more preferably 3700 CPM or more, even more preferably 4500 CPM or more, and still more preferably 6000 CPM or more on any day of culture, and the upper limit is not particularly limited, but is, for example, 25000 CPM or less, preferably 15000 CPM or less, and more preferably 10000 CPM or less.

The ratio of the expression level of the VEGFA gene to that of the HIF1A gene, VEGFA/ACTB, is preferably 0.65 or more, more preferably 0.70 or more, even more preferably 0.90, still more preferably 1.2 or more, and particularly preferably 1.5 or more on any day of culture, and the upper limit is not particularly limited, but is generally 5.0 or less, preferably 3.0 or less, and more preferably 2.0 or less.

In the cultured cell sheet of the present embodiment, the expression level of the APLN gene is preferably 100 CPM or more, more preferably 150 CPM or more, and even more preferably 200 CPM or more on any day of culture, and the upper limit is not particularly limited, but is, for example, 50000 CPM or less, preferably 3000 CPM or less, and more preferably 2000 CPM or less.

The ratio of the expression level of the APLN gene to that of the HIF1A gene, APLN/HIF1A, is preferably 0.005 or more, more preferably 0.020 or more, even more preferably 0.030 or more, and still more preferably 0.040 or more on any day of culture, and the upper limit is not particularly limited, but is generally 1.0 or less, preferably 0.50 or less, and more preferably 0.30 or less.

In the cultured cell sheet of the present embodiment, the expression level of the FABP3 gene is preferably 30000 CPM or more, more preferably 31000 CPM or more, and even more preferably 32000 CPM or more on any day of culture, and the upper limit is not particularly limited, but is, for example, 100000 CPM or less, preferably 60000 CPM or less, and more preferably 40000 CPM or less.

The ratio of the expression level of the FABP3 gene to that of the HIF1A gene, FABP3/HIF1A, is preferably 5.0 or more, more preferably 5.5 or more, and even more preferably 6.0 or more on any day of culture, and the upper limit is not particularly limited, but is generally 15.0 or less, preferably 12.0 or less, and more preferably 8.0 or less.

In the cultured cell sheet of the present embodiment, the expression level of the ESM1 gene is preferably 5 CPM or more, more preferably 25 CPM or more, even more preferably 40 CPM or more, still more preferably 100 CPM or more, and particularly preferably 200 CPM or more on any day of culture, and the upper limit is not particularly limited, but is, for example, 3000 CPM or less, preferably 1000 CPM or less, and more preferably 500 CPM or less.

The ratio of the expression level of the ESM1 gene to that of the HIF1A gene, ESM1/HIF1A, is preferably 0.002 or more, more preferably 0.005 or more, and even more preferably 0.010 or more on any day of culture, and the upper limit is not particularly limited, but is generally 0.200 or less, preferably 0.150 or less, and more preferably 0.100 or less.

In the cultured cell sheet of the present embodiment, the expression level of the EMCN gene is preferably 10 CPM or more, more preferably 20 CPM or more, even more preferably 40 CPM or more, and still more preferably 60 CPM or more on any day after day 7 of culture, and the upper limit is not particularly limited, but is, for example, 1000 CPM or less, preferably 500 CPM or less, and more preferably 300 CPM or less.

The ratio of the expression level of the EMCN gene to that of the HIF1A gene, EMCN/HIF1A, is preferably 0.002 or more, more preferably 0.004 or more, even more preferably 0.005 or more, and still more preferably 0.010 or more on any day after day 7 of culture, and the upper limit is not particularly limited, but is generally 0.100 or less, preferably 0.070 or less, and more preferably 0.050 or less.

In the cultured cell sheet of the present embodiment, the expression level of the BCL2 gene is preferably 60 CPM or more, more preferably 70 CPM or more, even more preferably 80 CPM or more, and still more preferably 90 CPM or more on any day after day 7 of culture, and the upper limit is not particularly limited, but is, for example, 1000 CPM or less, preferably 500 CPM or less, and more preferably 300 CPM or less.

The ratio of the expression level of the BCL2 gene to that of the HIF1A gene, BCL2/HIF1A, is preferably 0.013 or more, more preferably 0.015 or more, and even more preferably 0.017 or more on any day after day 7 of culture, and the upper limit is not particularly limited, but is generally 0.100 or less, preferably 0.070 or less, and more preferably 0.050 or less.

<Mitochondrial amount>

Cardiomyocytes tend to increase a mitochondrial amount and a mitochondrial activity as they mature.

Area and luminance of mitochondria and nuclei are measured by staining the cultured cell sheet of the present embodiment with a fluorescent dye that selectively labels the mitochondria based on membrane potential and a fluorescent dye that selectively labels the nuclei, and obtaining a fluorescent image.

Examples of the fluorescent dye that selectively labels the mitochondria based on the membrane potential include Mito-Tracker series manufactured by Thermo Fisher Scientific, Inc. Examples of the fluorescent dye that selectively labels the nuclei include Hoechst 33342.

At this time, the area and the luminance (intensity) of the mitochondria per cell are calculated by dividing the measured area and intensity of the mitochondria by the number of nuclei, that is, the number of cells. It should be noted that the area per cell reflects the number of mitochondria, and the luminance per cell reflects the mitochondrial activity.

The area of mitochondria per cell is preferably 200 $\mu m^2$ or more, more preferably 300 $\mu m^2$ or more, further preferably 400 $\mu m^2$ or more, and even more preferably 450 $\mu m^2$ or more, and although the upper limit thereof is not particularly limited, generally 1000 $\mu m^2$ or less, and preferably 800 $\mu m^2$ or less.

The luminance (intensity) of the mitochondria per cell is preferably $2 \times 10^6$ or more, more preferably $3 \times 10^6$ or more, and further preferably $4 \times 10^6$ or more, and although the upper limit thereof is not particularly limited, generally $10 \times 10^6$ or less, preferably $8 \times 10^6$ or less. It should be noted that the luminance per cell is the luminance measured by the method described in Examples.

The cell seeding conditions when measuring the area of mitochondria and the luminance of mitochondria per cell described above are $6 \times 10^4$ cells/well, day 9 of the number of days in culture (after day 5), and the area of 96 wells (0.33 $cm^2$).

<Live cell imaging>

Changes in motion associated with myocardial cell contraction and relaxation can be detected in the cultured cell sheet composed of cardiomyocytes. It is observed in vivo that cardiomyocytes are aligned in one direction and contract and relax in the alignment direction.

A beating rate (BR), a contractile velocity (CV), a relaxation velocity (RV), a contraction-relaxation duration (CRD) and a degree of alignment can be analyzed using a live cell imaging device (e.g., SI8000, manufactured by Sony Corporation) by shooting the cultured cell sheet (live cells) under the culture conditions to obtain a video data, and detecting motion vectors (velocity, direction, quantity) associated with the contraction and relaxation of cardiomyocytes from the video data. For the measurement of BR, CV, RV, CRD, and the like by live cell imaging, see, for example, Methods in Molecular Biology, vol. 2320, Chapter 15.

For the measurement method of these, see also the method described in Examples.

Here, the degree of alignment is calculated by determining the angle of motion vector with the screen horizontal direction regarded as 0°, and determining a ratio of the number of vectors showing an angle of ±5° from an angle of the mode of the vector angle to the sum of the number of vectors detected. The degree of alignment obtained from the live cell imaging device is determined by the following equation.

Degree of alignment (%) = (number of vectors included in range of ±5° of mode)/(total number of vectors) × 100

The degree of alignment measured using the live cell imaging of the cultured cell sheet of the present embodiment is preferably 10% or more, more preferably 12% or more, further preferably 16% or more, even more preferably 18% or more, and particularly preferably 20% or more. The upper limit of the degree of alignment is not particularly limited.

In the live cell imaging, the adult beating rate is on average 60 beats/min, and there are tendencies that the closer the beating rate (BR) to 60 beats/min, the closer to normal mature cardiomyocytes; the faster the contractile velocity (CV), the closer to normal mature cardiomyocytes; the faster the relaxation velocity (RV), the closer to mature cardiomyocytes; and the shorter the contraction-relaxation duration (CRD), the closer to normal mature cardiomyocytes.

It is preferable that the ratio of the contractile velocity (CV) to the relaxation velocity (RV), CV/RV, is smaller. The CV/RV of the cultured cell sheet of the present embodiment is preferably 2.8 or less, more preferably 2.6 or less, and preferably 1.0 or more. That is, the CV/RV preferably satisfies the following equation (2).

$$1.0 \leq CV/RV \leq 2.8 \quad (2)$$

<Ca-imaging analysis>

In cardiomyocytes, a phenomenon called calcium transient in which concentrations of Ca in the whole cytoplasm provoked by the action potential increase about the same time along with myocardial contraction is observed. The calcium transient can be observed over time by treating the cultured cell sheet of the present embodiment with a fluorescent Ca indicator and observing with confocal quantitative image cytometry (Ca-imaging analysis). A dedicated analysis software is used as a method to create a waveform graph from the signal information of calcium transient and analyze the parameters described below.

In cardiomyocytes with some abnormalities due to immature cardiomyocytes or diseases, the line width (duration) of 20% height of the waveform peak of calcium transient tends to be extended compared to that of normal mature cardiomyocytes. Since the line width (duration) of 20% height varies depending on the beating rate, it is preferable that the relationship between the duration (seconds) and the 1/2 power of interval (seconds) satisfies the following equation (3).

$$\text{Duration}/(\text{Interval})^{1/2} \geq 0.68 \qquad (3)$$

It has been reported that there is a correlation between the line width (duration) of 20% height, which reflects the calcium transient duration, and the action potential duration. In cardiomyocytes, as the maturation is longer, the action potential duration tends to be longer. The interval is affected by the heartbeat. In the analysis of the electrocardiogram, the value of the action potential duration corrected with the beating rate is usually employed, thus Duration/(Interval)$^{1/2}$ as shown in equation (3) has been adopted.

In the present embodiment, it is preferable that the cells constituting the cultured cell sheet in a patch-clamp test in a 25°C solution have an action potential duration at 80% repolarization of 600 msec or more and a maximum diastolic potential of -60 mV or less.

In the patch-clamp test, a current of 20 pA to 100 pA is applied at 0.2 Hz under a current clamp into the cells of cardiomyocyte in a Tyrode solution at 25°C to generate an action potential, and the change in potential is measured. The current can be selected as appropriate to the extent that it can generate an appropriate action potential.

As an indicator of the action potential duration, the action potential duration at 80% repolarization has been adopted. The action potential duration at 80% repolarization is the time from the application of current to the time required to become a potential equivalent to 80% repolarization when the difference between the peak height of the action potential shown as the first phase and the maximum diastolic potential is 100%. That is, it is the time from the rise time of action potential to the time required for the action potential to become a potential equivalent to 80% repolarization.

When cardiomyocytes mature, the action potential duration tends to be longer and the maximum diastolic potential tends to be deeper compared to immature cardiomyocytes.

The action potential duration at 80% repolarization of the cells constituting the cultured cell sheet of the present embodiment is preferably 600 msec or more, more preferably 650 msec or more, further preferably 700 msec or more, and even more preferably 750 msec or more, and the upper limit is not particularly limited, but it is preferably 1200 msec or less, more preferably 1050 msec or less, and further preferably 900 msec or less from the viewpoint that it is preferable that the action potential duration of the cells is about the same as that of cardiomyocytes in vivo.

In addition, the maximum diastolic potential of the cells constituting the cultured cell sheet of the present embodiment is preferably -60 mV or less, more preferably -62.5 mV or less, further preferably -65 mV or less, and even more preferably -67.5 mV or less from the viewpoint that it is preferable that the maximum diastolic potential value thereof is closer to that of new cells in vivo. Although the lower limit is not particularly limited, it is preferably a value close to -80 mV since the value of the adult ventricular cardiomyocytes is about -80 mV.

For the action potential duration and the maximum diastolic potential (resting membrane potential) in human-derived normal isolated ventricular myocytes, see Circulation, 2013; 127: 575-584.

<Reduction of oxygen consumption rate due to β-oxidation inhibitor>

In the present embodiment, it is preferable that, in the oxygen consumption rate measurement to measure the rate at which oxygen contained in the culture solution is used by the cells constituting the cultured cell sheet, the addition of a β-oxidation inhibitor that inhibits the β-oxidation activity of fatty acids reduces the oxygen consumption rate to 20% or less compared to before the addition of the β-oxidation inhibitor.

Since, after being taken up by the cells, the oxygen contained in the culture solution is consumed in the pathway through which ATP is produced by oxidative phosphorylation by mitochondria, the oxygen consumption rate (OCR) of the culture solution can be used as an index for mitochondrial function analysis. There are metabolic pathways in mitochondria in which glucose, fatty acids, or glutamine is mainly used, and a metabolic pathway can be speculated by adding inhibitors of enzymes involved in respective metabolisms.

The oxygen consumption amount can be measured using a commercially available oxygen consumption rate plate assay kit (manufactured by DOJINDO LABORATORIES) or the like.

Mature cardiomyocytes more depend on fatty acid metabolism than immature cardiomyocytes.

In the oxygen consumption rate measurement to measure the rate at which oxygen contained in the culture solution is used by the cells constituting the cultured cell sheet of the present embodiment, the oxygen consumption rate after the addition of a β-oxidation inhibitor that inhibits the β-oxidation activity of fatty acids is preferably 30% or less, more preferably 20% or less, and further preferably 15% or less when the oxygen consumption rate before the addition of the β-oxidation inhibitor is 100%.

<Application of cultured cell sheet>

The cultured cell sheet of the present embodiment can be used for various applications, for example, regenerative medicine applications. The cultured cell sheet may be a monolayer sheet formed of a single layer of cells or a multilayer sheet formed of two or more layers of cells.

Specifically, a method of using the cultured cell sheet includes, for example, directly attaching a cultured cell sheet to a damaged site in the myocardial tissue of a heart damaged by an ischemic disease such as myocardial infarction or angina pectoris (hereinafter, also referred to as "damaged heart") in the same direction as the contraction of the cardiac tissue, and after attachment, suturing and inserting the sheet. When used for regenerative medicine, several single-layer or double-layer cell sheets may be stacked on top of each other to form a laminate and then used for regenerative medicine. In the case of lamination, the cultured cell sheets are preferably laminated with their alignment directions aligned and may specifically be laminated with their contraction directions or alignment directions aligned.

The cultured cell sheet of the present embodiment may also be used by allowing a compound or drug to be evaluated to act on the cultured cell sheet, as described below, to evaluate the action of the compound or drug on cardiomyocytes.

[Method for producing cultured cell sheet]

The method for producing a cultured cell sheet comprising cardiomyocytes of the present embodiment includes culturing cardiomyocytes by using a specific cell sheet-forming member, the cell sheet-forming member comprising a surface for forming a cultured cell sheet, wherein the surface comprises a plurality of flat portions and a plurality of recession and protrusion portions; each of the flat portions has a shape extending in a first direction, and a plurality of the flat portions are arrayed in a second direction intersecting the first direction over an entirety of the surface; each of the recession and protrusion portions comprises a plurality of stepped structures filling a gap between the flat portions adjacent to each other, and a pitch of the stepped structures is 100 nm or more and 10 μm or less; the stepped structures each comprise a protrusion portion; the recession and protrusion portions each comprise a plurality of the protrusion portions in a bottom surface of a recession portion sandwiched between the flat portions adjacent to each other; and a difference between a height of a distal end surface of each of the recession and protrusion portions and a height of each of the flat portions is 0.5 μm or less in a thickness direction of the cell sheet-forming member.

As shown in Fig. 1(a), the cell sheet-forming member 100 is, for example, a sheet material placed on a culture dish 110 of a petri dish. The petri dish retains the cell suspension in a space surrounded by the culture dish 110 and a lid 120. The bottom of the petri dish may be processed into a surface shape having a specific flat portion and a recession and protrusion portion as described above, where the petri dish itself is a cell sheet-forming member.

As shown in Fig. 1(b), the surface 111 of the cell sheet-forming member 100 includes a plurality of flat portions 130 and a plurality of recession and protrusion portions 140. The recession and protrusion portions 140 are composed of a plurality of stepped structures, and the plurality of stepped structures fill a gap between the flat portions 130 adjacent to each other. The stepped structure is a protrusion portion. The recession and protrusion portion 140 includes a recession portion sandwiched between flat portions 130 adjacent to each other and a plurality of protrusion portions 141 located in a bottom surface of the recession portion.

As shown in Fig. 1(c), each flat portion 130 is a flat surface extending in one direction, a first direction (top-bottom direction of Fig. 1(c)). Each flat portion 130 is arrayed in a second direction (left-right direction of Fig. 1(c)) orthogonally intersecting the first direction over an entirety of the surface 111. Each recession and protrusion portion 140 also extends in the first direction and is arrayed in the second direction over an entirety of surface 111.

Each protrusion portion 141 constituting the recession and protrusion portion 140 is located, for example, at each vertex of the triangular grid, seen from a direction opposite the surface 111. Each recession and protrusion portion 140 repeats such an alignment of the protrusion portions 141 in the first direction and the second direction. When the recession and protrusion portion 140 is one in which the protrusion portions 141 are located at each vertex of a triangular grid, an original plate for forming the protrusion portion 141 can be formed by an etching method using a mask suitable for forming a micro-repetitive structure, for example, using a single particle film as a mask.

Seen from the direction opposite the surface 111, each protrusion portion 141 has, for example, a circular shape. The mode of the distance between the centers of the protrusion portions 141 adjacent to each other is a pitch of the protrusion portion 141. The maximum width of the protrusion portion in the planar view shape of the protrusion portion 141 is the diameter of the protrusion portion 141.

The configuration in which the pitch of the protrusion portion 141 satisfies the following (A) and (B) is suitable from the viewpoint of aligning the extension direction of the cardiomyocytes in the first direction. That is, the configuration in which the pitch of the protrusion portion 141 satisfies the following (A) and (B) is suitable from the viewpoint that advantages and disadvantages for adhesion to the cardiomyocyte are clearly divided between the flat portion 130 and the recession and protrusion portion 140.

(A) Pitch of protrusion portion 141: 100 nm or more and 10 $\mu$m or less
(B) Diameter of protrusion portion 141: 50% or more and 100% or less of pitch of protrusion portion 141

The length of each flat portion 130 in the second direction (short side direction) is a width of the flat portion 130. The length in the second direction (short side direction) between flat portions 130 adjacent to each other is a width of the recession and protrusion portion 140.

The width of the flat portion 130 and the width of the recession and protrusion portion 140 are, for example, 1/10 times or more and 10 times or less in the size of the cell (5 $\mu$m or more and 100 $\mu$m or less) to be cultured. The configuration in which the width of the flat portion 130 and the width of the recession and protrusion portion 140 satisfies the following (C) and (D) is suitable from the viewpoint facilitating aligning the extension direction of the cardiomyocyte in the first direction.

(C) Width of flat portion 130: 10 $\mu$m or more and 50 $\mu$m or less
(D) Width of recession and protrusion portion 140: 10 $\mu$m or more and 50 $\mu$m or less

As shown in Fig. 1(d), the recession and protrusion portion 140 may include a recession potion 142 between protrusion portions 141 adjacent to each other and between the flat portion 130 and the adjacent protrusion portion 141. Since a plurality of the protrusion portions 141 are dotted in the recession and protrusion portion 140, the recession portions 142 that are spaces between the protrusion portions 141 are present in a row in the first direction and the second direction in the recession and protrusion portion 140.

In the thickness direction of the cell sheet-forming member 100, the length between the bottom surface of the recession portion 142 and the flat portion 130 is a height of the flat portion 130. In the thickness direction of the cell sheet-forming member 100, the height difference between a distal end surface of each protrusion portion 141 and the flat portion 130 is a boundary step. The height difference between the bottom surface of the recession portion 142 and the distal end surface of each protrusion portion 141 is a height of the protrusion portion 141. In a configuration in which the distal end surface of each protrusion portion 141 and the flat portion 130 are in one surface, the height of the flat portion 130 and the height of the protrusion portion 141 are equal to each other. The ratio of the pitch of the protrusion portion 141 to the height of the protrusion portion 141 is an aspect ratio of the protrusion portion 141.

The configuration in which the boundary step satisfies the following (E) is suitable from the viewpoint of increasing the flatness of the cell sheet. The configuration in which the height of the protrusion portion 141 satisfies the following (F) and the configuration in which the aspect ratio of the protrusion portion 141 satisfies the following (G) are suitable from the viewpoint of increasing the stability in structure of the recession and protrusion portion 140 and the viewpoint of facilitating forming the recession and protrusion portion 140.

(E) Boundary step: 0.5 $\mu$m or less, preferably 0.3 $\mu$m or less
(F) Height of protrusion portion 141: 50 nm or more and 5 $\mu$m or less
(G) Aspect ratio of protrusion portion 141: 0.1 or more and 10 or less

Then, when the configuration satisfies the above (A) and (B), whether the adhesion to the flat portion 130 is predominant or the adhesion to the recession and protrusion portion 140 is predominant, coupled with that the cells preferentially adheres to one structure and inferiorly adheres to the other structure, the extension directions of the cells align in the first direction that is the extension direction of both structures. As a result, in the cell sheet spreading in two-dimensional direction along the surface 111, it is possible to align the extension direction of the cells in one-dimensional direction, that is, to improve the alignment of the cells.

The configuration that satisfies the above (E), in particular, the configuration in which the distal end surface of each protrusion portion 141 and the flat portion 130 are in one surface, enables the flatness of the cell sheet formed to cover the recession and protrusion portion 140 and the flat portion 130 to increase. Furthermore, the configuration satisfying the above (F) enables the flatness of the cell sheet to further increase.

It should be noted that since the surface 111 of the cell sheet-forming member 100 includes the flat portion 130 and the recession and protrusion portion 140, it is also possible to apply a common cell sheet-forming member 100 to both of the cells in which adhesion to the flat portion 130 is predominant and the cells in which adhesion to the recession and protrusion portion 140 is predominant. That is, it is also possible to increase the versatility of the cell sheet-forming member 100.

The surface 111 of the cell sheet-forming member 100 may be a surface in which an organic substance including, for example, an extracellular matrix such as laminin, collagen, gelatin, fibronectin, polylysine (PDL or PLL), or hyaluronic acid, a polymer, or an adhesion factor such as a gel is applied or may be a surface composed of a metal for the purpose of enhancing the adhesion of cells. The surface 111 of the cell sheet-forming member 100 may be hydrophilic or hydrophobic for the purpose of enhancing the adhesion of cells or the flatness of the cell sheet.

In addition, a stimulus-responsive material may be applied to facilitate peeling and recovery of the cell sheet after forming the cell sheet. As the stimulus-responsive material, the temperature-responsive polymer whose water affinity changes depending on a temperature change is preferred. Specifically, it is preferable that the stimulus-responsive material is poly-N-isopropyl acrylamide (PIPAAm). The stimulus-responsive material may be applied to the substrate using a conventional application method, or the structure may be processed into a substrate treated with the stimulus-responsive material using the method described below.

<Method for producing cell sheet-forming member>

An example of the method for producing a cell sheet-forming member will be described. Note that, in the example described below, a surface 111 of a cell sheet-forming member is formed by transcription of an intaglio 150 using a nano-imprint method.

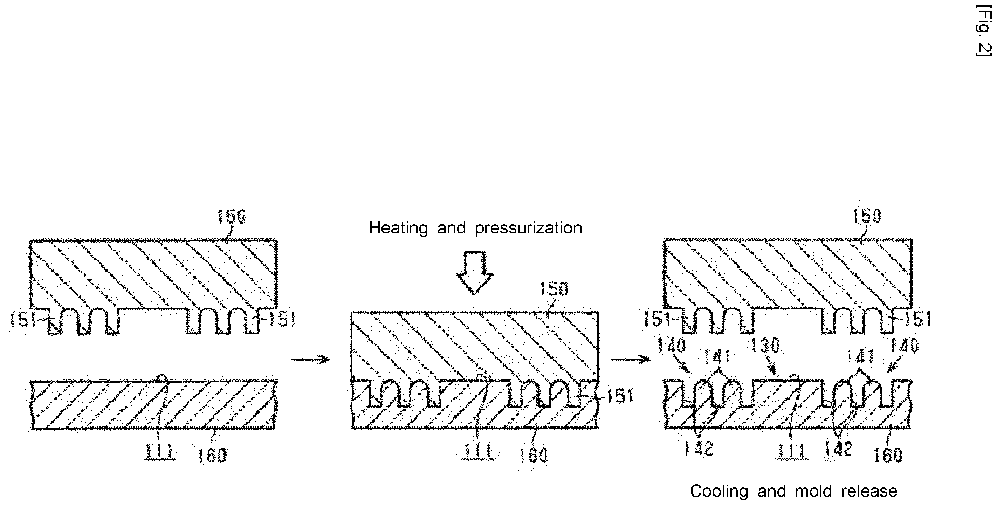

As shown in Fig. 2, the method for producing a cell sheet-forming member includes a step of forming an intaglio 150 and a step of forming a surface 111 of a cell sheet-forming member 100 by transcription of the intaglio 150.

The underside of the intaglio 150 has a shape extending in a first direction (the direction orthogonal to a paper surface) and includes a plurality of flat portions arrayed in a second direction (the left-right direction of the paper surface) intersecting the first direction and a plurality of recession and protrusion portions composed of a stepped structure filling a gap between flat portions adjacent to each other. The flat portion of the intaglio 150 is a portion for forming the flat portion 130 of the cell sheet-forming member 100 by transcription. The recession and protrusion portion of the intaglio 150 is a portion for forming the recession and protrusion portion 140 of the cell sheet-forming member 100 by transcription.

The stepped structure of the intaglio 150 is a protrusion portion or a recession portion. It should be noted that the stepped structure of the intaglio 150 in the present embodiment is a recession portion 151 for forming a protrusion portion 141, and the pitch of the recession portion 151 is 100 nm or more and 10 μm or less. In the step of forming an intaglio 150, a recession and protrusion portion is formed using, for example, at least one of a photolithography method, a colloidal lithography method, an anodic oxidation method, and an interference exposure method for a silicon substrate for forming the intaglio 150. The intaglio 150 itself may be obtained by one or more transcriptions from an original plate. The original plate has a formed shape corresponding to the surface shape of the intaglio 150 using, for example, at least one of a photolithography method, a colloidal lithography method, an anodic oxidation method, and an interference exposure method for a silicon substrate.

The surface 111 of the substrate 160 for forming the cell sheet-forming member 100 is then opposed to the underside of the intaglio 150. The forming material of the substrate 160 is, for example, a thermoplastic resin or a photocurable resin. The underside of the intaglio 150 is then pressed onto the surface 111 of the substrate 160 while the substrate 160 has fluidity. The intaglio 150 is then released from the surface 111 of the substrate 160 while the fluidity of the substrate 160 is suppressed. As a result, a recession portion 151 of the intaglio 150 is transcribed onto the surface 111 of the substrate 160 to form a flat portion 130 and a recession and protrusion portion 140.

To the surface of a thermoplastic resin or photocurable resin, which is a forming material of the substrate 160, an organic substance including, for example, an extracellular matrix such as laminin, collagen, gelatin, fibronectin, polylysine (PDL or PLL),or hyaluronic acid, a polymer, or an adhesion factor such as a gel may be applied for the purpose of enhancing the adhesion of the cells. A biomaterial such as a polysaccharide or a protein may also be used as the forming material of the substrate 160.

<Production method of cultured cell sheet>

A cell sheet produced using the cell sheet-forming member 100 will be described.

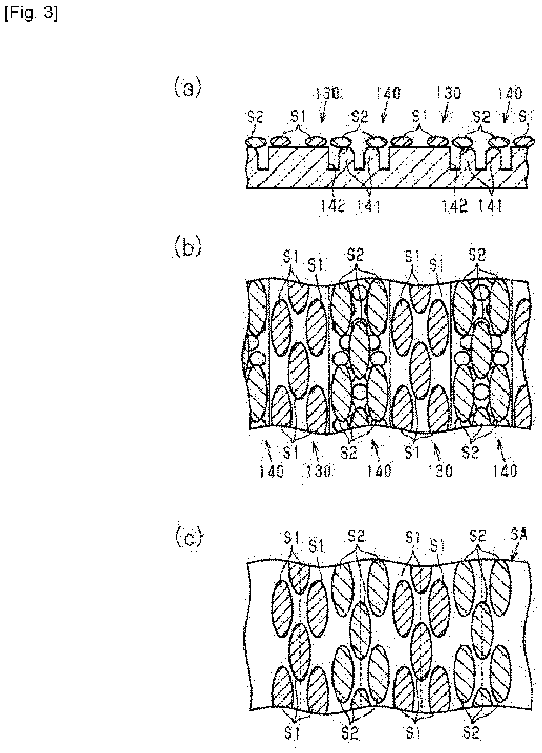

In a cell sheet forming substrate satisfying the above (A), as shown in Fig. 3(a), a cell in the cell suspension retained in the cell sheet-forming member 100 may be a cell S1 that preferentially adheres to the flat portion 130 or a cell S2 that is allowed to adhere to the recession and protrusion portion 140 although the adhesion is inferior to that to the flat portion 130. Alternatively, the cell in a cell suspension retained in the cell sheet-forming member 100 may be a cell S2 that preferentially adheres to the recession and protrusion portion 140 or a cell S1 that is allowed to adhere to the flat portion 130 although the adhesion is inferior to that to the recession and protrusion portion 140.

In this case, as shown in Fig. 3(b), the flat portion 130 and the recession and protrusion portion 140 extend in the first direction and are alternately arranged in the second direction. Thus, in the surface 111 of the cell sheet-forming member, for example, the alignment of the cell S1 that is preferentially adhered to the flat portion 130 is controlled by the structure of the flat portion 130 and the structure of the recession and protrusion portion 140 that compartmentalizes the flat portion 130.

In the recession and protrusion portion 140 sandwiched between the flat portions 130 adjacent to each other, the control of the alignment by the flat portion 130 is reflected in the cell S2 adhered to the recession and protrusion portion 140 although the adhesion is inferior to that to the flat portion 130. As a result, as shown in Fig. 3(c), a cultured cell sheet SA in which the cells S1 and S2 whose alignments are controlled in the first direction are extended across the surface 111 is formed.

Alternatively, the alignment of the cell S2 that is preferentially adhered to the recession and protrusion portion 140 is controlled by the structure of the recession and protrusion portion 140 and the structure of the flat portion 130 that compartmentalizes the recession and protrusion portion 140. In the flat portion 130 sandwiched between the recession and protrusion portions 140 adjacent to each other, the control of the alignment by the recession and protrusion portion 140 is reflected in cell S1 adhered to the flat portions 130 although the adhesion is inferior to that to the recession and protrusion portion 140. As a result, as shown in Fig. 3(c), a cultured cell sheet SA in which the cells S1 and S2 whose alignments are controlled in the first direction are extended across the surface 111 is formed.

[Method for evaluating compound or drug]

The method for evaluating a compound or drug of the present embodiment includes allowing a compound or drug to be evaluated to act on the cultured cell sheet described above.

Preferably, the compound or drug to be evaluated is preferably allowed to act on the cultured cell sheet to evaluate at least one change selected from the group consisting of a change in physiological properties and a change in motor function of the cultured cell sheet, thereby evaluating the compound or drug. Examples of the change in physiological properties include a change in BR, Duration, Interval, peak height (Intensity) of calcium transients, or the like in the above-described observation of calcium transients, and examples of the change in motor function include a change in BR, CV, RV, CRD, contraction duration, relaxation duration, or the like in the above-described observation of live cell imaging.

Although the number of days in culture of the cultured cell sheet for the evaluation is not particularly limited, it is preferable to carry out the evaluation using a cultured cell sheet which requires about 15 days of culture since the maturity of cardiomyocytes is higher.

The compound or drug to be evaluated is not particularly limited, but is preferably an existing compound known to act on cardiomyocytes or a candidate compound thereof, and preferred examples thereof include at least one existing compound selected from the group consisting of an INa blocker, an Ikr blocker, an Iks blocker, an ICa blocker, a 5-HT4 receptor agonist, an alpha-receptor blocker, a beta-receptor blocker, an alpha-receptor agonist, a beta-receptor agonist, a toxic deleterious substance, an anti-cancer drug, a lipid metabolic inhibitor, phosphodiesterase-3 inhibitor, and another bioactive substance (e.g., a protein, a peptide, an antibody, DNA, RNA, etc.) or a candidate compound thereof.

Examples of the existing INa blocker include quinidine and propranolol.

Examples of the existing Ikr blocker include E-4031, quinidine, sotarol, cisapride, and bepridil.

Examples of the existing Iks blocker include bepridil and chromanol.

Examples of an existing beta blocker include sotarol, propranolol, and carvedilol.

Examples of an existing alpha blocker include carvedilol.

Examples of the existing beta-receptor agonist include isoproterenol.

Examples of the existing phosphodiesterase-3 inhibitor include milrinone.

[Method for evaluating quality of cultured cell sheet]

According to the method for evaluating the quality of a cultured cell sheet of the present embodiment, provided is a method for evaluating the quality of a cultured cell sheet comprising cardiomyocytes as to whether the cultured cell sheet has matured and can be suitably used as a cultured cell sheet for regenerative medicine or in a method for evaluating a compound or drug. In addition, the method for producing a cultured cell sheet includes the quality evaluation method above as one step, thereby providing a cultured cell sheet with superior quality. In other words, the above quality evaluation method is preferably incorporated in the present embodiment as one process of the method for producing a cultured cell sheet.

The method for evaluating quality of a cultured cell sheet preferably satisfies at least one of the following (i) to (xii), more preferably at least (i) and at least one of (ii) to (xii), and even more preferably all of (i) to (xii):

(i) a degree of alignment determined according to the following formula (1) is 23% or more:

Degree of alignment (%) = (number of rod-shaped structures included within $\pm 15°$ of mode)/(total number of rod-shaped structures) $\times$ 100 (1)

wherein in an image obtained by microscopy of the cultured cell sheet immunostained with an anti-$\alpha$-actinin antibody, where a screen horizontal direction is regarded as 0°, the degree of alignment is a frequency of rod-shaped structures included within $\pm 15°$ of the mode of angles which are measured in a longitudinal direction of the rod-shaped structures detected with an $\alpha$-actinin antibody within a measurement range of 71.6 $\mu$m $\times$ 71.6 $\mu$m,
(ii) the number of vectors (degree of alignment) showing an angle of $\pm 5°$ from an angle of the mode of motion vectors is 12% or more in live cell imaging,
(iii) the cultured cell sheet satisfies at least one of the following requirements A1 and A2:

Requirement A1: a ratio of an expression level of myosin light chain 3 (MYL3) gene to an expression level of actin beta (ACTB) gene, MYL3/ACTB, is 12.0 or more, wherein MYL3/ACTB is preferably 12.0 or more after day 7 of culture; and
Requirement A2: a ratio of an expression level of a myosin heavy chain 7 (MYH7) gene to an expression level of a myosin heavy chain 6 (MYH6) gene, MYH7/MYH6, is 6.0 or more, wherein MYH7/MYH6 is preferably 6.0 or more after day 7 of culture,

(iv) the cultured cell sheet satisfies at least one of the following requirements B1 and B2:

Requirement B1: a ratio of an expression level of creatine kinase M-type (CKM) gene to an expression level of actin beta (ACTB) gene, CKM/ACTB, is 1.80 or more, wherein CKM/ACTB is preferably 1.80 or more on day 15 to day 45 of culture; and
Requirement B2: a ratio of an expression level of a lactate dehydrogenase A subunit (LDHA) gene to an expression level of ACTB gene, LDHA/ACTB, is 0.80 or more, wherein LDHA/ACTB is preferably 0.80 or more, and more preferably 1.2 or more after day 7 of culture,

(v) the cultured cell sheet satisfies at least one of the following requirements C1 to C4:

Requirement C1: a ratio of an expression level of calcium voltage-gated channel auxiliary subunit alpha2delta1 (CACNA2D1) gene to an expression level of ATPase Na+/K+ transporting subunit alpha 1 (ATP1A1) gene, CACNA2D1/ATP1A1, is 0.085 or more on any of days 15 to 30 of culture;
Requirement C2: a ratio of an expression level of potassium inwardly rectifying channel subfamily J member 2 (KCNJ2) gene to an expression level of ATPase Na+/K+ transporting subunit alpha 1 (ATP1A1) gene, KCNJ2/ATP1A1, is 0.038 or more on any of days 15 to 30 of culture;
Requirement C3: a ratio of an expression level of potassium voltage-gated channel subfamily E regulatory subunit 1 (KCNE1) gene to an expression level of ATPase Na+/K+ transporting subunit alpha 1 (ATP1A1) gene, KCNE1/ATP1A1, is 0.003 or more on any of days 15 to 30 of culture; and
Requirement C4: a ratio of an expression level of sodium voltage-gated channel alpha subunit 5 (SCN5A) gene to an expression level of ATPase Na+/K+ transporting subunit alpha 1 (ATP1A1) gene, SCN5A/ATP1A1, is 0.87 or more on any of days 15 to 30 of culture,

(vi) the cultured cell sheet satisfies at least one of the following requirements D1 to D4:

Requirement D1: a ratio of an expression level of lipoprotein lipase (LPL) gene to an expression level of actin beta (ACTB) gene, LPL/ACTB, is 0.45 or more on any of days 15 to 45 of culture;
Requirement D2: a ratio of an expression level of acetyl-CoA acetyltransferase 1 (ACAT1) gene to an expression level of actin beta (ACTB) gene, ACAT1/ACTB, is 0.43 or more on any of days 15 to 30 of culture;
Requirement D3: a ratio of an expression level of hydroxyacyl-CoA dehydrogenase trifunctional multienzyme complex subunit alpha (HADHA) gene to an expression level of actin beta (ACTB) gene, HADHA/ACTB, is 0.75 or more on any of days 15 to 30 of culture; and
Requirement D4: a ratio of an expression level of hydroxyacyl-CoA dehydrogenase trifunctional multienzyme complex subunit beta (HADHB) gene to an expression level of actin beta (ACTB) gene, HADHB/ACTB, is 0.95 or more on any of days 15 to 30 of culture,

(vii) the cultured cell sheet satisfies at least one of the following requirements E1 to E8:

Requirement E1: a ratio of an expression level of PPARG (peroxisome proliferator-activated receptor gamma) coactivator 1 alpha) (PPARGC1A) gene to an expression level of hypoxia inducible factor 1 alpha (HIF1A) gene, PPARGC1A/HIF1A, is 0.80 or more on any day of culture;
Requirement E2: a ratio of an expression level of estrogen related receptor alpha (ESRRA) gene to a gene expression level of hypoxia inducible factor 1 alpha (HIF1A), ESRRA/HIF1A, is 0.55 or more on any day of culture;
Requirement E3: a ratio of an expression level of vascular endothelial growth factor A (VEGFA) gene to an expression level of hypoxia inducible factor 1 alpha (HIF1A) gene, VEGFA/HIF1A, is 0.65 or more on any day of culture;
Requirement E4: a ratio of an expression level of apelin (APLN) gene to an expression level of hypoxia inducible factor 1 alpha (HIF1A) gene, APLN/HIF1A, is 0.005 or more on any day of culture;
Requirement E5: a ratio of an expression level of fatty acid binding protein 3 (FABP3) gene to an expression level of hypoxia inducible factor 1 alpha (HIF1A) gene, FABP3/HIF1A, is 5.0 or more on any day of culture;
Requirement E6: a ratio of an expression level of endothelial cell specific molecule 1 (ESM1) gene to an expression level of hypoxia inducible factor 1 alpha (HIF1A) gene, ESM1/HIF1A, is 0.002 or more on any day of culture;
Requirement E7: a ratio of an expression level of endomucin (EMCN) gene to an expression level of hypoxia inducible factor 1 alpha (HIF1A) gene, EMCN/HIF1A, is 0.002 or more on any day after day 7 of culture; and
Requirement E8: a ratio of an expression level of BCL2 apoptosis regulator (BCL2) gene to an expression level of hypoxia inducible factor 1 alpha (HIF1A) gene, BCL2/HIF1A, is 0.013 or more on any day after day 7 of culture,

(viii) an area of mitochondria per cell is 200 $\mu$m$^2$ or more,
(ix) the cultured cell sheet satisfies the following formula (2):

$$1.0 \leq CV/RV \leq 2.8 \qquad (2)$$

wherein CV (m/sec) and RV (m/sec) denote contractile velocity and relaxation velocity, respectively, detected in live cell imaging.
(x) the cultured cell sheet, which satisfies the following formula (3):

$$\text{Duration}/(\text{Interval})^{1/2} \geq 0.68 \qquad (3)$$

wherein in calcium imaging analysis, when a waveform peak height of a calcium transient is 100%, Duration (sec) denotes a waveform width at a height of 20%, and Interval (sec) denotes an interval between peaks,
(xi) a cell constituting the cultured cell sheet has an action potential duration at 80% repolarization of 600 msec or more and a maximum diastolic potential of -60 mV or less in a patch-clamp test in a solution at 25°C, and
(xii) in an oxygen consumption rate measurement for measuring a rate at which a cell uses oxygen contained in a culture solution, an addition of a $\beta$-oxidation inhibitor that inhibits $\beta$-oxidation activity of a fatty acid decreases an oxygen consumption rate to 20% or less as compared with that before the addition of the $\beta$-oxidation inhibitor.

Examples

Hereinafter, Examples are given to specifically illustrate the present invention, but the present invention is not limited to these Examples.

(1) Culture-1 of iPS cell-derived cardiomyocytes (CM1)

iCell cardiomyocytes (iCell Cardiomyocyte v1.0, manufactured by FUJIFILM Cellular Dynamics, Inc.) were used as human iPS cell-derived cardiomyocytes (hereinafter referred to as CM1). The culture was performed by the following procedure.
Frozen cell samples were warmed in a 37°C water bath for 3 minutes and lysed. The cell solution was transferred to a 50 mL centrifuge tube, and 9 mL of plating medium (iCell cardiomyocyte thawing medium, manufactured by FUJIFILM Cellular Dynamics, Inc.) prewarmed to 37°C was added thereto and mixed to dilute the cell solution.
A portion of the diluted cell suspension was collected, mixed with an equal amount of trypan blue, and the viable cells were counted using a hemocytometer. The cells were adjusted to a concentration of $1 \times 10^6$ cells/2 mL ($= 5 \times 10^5$/mL) using a plating medium, and then each 2 mL was seeded into a 6-well plate coated with gelatin in advance.

The gelatin coating of the culture vessel was performed by adding a sterilized 0.1% gelatin solution to the vessel (2 mL/well) and allowing to stand at 37°C for 1 hour. The gelatin solution was removed prior to use and the cells were seeded.

The cells after seeding were allowed to stand in a 37°C $CO_2$ incubator, and the culture medium was exchanged on day 3 of culture with setting the day of seeding as day 0.

The cells were peeled off with trypsin and collected on day 5 of culture for reseeding. A portion of the collected cell suspension was taken, mixed with an equal amount of trypan blue, and the viable cells were counted. The cells were adjusted for concentration using a plating medium, and then each 0.1 mL was seeded at a concentration of $8 \times 10^4$ cells/well into a 96-well plate coated in advance by adding and applying 40 μL of 0.05 mg/mL fibronectin for 2 hours or more. The 96-well plates used were an ND Cell Aligner (aligned plate, 96-well plate type) and a commercially available 96-well plate (planar plate) having a flat culture surface as a control.

The cells after seeding were allowed to stand for 4 hours in a 37°C $CO_2$ incubator, and the medium was exchanged every 2 days using a maintenance medium (iCell cardiomyocyte maintenance medium, manufactured by FUJIFII,M Cellular Dynamics, Inc.), and the maintenance culture was performed for a predetermined period of time.

(2) Culture-2 of iPS cell-derived cardiomyocytes (CM2)

iCell cardiomyocytes (iCell Cardiomyocyte v2.0, manufactured by FUJIFILM Cellular Dynamics, Inc.) were used as human iPS cell-derived cardiomyocytes (hereinafter referred to as CM2). The culture was performed by the following procedure.

The frozen cell sample was warmed in a 37°C water bath for 3 minutes and lysed. The cell solution was transferred to a 50 mL centrifuge tube, and 9 mL of plating medium prewarmed to 37°C was added thereto and mixed to dilute the cell solution. A portion of the diluted cell suspension was collected, mixed with an equal amount of trypan blue, and the viable cells were counted using a hemocytometer.

The cell concentration was adjusted using a plating medium and the cells were seeded at a concentration of $6 \times 10^4$ cells/well into a 96-well plate coated in advance with fibronectin. The 96-well plates into which the cells were seeded were an ND Cell Aligner (aligned plate, 96-well plate type) and a commercially available 96-well plate (planar plate) having a flat culture surface as a control.

After allowing the well plate to stand for 4 hours in a 37°C $CO_2$ incubator, the medium was exchanged using maintenance medium. The cells after the medium exchange were subjected to the medium exchange every 2 days, followed by the maintenance culture for a predetermined period.

The ND Cell Aligner used in (1) and (2) above had flat portions and recession and protrusion portions, in which each flat portion had a shape extending in a first direction and flat portions were arrayed in a second direction intersecting the first direction over an entirety of the surface, and the width (length in the second direction) of each flat portion was 10 μm. Each recession and protrusion portion was composed of a plurality of stepped structures filling a gap between flat portions adjacent to each other, the length between respective flat portions in the second direction was 10 μm, and the pitch of the protrusion portion in the recession and protrusion portion was 300 nm. As a result of measuring the height of each protrusion portion in the recession and protrusion portion using AFM, the average height from the bottom surface of the recession portion to the distal end of the protrusion portion was 446 nm. The average height of the flat portion from the bottom surface of the recession portion was 455 nm.

(3) Analysis of alignment

Using maintenance-cultured iCell cardiomyocytes CM2, the cells on days 15, 30, 45 and 60 were immunostained with an anti-α-actinin antibody for a sarcomere structure and stained with Hoechst 33342 for nuclei. A fluorescence microscope was used to obtain a fluorescence image.

Immunostaining was performed by the following procedure. The medium was removed from the well plate cultured for predetermined days and the cells were washed with PBS. Then, 4% paraformaldehyde was added to fix the cells. After washing the cells with PBST (0.1% tween 20), an anti-α-actinin antibody (manufactured by Abcam plc., Anti-Sarcomeric Alpha Actinin antibody [EA-53]) was added as a primary antibody, and allowed to react at 4°C overnight. After washing with PBST (0.1% tween 20) to remove the primary antibody, a secondary antibody (Alexa Fluor 647 labeled, manufactured by Life Technologies Corporation, Alexa Fluor(R) 647 F{ab}$_2$ fragment of goat anti mouse IgG (H+L)) was added to react. Subsequently, Hoechst 33342 diluted was added to stain nuclei.

The fluorescence image was obtained at 60× (objective lens), and the sarcomere was detected at an excitation wavelength of 640 nm/fluorescence wavelength of 685 nm, and the nuclei were detected at an excitation wavelength of 405 nm/fluorescence wavelength of 461 nm.

From the image (size: area of 215 μm × 215 μm) observed with a 60× objective lens, a rod-shaped structure detected with α-actinin was detected by angular distribution analysis using an image analysis software (A-image-kun, manufactured by Asahi Kasei Engineering Corporation), and the angular distribution (0 to 180°) of the rod-shaped structure was determined with setting the horizontal direction of the image as 0°.

From the number of the rod-shaped structures included in the range of ±15° of the angle of the mode of the angle distribution and the total number of the rod-shaped structures included in the measurement range, the degree of alignment was determined by the following equation.

Degree of alignment (%) = (number of rod-shaped structures included within ±15° of the mode)/(total number of rod-shaped structures) × 100

The image analysis was performed at n = 3 to 6, and the average value was determined. On days 15 and 30 of the number of days in culture, the analysis was performed using images (upper left section of nine equally divided sections) obtained from a plurality of different wells. On days 45 and 60 of the number of days in culture, the images obtained from one well were divided into nine equal parts, and analysis was performed using three sections (upper left, middle, and lower right sections).

The results of the angle distribution analysis were shown as the degrees of alignment of the rod-shaped structures included every 10°, and the degrees of alignment in the range of ±15°, including the highest values of the aligned plate and the planar plate, were compared. For the statistical test, a T-test was performed for the degrees of alignment of the aligned plate and the planar plate, and it was confirmed that there was a significant difference by the hazard rate of 5% in the degree of alignment of the aligned plate and the planar plate after 30 days of culture.

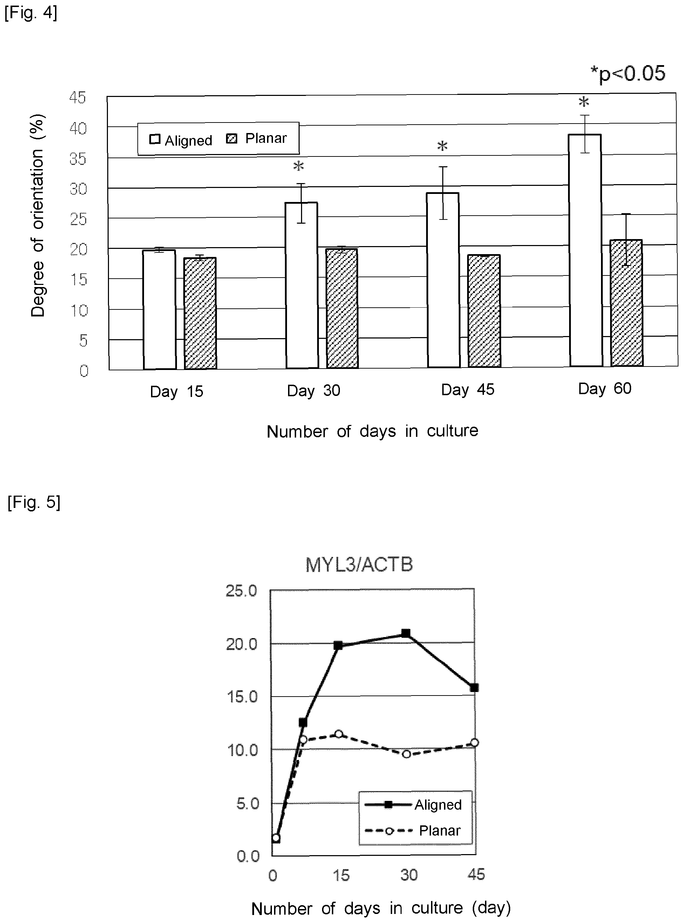

As a result of measuring the degree of alignment, after day 15 of culture, the degree of alignment of CM2 cultured in the aligned plate was higher than that of CM2 cultured in the planar plate. On days 30 to 60 of the number of days in culture, the degree of alignment of the aligned plate was 23% or more. On the other hand, the degree of alignment of the planar plate was less than 21%. The results are shown in Fig. 4.

(4) Maturation confirmation test (gene expression)

Cells of iCell cardiomyocytes (CM1) cultured in the aligned plate and the planar plate were collected on days 1, 7, 15, 30, and 45 of the number of days in culture, and gene expression analysis was performed.

The collected cells were crushed to extract RNA (using NucleoSpin RNA Plus XS manufactured by Takara Bio Inc.), and reverse transcription and library creation were performed using SMART-Seq v4 Low Input Kit. Next, it was subjected to RNA sequencing (comprehensive gene expression analysis) to obtain gene expression data. The analysis program used was RaNA-seq (Reference 3: Bioinformatics, 2020 Mar 10: 36(6), 1955-1956). Characteristic gene expression changes in the maturation stage of human iPS cell-derived myocardium (Reference 4: Circ. Res. 2020 Apr 10: 126(8), 1086-1106) were used as evaluation indicators to compare the changes over time in the gene expression levels of cardiomyocytes cultured in the aligned plate and planar plate.

For the samples used for RNA sequencing in the (4-1) to (4-3) below, RNA extracted from 5 wells were mixed in equal amounts (by mass of RNA) and used for the experiment. The results of gene expression analysis by RNA sequencing is considered to be the average value of 5 wells.

For the samples used for RNA sequencing in the (4-4) to (4-6) below, RNA extracted from 3 wells were subjected to RNA sequencing, respectively, and the average value of the expression levels (CPM) was determined. On each day of the culture, a T-test was performed for the expression level (CPM) of each gene to detect the difference in gene expression level between the planar plate and the aligned plate. When the P value was $0.01 \leq P < 0.05$, * was written in the figure, and when the P value was $P < 0.01$, ** was written in the figure.

As a result of gene expression analysis by RNA sequencing, the following results were obtained.

(4-1) MYL3/ACTB

The gene expression level of myosin light chain 3 (MYL3) expressed in mature myocardium was increased in CM1 cultured in the aligned plate compared to CM1 cultured in the planar plate. On the other hand, the expression level of the Actin-β (ACTB) gene, which is assumed to be expressed at constant level regardless of tissue and culture period, was at the same level between CM1 cultured in the aligned plate and CM1 cultured in the planar plate.

In each plate, the ratio of the expression level of the MYL3 gene to the expression level of the ACTB gene after day 7 of culture was 12.5 or more in the aligned plate and 11.4 or less in the planar plate (Fig. 5).

(4-2) MYH7/MYH6

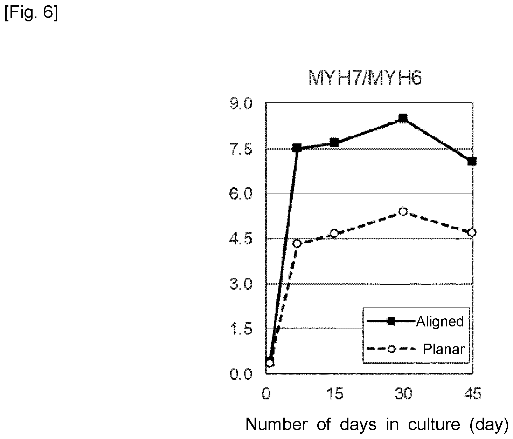

It was confirmed that, as the isoform change accompanying the maturation from the fetal heart muscle to the adult heart muscle, the isoform switching from myosin heavy chain 6 (MYH6) to myosin heavy chain 7 (MYH7) is significant. In each plate, the ratio of the expression level of the MYH7 gene to the expression level of the MYH6 gene after day 7 of culture was 7.1 or more in CM1 cultured in the aligned plate and 5.4 or less in CM1 cultured in the planar plate (Fig. 6).

(4-3) CKM, CKM/ACTB, COX6A2, COX6A2/ACTB, CKMT2, CKMT2/ACTB, LDHA, LDHA/ACTB

The expression levels of creatine kinase, M-type (CKM) that is related to myocardial contraction; cytochrome C oxidase subunit 6A2 (COX6A2) and creatine kinase, mitochondrial 2 (CKMT2) that are proteins localized in mitochondria; and lactate dehydrogenase A subunit (LDHA) that is an enzyme in glycolysis were increased over time in CM1 cultured in the aligned plate compared to CM1 cultured in the planar plate (Figs. 7-1 and 7-2).

The expression level of the CKM gene after day 15 of culture was 1161 transcripts per kilobase million (TPM) or more in CM1 cultured in the aligned plate while 824 TPM or less in CM1 cultured in the planar plate (Fig. 7-1(A)).

The expression level of the COX6A2 gene after day 15 of culture was 875 TPM or more in CM1 cultured in the aligned plate while 842 TPM or less in CM1 cultured in the planar plate (Fig. 7-1(B)).

The expression level of the CMKT2 gene after day 30 of culture was 377 TPM or more in CM1 cultured in the aligned plate while 325 TPM or less in CM1 cultured in the planar plate (Fig. 7-1(C)).

The expression level of the LDHA gene after day 7 of culture was 682 TPM or more in CM1 cultured in the aligned plate while 372 TPM or less in CM1 cultured in the planar plate (Fig. 7-1(D)).

In each plate, the ratio of the expression level of the CKM gene/the expression level of the ACTB gene after day 15 of culture was 2.1 or more in CM1 cultured in the aligned plate and 1.7 or less in CM1 cultured in the planar plate (Fig. 7-2(A)).

The ratio of the expression level of the COX6A2 gene/the expression level of the ACTB gene after day 15 of culture was 1.95 or more in CM1 cultured in the aligned plate and 1.92 or less in CM1 cultured in the planar plate (Fig. 7-2(B)).

The ratio of the expression level of the CKMT2 gene/the expression level of the ACTB gene on day 15 to day 30 of culture was 0.68 or more in CM1 cultured in the aligned plate and 0.57 or less in CM1 cultured in the planar plate (Fig. 7-2(C)).

Furthermore, the ratio of the expression level of the LDHA gene/the expression level of the ACTB gene after day 7 of culture was 1.3 or more in CM1 cultured in the aligned plate and 0.6 or less in CM1 cultured in the planar plate (Fig. 7-2(D)).

(4-4) CACNA2D1, CACNA2D1/ATP1A1, KCNJ2, KCNJ2/ATP1A1, KCNE1, KCNE1/ATP1A1, SCNSA, SCN5A/ATP1A1

The expression levels of calcium voltage-gated channel auxiliary subunit alpha2delta1 (CACNA2D1) that is related to the formation of calcium ion channels in myocardium; potassium inwardly rectifying channel subfamily J member2 (KCNJ2) and potassium voltage-gated channel subfamily E regulatory subunit 1 (KCNE1) that are related to the formation of potassium ion channels in myocardium; and sodium voltage-gated channel alpha subunit 5 (SCN5A) that is related to the formation of sodium ion channels in myocardium were increased over time in CM1 cultured in the aligned plate compared to CM1 cultured in the planar plate (Figs. 7-3 and 7-4).

The expression level of the CACNA2D1 gene on day 15 to day 30 of culture was 2469 count per million (CPM) or more in CM1 cultured in the aligned plate while 2004 CPM or less in CM1 cultured in the planar plate (Fig. 7-3(A)).

The expression level of the KCNJ2 gene after day 15 of culture was 1026 CPM or more in CM1 cultured in the aligned plate while 926 CPM or less in CM1 cultured in the planar plate (Fig. 7-3(B)).

The expression level of the KCNE1 gene on day 15 to day 45 of culture was 79 CPM or more in CM1 cultured in the aligned plate while 42 CPM or less in CM1 cultured in the planar plate (Fig. 7-3(C)).

The expression level of the SCN5A gene on day 15 to day 45 of culture was 23580 CPM or more in CM1 cultured in the aligned plate while 21571 CPM or less in CM1 cultured in the planar plate (Fig. 7-3(D)).

In each plate, the ratio of the expression level of the CACNA2D1 gene/the expression level of the ATP1A1 gene on day 15 to day 30 of culture was 0.085 or more in CM1 cultured in the aligned plate and 0.23 or less in CM1 cultured in the planar plate (Fig. 7-4(A)).

The ratio of the expression level of the KCNJ2 gene/the expression level of the ATP1A1 gene on day 15 to day 30 of culture was 0.038 or more in CM1 cultured in the aligned plate and 0.115 or less in CM1 cultured in the planar plate (Fig. 7-4(B)).

The ratio of the expression level of the KCNE1 gene/the expression level of the ATP1A1 gene after day 15 of culture was 0.003 or more in CM1 cultured in the aligned plate and 0.007 or less in CM1 cultured in the planar plate (Fig. 7-4(C)).

Furthermore, the ratio of the expression level of the SCN5A gene/the expression level of the ATP1A1 gene on day 15 to day 30 of culture was 0.87 or more in CM1 cultured in the aligned plate and 2.6 or less in CM1 cultured in the planar plate (Fig. 7-4(D)).

(4-5) LPL, LPL/ACTB, ACAT1, ACAT1/ACTB, HADHA, HADHA/ACTB, HADHB, HADHB/ACTB

The expression levels of lipoprotein lipase (LPL), acetyl-CoA acetyltransferase 1 (ACAT1), hydroxyacyl-CoA dehydrogenase trifunctional multienzyme complex subunit alpha (HADHA), and hydroxyacyl-CoA dehydrogenase trifunctional multienzyme complex subunit beta (HADHB) that are localized in mitochondria of myocardium and related to β-oxidation of fatty acids were increased over time in CM1 cultured in the aligned plate compared to CM1 cultured in the planar plate (Figs. 7-5 and 7-6).

The expression level of the LPL gene after day 15 of culture was 3832 count per million (CPM) or more in CM1 cultured in the aligned plate while 3024 CPM or less in CM1 cultured in the planar plate (Fig. 7-5(A)).

The expression level of the ACAT1 gene on day 30 to day 45 of culture was 3663 CPM or more in CM1 cultured in the aligned plate while 3357 CPM or less in CM1 cultured in the planar plate (Fig. 7-5(B)).

The expression level of the HADHA gene on day 30 to day 45 of culture was 6240 CPM or more in CM1 cultured in the aligned plate while 5920 CPM or less in CM1 cultured in the planar plate (Fig. 7-5(C)).

The expression level of the HADHB gene after day 15 of culture was 8505 CPM or more in CM1 cultured in the aligned plate while 8072 CPM or less in CM1 cultured in the planar plate (Fig. 7-5(D)).

In each plate, the ratio of the expression level of the LPL gene/the expression level of the ACTB gene after day 15 of culture was 0.46 or more in CM1 cultured in the aligned plate and 0.43 or less in CM1 cultured in the planar plate (Fig. 7-6(A)).

The ratio of the expression level of the ACAT1 gene/the expression level of the ACTB gene on day 15 to day 30 of culture was 0.47 or more in CM1 cultured in the aligned plate and 0.42 or less in CM1 cultured in the planar plate (Fig. 7-6(B)).

The ratio of the expression level of the HADHA gene/the expression level of the ACTB gene on day 15 to day 30 of culture was 0.79 or more in CM1 cultured in the aligned plate and 0.74 or less in CM1 cultured in the planar plate (Fig. 7-6(C)).

Furthermore, the ratio of the expression level of the HADHB gene/the expression level of the ACTB gene on day 15 to day 30 of culture was 1.00 or more in CM1 cultured in the aligned plate and 0.91 or less in CM1 cultured in the planar plate (Fig. 7-6(D)).

(4-6) PPARGC1A, PPARGC1A/HIF1, ESRRA, ESRRA/HIF1, VEGFA, VEGFA/HIF1, APLN, APLN/HIF1, FABP3, FABP3/HIF1, ESM1, ESM1/HIF1, EMCN, EMCN/HIF1, BCL2, BCL2/ACTB

The expression levels of the PPARG coactivator 1 alpha (PPARGC1A) gene encoding PGC-1α related to mitochondrial biosynthesis and functionalization; the estrogen related receptor alpha (ESRRA) gene encoding estrogen-related receptor ERR related to cardiomyocyte maturation; the vascular endothelial growth factor A (VEGFA) gene encoding vascular growth factor; the apelin (APLN) gene encoding myokine related to cardiac contraction in myocardiac tissues; the fatty acid binding protein 3 (FABP3) gene encoding myokine that regulates the uptake of long-chain fatty acids in myocardium; the endothelial cell specific molecule 1 (ESM1) gene that is specifically expressed in endothelial cells; the endomucin (EMCN) gene encoding mucin-like glycoprotein secreted from epithelial cells; and the BCL2 apoptosis regulator (BCL2) gene related to the regulation of mitochondrial function were increased over time in CM1 cultured in the aligned plate compared to CM1 cultured in the planar plate (Figs. 7-7 to 7-10).

The expression level of the PPARGC1A gene after day 15 of culture was 4577 count per million (CPM) or more in CM1 cultured in the aligned plate while 4282 CPM or less in CM1 cultured in the planar plate (Fig. 7-7(A)).

The expression level of the ESRRA gene after day 15 of culture was 3081 CPM or more in CM1 cultured in the aligned plate and 3061 CPM or less in CM1 cultured in the planar plate (Fig. 7-7(B)).

The expression level of the VEGFA gene after day 7 of culture was 3737 CPM or more in CM1 cultured in the aligned plate while 3237 CPM or less in CM1 cultured in the planar plate (Fig. 7-7(C)).

The expression level of the APLN gene after day 7 of culture was 28.7 CPM or more in CM1 cultured in the aligned plate while 20.7 CPM or less in CM1 cultured in the planar plate (Fig. 7-7(D)).

The expression level of the FABP3 gene after day 15 of culture was 28788 CPM or more in CM1 cultured in the aligned plate while 28529 CPM or less in CM1 cultured in the planar plate (Fig. 7-9(A)).

The expression level of the ESM1 gene after day 30 of culture was 40 CPM or more in CM1 cultured in the aligned plate while 1.3 CPM or less in CM1 cultured in the planar plate (Fig. 7-9(B)).

The expression level of the EMCN gene after day 15 of culture was 24.6 CPM or more in CM1 cultured in the aligned plate while 1 CPM or less in CM1 cultured in the planar plate (Fig. 7-9(C)).

The expression level of the BCL2 gene after day 7 of culture was 83.3 CPM or more in CM1 cultured in the aligned plate while 58.3 CPM or less in CM1 cultured in the planar plate (Fig. 7-9(D)).

For each plate, the ratio of the expression level of the PPARGC1A gene/the expression level of the HIF1 gene after day 7 of culture was 0.86 or more in CM1 cultured in the aligned plate and 0.80 or less in CM1 cultured in the planar plate (Fig. 7-8(A)).

The ratio of the expression level of the ESRRA gene/the expression level of the HIF 1 gene after day 7 of culture was 0.57 or more in CM1 cultured in the aligned plate and 0.54 or less in CM1 cultured in the planar plate (Fig. 7-8(B)).

The ratio of the expression level of the VEGFA gene/the expression level of the HIF 1 gene after day 7 of culture was 0.71 or more in CM1 cultured in the aligned plate and 0.64 or less in CM1 cultured in the planar plate (Fig. 7-8(C)).

Furthermore, the ratio of the expression level of the APLN gene/the expression level of the HIF1 gene after day 7 of culture was 0.013 or more in CM1 cultured in the aligned plate and 0.004 or less in CM1 cultured in the planar plate (Fig. 7-8(D)).

Furthermore, the ratio of the expression level of the FABP3 gene/the expression level of the HIF1 gene after day 7 of culture was 5.3 or more in CM1 cultured in the aligned plate and 5.2 or less in CM1 cultured in the planar plate (Fig. 7-10(A)).

Furthermore, the ratio of the expression level of the ESM1 gene/the expression level of the HIF1 gene after day 7 of culture was 0.001 or more in CM1 cultured in the aligned plate and 0.0003 or less in CM1 cultured in the planar plate (Fig. 7-10(B)).

Furthermore, the ratio of the expression level of the EMCN gene/the expression level of the HIF1 gene after day 7 of culture was 0.003 or more in CM1 cultured in the aligned plate and 0.001 or less in CM1 cultured in the planar plate (Fig. 7-10(C)).

Furthermore, the ratio of the expression level of the BCL2 gene/the expression level of the HIF1 gene on day 7 to day 30 of culture was 0.018 or more in CM1 cultured in the aligned plate and 0.012 or less in CM1 cultured in the planar plate (Fig. 7-10(D)).

(5) Maturation confirmation test: confirmation of mitochondrial activity

Using maintenance-cultured iCell cardiomyocytes CM2, the cells on day 9 were stained with a membrane potential-dependent fluorescent dye Mito-Tracker Red (manufactured by Thermo Fisher Scientific, Inc., M7510) for mitochondria and with Hoechst 33342 for nuclei. The staining was performed by removing the medium from the well plate, adding Mito-tracker Red diluted to a predetermined concentration with the maintenance medium, allowing the plate to stand in a $CO_2$ incubator at 37°C for 30 minutes, then adding Hoechst 33342 diluted to a predetermined concentration with the maintenance medium, and allowing the plate to stand in a $CO_2$ incubator at 37°C for 30 minutes. Before observation, the cells were washed with medium, and the fluorescent images were obtained using a confocal fluorescence microscope (confocal quantitative image cytometer CQ1, manufactured by Yokogawa Electric Corporation).

The fluorescence image was obtained at magnification of 60× (objective lens), and the mitochondria were detected with an excitation wavelength of 561 nm/fluorescence wavelength of 617 nm, and the nuclei were detected with an excitation wavelength of 405 nm/fluorescence wavelength of 461 nm.

For the analysis of the image, an analysis software (CellPathfinder, manufactured by Yokogawa Electric Corporation) was used. The area and intensity detected with the fluorescence wavelength of 617 nm were measured from the measurement range of the field of view observed at the magnification of 60× of the objective lens (size: area of 215 $\mu$m × 215 $\mu$m), and the number of nuclei detected with the fluorescence wavelength of 461 nm was measured.

The detected value of the fluorescence wavelength of 617 nm is the value detected with a detector of the camera of CQ1, the area is the sum of the area (unit: $\mu m^2$) where the fluorescence wavelength of 617 nm was detected, and the luminance (intensity) is the sum of values of brightness detected by the detector. The luminance (brightness) was measured at full scale under the same conditions.

The analysis was performed at n = 6 to 10. The area and the luminance (intensity) were corrected with the number of nuclei, and the area and the luminance (intensity) per cell were compared. For the statistical test, a T-test was performed, and the significant difference was confirmed with the hazard rate of 1%.

It should be noted that the area represents the number of mitochondria, and the luminance (intensity) represents the activity of mitochondria. The results are shown in Fig. 8. The error bar indicates the standard error.

As shown in Fig. 8, on day 9 of culture, the number of mitochondria and the mitochondrial activity were significantly increased in CM2 cultured in the aligned plate compared to CM2 cultured in the planar plate.

Fig. 9 shows the fluorescence images of CM2 cultured in the aligned plate on day 9 of culture and CM2 cultured in the planar plate on day 9 of culture. Blue indicates the nuclei, and red indicates the mitochondria. The photos also clarify that the number of mitochondria and the mitochondrial activity were increased in CM2 cultured in the aligned plate.

(6) Maturation confirmation test: analysis by motion test method using live cell imaging device

To detect motion changes associated with the contraction and relaxation of cardiomyocytes seeded on each culture plate, video data was obtained using SI8000 (manufactured by Sony Corporation). The measurement was performed under conditions of phase contrast image, magnification of $10\times$ (objective lens), 150 frames/sec, resolution $2048 \times 2048$ pixels, and 8-bit depth. The image acquisition time was 10 seconds. The cells used were CM2 that was maintenance-cultured until days 15, 31, 45 and 61 with setting the day of seeding as day 0.

Motion vectors (velocity, direction, quantity) associated with the contraction and relaxation of cardiomyocytes were detected from the video data, and each parameter of a beating rate (BR), a contractile velocity (CV), a relaxation velocity (RV), a contraction-relaxation duration (CRD) and a degree of alignment was analyzed.

The degree of alignment was calculated based on the following equation by determining the angle of motion vector with setting the horizontal direction of the screen as 0°, and determining a ratio of the number of vectors indicating an angle of $\pm5°$ from an angle of the mode of the vector angle to the sum of the number of vectors detected.

Degree of alignment (%) = (number of vectors included in range of $\pm5°$ of mode)/(total number of vectors) $\times$ 100

Fig. 10 shows an example of the measurement results of the angles indicated by the motion vectors and their frequency.

The results of CM2 in which the cells were seeded in each culture plate and maintenance-cultured are shown in Fig. 11-1.

It was confirmed that the beating rate (BR) and the relaxation velocity (RV) were significantly increased on day 15 to day 61 of culture and the contraction-relaxation duration (CRD) was tended to shorten after day 31 of culture in CM2 cultured in the aligned plate compared to CM2 cultured in the planar plate. The vector directional distribution (mode $\pm5°$) indicating the degree of alignment of cells was maintained significantly high on day 15 to day 61 of culture.

It should be noted that there are tendencies that the higher the beating rate (BR), the closer to mature cardiomyocytes; the faster the contractile velocity (CV) and the relaxation velocity (RV), the closer to mature cardiomyocytes; and the shorter the contraction-relaxation duration (CRD), the closer to mature cardiomyocytes.

The ratio of the contractile velocity (CV) to the relaxation velocity (RV) on this case is shown in Fig. 11-2. The ratio (CV/RV) of the contractile velocity (CV) to the relaxation velocity (RV) was 1.8 to 2.5 for the period of day 15 to day 45 of culture in CM2 cultured in the aligned plate. On the other hand, that was 3.1 to 3.5 in CM2 cultured in the planar plate.

From the result above, the ratio (CV/RV) of the contractile velocity (CV) to the relaxation velocity (RV) in CM2 cultured in the aligned plate falls within the following range.

$$\text{(Equation)} \ 1.0 \leq CV/RV \leq 2.8$$

For the vector directional distribution, the degree of alignment at the mode $\pm1.25°$, $\pm5°$, and $\pm10°$ on day 15 is shown in Table 1. At the mode $\pm1.25°$, the ratio of the aligned plate/the planar plate was the highest, but the ratio of the planar plate to the theoretical value was also the highest, thus it is believed that when the alignment angle to be analyzed is small, the influence of noise will be large. In comparison between the mode $\pm5°$ and the mode $\pm10°$, the ratio of the planar plate to the theoretical value was $\pm5° > \pm10°$, but the ratio of the aligned plate/the planar plate was $\pm5° > \pm10°$. Thus, the degree of alignment of the mode $\pm5°$ was evaluated as a range in which the trend of the degree of alignment is likely to appear.

[Table 1]

Table 1

| | Degree of alignment (%) | | |
|---|---|---|---|
| | Mode $\pm1.25°$ | Mode $\pm5°$ | Mode $\pm10°$ |
| Case of uniform degree of alignment (theoretical value) | 1.38 | 5.55 | 11.11 |
| Planar plate | 3.01 | 8.20 | 13.02 |
| Aligned plate | 12.60 | 24.95 | 35.35 |
| Planar/theoretical value | 2.18 | 1.48 | 1.17 |

(continued)

| | Degree of alignment (%) | | |
|---|---|---|---|
| | Mode ±1.25° | Mode ±5° | Mode ±10° |
| Aligned/theoretical value | 9.13 | 4.50 | 3.18 |
| Aligned/Planar | 4.19 | 3.04 | 2.71 |

(7) Maturation confirmation test: Ca-imaging analysis using confocal imaging device

The change in Ca fluorescence intensity associated with the contraction of cardiomyocytes seeded in each culture plate was measured by CQ1 (confocal fluorescence microscope, manufactured by Yokogawa Electric Corporation).

To visualize Ca ion flux, a fluorescent Ca indicator (EarlyTox carditotoxicity kit, manufactured by Molecular Devices, LLC.) was added to CM2 and allowed to stand at 37°C for 15 minutes. The measurement of CQ1 was performed under conditions of the excitation wavelength of 488 nm/the fluorescence wavelength of 525 nm and the data acquisition time of 60 seconds. For the analysis, CellPathfinder was used.

The cells used were CM2 that was maintenance-cultured until day 5 with setting the day of seeding as day 0. From the measurement data, the line width (duration) of 20% height of Ca transient waveform peak and the interval of the waveform peak were selected as parameters and analyzed.

The line width (duration) of 20% height of the Ca transient waveform peak and the interval of the Ca transient waveform were significantly extended in CM2 cultured in the aligned plate compared to CM2 cultured in the planar plate (Fig. 12(A)).

Fig. 12(B) shows the relationship between the duration and the square root of the interval.

From the above results, the ratio of the line width (duration) of 20% height of the peak to the square root of the interval of the waveform in CM2 cultured in the aligned plate falls within the following range.

$$\mathrm{Duration}/(\mathrm{Interval})^{1/2} \geq 0.68$$

It has been reported that there is a correlation between the Ca transient duration (line width (duration) of 20% height) and the action potential duration. In general, there is a tendency in cultured cardiomyocytes that the higher the degree of differentiation is, the longer the action potential duration is. Thus, it is believed that maturation is more advanced in the aligned plate than the planar plate.

The time of the interval is affected by the beating rate, and the value corrected with the beating rate is usually used, thus the interval was calculated using the value of $(\mathrm{Interval})^{1/2}$.

(8) Maturation confirmation test: measurement of intracellular potential by patch-clamp method

In the above procedure, CM2 was seeded into the aligned plate and the planar plate at a concentration of $6 \times 10^4$ cells/well. At this time, each plate was not a 96-well plate, but small pieces of the bottom film cut out to a size of 4 mm × 4 mm were placed on the bottom surface of a commercially available 96-well plate and used as the plate. In the measurement of intracellular potential by the patch-clamp test, a current of 20 pA to 100 pA was applied at 0.2 Hz into the cells of CM2 in a Tyrode solution at 25°C under a current clamp to generate an action potential. It should be noted that the current applied was selected so that a change in normal potential was observed.

The results of two representative examples are shown in Fig. 13. The results are those of day 8 of culture for CM2 cultured in the aligned plate and day 22 of culture for CM2 cultured in the planar plate. The action potential duration was longer and the maximum diastolic potential was deeper in intracellular potential of CM2 cultured in the aligned plate compared to that of CM2 cultured in the planar plate.

The action potential duration at 80% repolarization was 791 ms and the maximum diastolic potential was -68.6 mV in CM2 cultured in the aligned plate. On the other hand, the action potential duration at 80% repolarization was 478 ms and the maximum diastolic potential was -57.9 mV in CM2 cultured in the planar plate.

Regarding the action potential in human-derived normal isolated ventricular myocytes, the duration is as long as that of CM2 cultured in the aligned plate and the maximum diastolic potential (resting membrane potential) is deeper even than that of CM2 cultured in the aligned plate (Circulation, 2013; 127: 575-584). From this, it can be said that CM2 cultured in the aligned plate has an action potential closer to that of normal cardiomyocytes than CM2 cultured in the planar plate.

(9) Drug safety test-1

The capacity-dependent action of four drugs that are often used to induce side effects on myocardium was investigated using iCell cardiomyocyte CM2.

In the above procedure, CM2 was seeded into the aligned plate (96 well plate, ND Cell Aligner) and the planar plate (96 well plate) at a concentration of $5 \times 10^4$ cells/well. A maintenance culture was performed in a 37°C $CO_2$ incubator, and the drug was administered on day 5 or 6 with setting the day of seeding as day 0.

The drugs used were E-4031, quinidine, cisapride, and isoproterenol. The drug was dissolved in DMSO and cumulatively administered. First, the measurement as dose0 was performed without adding drugs. Immediately after the measurement, the drug dissolved in DMSO was added to each well so that the concentration was dose1, and the measurement was performed after 15 minutes. Subsequently, immediately after the measurement, the drug was added to the same well, respectively so that the concentration was dose2, and the measurement was performed after 15 minutes. The same was done for dose3.

The type, mechanism of action, and concentration of the drugs are shown in the table below.

[Table 2]

Table 2

| Agent | Mechanism of action | drug concentration(μM) | | |
|---|---|---|---|---|
| | | dose1 | dose2 | dose3 |
| E-4031 | Ikr blocker | 0.1 | 0.3 | 1 |
| Quinidine | INa blocker, Ikr blocker | 3 | 10 | 30 |
| Cisapride | Ikr blocker, 5-HT4 receptor agonist | 0.01 | 0.03 | 0.1 |
| Isoproterenol | β receptor agonist | 1 | 3 | 10 |
| DMSO (%) | Solvent | 0.025 | 0.075 | 0.25 |

For the cell responsiveness after the drug administration, an arrhythmia measurement method by Ca-imaging using a confocal imaging device (CQ1, manufactured by Yokogawa Electric Corporation) and a motion test method using a live cell imaging device (SI8000, manufactured by Sony Corporation) were performed to investigate the effect on the beating rate and the line width of the beating peak.

(9-1) Measurement by motion test method using live cell imaging device

The measurement was performed with the following procedure.

CM2 on day 5 of maintenance culture was used, and video data was first obtained as Dose0 using SI8000 (manufactured by Sony Corporation) without adding drugs. Immediately after the measurement, each drug at the concentration of dose1 was added to the same well plate, and video data was obtained after 15 minutes. For Dose2 and Dose3, the drug was cumulatively administered in the same manner, and video data was obtained. The addition test of each drug was performed at n = 6.

The beating rate (BR), the contractile velocity (CV), the relaxation velocity (RV), and the contraction-relaxation duration (CRD) were analyzed from the video data of SI8000, and the effects of the drugs on the motion of the cells were compared. The contraction-relaxation duration (CRD) was corrected by the following equation with reference to the equation of Frederica to correct variation due to the beating rate (BR).

$$CRD/(60/BR)^{1/3}$$

For the significant difference test, a T test was performed between the subject group in which DMSO was added and the drug addition group in the planar plate and the aligned plate.

For the side effects seen from the change in the parameters of SI8000, early after depolarization (EAD) and delayed after depolarization (DAD), small regular waveforms (VT-like), and reaction (waveform) disappearance were recorded. Changes in waveforms not classified into EAD or DAD were described as arrhythmia. The "-" in the table indicates the state in which no arrhythmia or the like has occurred.

The degree of arrhythmia is mild for EAD, moderate for DAD, and severe for vt-like. The degree of those indicated as "arrhythmia" is mild.

E-4031 (Ikr blocker)

The addition of E-4031 reduced the beating rate (BR), the contractile velocity (CV), and the relaxation velocity (RV) in both CM2 cultured in the planar plate and CM2 cultured in the aligned plate. The CRD (corrected with the beating rate) was significantly reduced at medium concentration (Dose2, 0.3 $\mu$M) and high concentration (Dose3, 1 $\mu$M) in the aligned plate and the planar plate (Fig. 14(A)). In the planar plate, the CRD was reduced more remarkably.

In CM2 cultured in the planar plate, arrhythmia occurred in 6 of 6 wells, but in CM2 cultured in the aligned plate, arrhythmia occurred in 1 of 6 wells (Table 3-1, Table 3-2).

The incidence of arrhythmia in CM2 cultured in the aligned plate was 1/6 at low concentration (Dose1, 0.1 $\mu$M), 1/6 at medium concentration (Dose2, 0.3 $\mu$M), and 1/6 at high concentration (Dose3, 1 $\mu$M). On the other hand, that in CM2 cultured in the planar plate was 3/6 at low concentration (Dose1, 0.1 $\mu$M), 5/6 at medium concentration (Dose2, 0.3 $\mu$M), and 6/6 at high concentration (Dose3, 1 $\mu$M). Thus, the incidence of arrhythmia tended to be low in CM2 cultured in the aligned plate and high in CM2 cultured in the planar plate.

[Table 3-1]

Table 3-1

| Aligned plate | Agent concentration | | | |
|---|---|---|---|---|
| No. | dose0 | dose1 | dose2 | dose3 |
| plate 1 | - | - | - | - |
| plate 2 | - | - | - | - |
| plate 3 | - | - | - | - |
| plate 4 | - | - | - | - |
| plate 5 | - | - | - | - |
| plate 6 | - | Arrhythmia | Arrhythmia | Arrhythmia |

[Table 3-2]

Table 3-2

| Planar plate | Agent concentration | | | |
|---|---|---|---|---|
| No. | dose0 | dose1 | dose2 | dose3 |
| plate 1 | - | Arrhythmia | Arrhythmia | Arrhythmia |
| plate 2 | - | EAD | EAD | EAD |
| plate 3 | - | EAD | EAD | EAD |
| plate 4 | - | - | Arrhythmia | Arrhythmia |
| plate 5 | - | - | Arrhythmia | Arrhythmia |
| plate 6 | - | - | - | Arrhythmia |

Isoproterenol ($\beta$ receptor agonist)

Isoproterenol was applied at the dose of 1, 3, and 10 $\mu$M. BR and RV tended to increase in CM2 cultured in the aligned plate and CM2 cultured in the planar plate, and CV was remained at the same level in both CM2 cultured in the planar plate and CM2 cultured in the aligned plate. CRD (corrected with beating rate) was extended at low concentration

(Dose1, 1 $\mu$M), medium concentration (Dose2, 3 $\mu$M), and high concentration (Dose3, 10 $\mu$M) in the aligned plate and the planar plate. In the aligned plate, the change was similar to that of the solvent (DMSO), but in the planar plate, the change was largely different from that of the solvent (DMSO), which is believed due to the influence of the drug (Fig. 14(B)).

No arrhythmia or the like was observed at any concentration.

(9-2) Arrhythmia measurement by Ca-imaging

The measurement was performed with the following procedure.

To CM2 on day 6 of maintenance culture, a fluorescent Ca indicator (EarlyTox carditotoxicity kit, manufactured by Molecular Devices, LLC.) was added, and living cells were stained. First, the change in Ca fluorescence intensity associated with the contraction of cardiomyocytes was measured as Dose0 in CQ1 without adding drugs. Immediately after the measurement, each drug at the concentration of Dose1 was added to the same well plate, and the change in Ca fluorescence intensity was measured after 15 minutes. For Dose2 and Dose3, the drug was cumulatively administered in the same manner, and the change in Ca fluorescence intensity was measured. The addition test of each drug was performed at n = 6.

The actions of the drugs were compared in CM2 seeded and cultured for 6 days in the planar plate and the aligned plate using data of 20% waveform width (Duration) when the peak of the waveform of Ca transient was set as 100%, interval between peaks (Interval) and peak height (Peak of Ca transient) using CQ1. The significant difference test was performed between the subject group in which DMSO was added and the drug addition group in CM2 cultured in the planar plate and CM2 cultured the aligned plate.

For the side effects seen from the waveform changes of Ca transient, early after depolarization (EAD) and delayed after depolarization (DAD), small regular waveforms (VT-like), and reaction (waveform) disappearance were recorded. The "-" in the table indicates the state in which no arrhythmia or the like has occurred. The degree of arrhythmia is mild for EAD, moderate for DAD, and severe for vt-like.

E-4031 (Ikr blocker)

Duration of Ca transient was slightly longer (significantly) in CM2 cultured in the aligned plate than CM2 cultured in the planar at 0 $\mu$M, slightly longer in CM2 cultured in the planar plate at 0.1 $\mu$M, significantly longer in CM2 cultured in the planar plate than CM2 cultured in the aligned plate at 0.3 $\mu$M, and was not significantly but remarkably extended in CM2 cultured in the planar plate at 1 $\mu$M.

From these results, it can be said that the effect of IKr blockade by addition of E-4031 is more easily appeared in CM2 cultured in the planar plate and less in CM2 cultured in the aligned plate (Fig. 15-1).

The incidence of moderate to severe arrhythmia (DAD, VT-like) in CM2 cultured in the aligned plate was 0/6 at low concentration (Dose1, 0.1 $\mu$M), 1/6 at medium concentration (Dose2, 0.3 $\mu$M), and 4/6 at high concentration (Dose3, 1 $\mu$M). The incidence of moderate to severe arrhythmia in CM2 cultured in the planar plate was 3/6 at low concentration (Dose1, 0.1 $\mu$M), 6/6 at medium concentration (Dose2, 0.3 $\mu$M), and 6/6 at high concentration (Dose3, 1 $\mu$M), and thus, the incidence of moderate to severe arrhythmia tended to be low in CM2 cultured in the aligned plate and high in CM2 cultured in the planar plate (Table 4-1, Table 4-2).

[Table 4-1]

Table 4-1

| Aligned plate | Agent concentration | | | |
|---|---|---|---|---|
| No. | dose0 | dose1 | dose2 | dose3 |
| plate 1 | - | EAD | EAD | VT-like |
| plate 2 | - | - | EAD | VT-like |
| plate 3 | - | - | DAD | VT-like |
| plate 4 | - | - | - | VT-like |
| plate 5 | - | - | EAD | EAD |
| plate 6 | - | - | - | EAD |

[Table 4-2]

Table 4-2

| Planar plate | Agent concentration | | | |
|---|---|---|---|---|
| No. | dose0 | dose1 | dose2 | dose3 |
| plate 1 | - | - | DAD | VT-like |
| plate 2 | - | DAD | DAD | VT-like |
| plate 3 | - | DAD | DAD | VT-like |
| plate 4 | - | - | DAD | DAD |
| plate 5 | - | - | DAD | DAD |
| plate 6 | - | DAD | DAD | VT-like |

Qinidine (INa blocker, Ikr blocker)

The results of investigating quinidine at 3, 10, and 30 $\mu$M for the similar effect are shown in Fig. 15-2 below. At the medium concentration (Dose2, 10 $\mu$M), a longer extension of duration and interval, and a significantly weaker decrease in peak height were observed in CM2 cultured in the planar plate compared to CM2 cultured in the aligned plate. At the high concentration (Dose3, 30 $\mu$M), the contraction force was greatly reduced and the waveform was changed to a higher frequency waveform, thus the result of contraction force at the high concentration is not shown in the figure.
The incidence of moderate to severe arrhythmia (DAD, VT-like) in CM2 cultured in the aligned plate was 0/6 at low concentration (Dose1, 3 $\mu$M), 0/6 at medium concentration (Dose2, 10 $\mu$M), and 6/6 at high concentration (Dose3, 30 $\mu$M). The incidence of moderate to severe arrhythmia in CM2 cultured in the planar plate was 0/6 at low concentration (Dose1, 3 $\mu$M), 0/6 at medium concentration (Dose2, 10 $\mu$M), and 4/6 at high concentration (Dose3, 30 $\mu$M), and thus, the incidence of moderate to severe arrhythmia tended to be slightly higher in the aligned plate (Table 5-1, Table 5-2). The incidence of arrhythmia tended to be slightly higher in the aligned plate, but the changes in duration and interval were smaller in CM2 cultured in the aligned plate, thus it is thought that the effect of the drug is hardly to appear.

[Table 5-1]

Table 5-1

| Aligned plate | Agent concentration | | | |
|---|---|---|---|---|
| No. | dose0 | dose1 | dose2 | dose3 |
| plate 1 | - | EAD | EAD | VT-like |
| plate 2 | - | - | - | VT-like |
| plate 3 | - | EAD | EAD | VT-like |
| plate 4 | - | - | - | VT-like |
| plate 5 | - | - | - | VT-like |
| plate 6 | - | EAD | EAD | VT-like |

[Table 5-2]

Table 5-2

| Planar plate | Agent concentration | | | |
|---|---|---|---|---|
| No. | dose0 | dose1 | dose2 | dose3 |
| plate 1 | - | - | - | VT-like |
| plate 2 | - | - | - | VT-like |
| plate 3 | - | - | EAD | VT-like |
| plate 4 | - | - | EAD | VT-like |
| plate 5 | - | - | EAD | EAD |
| plate 6 | - | - | EAD | EAD |

Cisapride (Ikr blocker, 5-HT4 receptor agonist)

The effect of cisapride at 0.01, 0.03, and 0.1 $\mu$M was investigated. Both duration and interval were extended in a dose-dependent manner (Fig. 15-3). The difference between the value shown at Dose0 and the value shown at Dose3 was smaller in CM2 cultured in the aligned plate than in CM2 cultured in the planar plate. It is thought that CM2 cultured in the aligned plate is more resistant to cisapride than CM2 cultured in the planar plate.

The incidence of arrhythmia was 1/6 at low concentration (Dose1, 0.01 $\mu$M) to high concentration (Dose3, 0.1 $\mu$M) in the aligned plate. No arrhythmia occurred in the planar plate (Table 6-1, Table 6-2).

The incidence of arrhythmia tended to be slightly higher in the aligned plate, but the change in duration and interval was smaller in CM2 cultured in the aligned plate, thus it is thought that the effect of the drug is hardly to appear.

[Table 6-1]

Table 6-1

| Aligned plate | Agent concentration | | | |
|---|---|---|---|---|
| No. | dose0 | dose1 | dose2 | dose3 |
| plate 1 | - | - | - | - |
| plate 2 | - | - | - | - |
| plate 3 | - | - | - | - |
| plate 4 | - | - | - | - |
| plate 5 | EAD | EAD | EAD | EAD |
| plate 6 | - | - | - | - |

[Table 6-2]

Table 6-2

| Planar plate | Agent concentration | | | |
|---|---|---|---|---|
| No. | dose0 | dose1 | dose2 | dose3 |
| plate 1 | - | - | - | - |
| plate 2 | - | - | - | - |
| plate 3 | - | - | - | - |
| plate 4 | - | - | - | - |
| plate 5 | - | - | - | - |

(continued)

| Planar plate | Agent concentration | | | |
|---|---|---|---|---|
| No. | dose0 | dose1 | dose2 | dose3 |
| plate 6 | - | - | - | - |

Isoproterenol (β receptor agonist)

The effect of isoproterenol at 1, 3, and 10 μM was investigated. Reduction was observed in both duration and interval (Fig. 15-4). However, the reduction in duration in the planar plate was small. This effect of reduction in duration is a typical response seen in β receptor stimulation.

With isoproterenol, arrhythmia did not occur even at high concentration (Dose3, 10 μM). This tendency was the same in CM2 cultured in the aligned plate and CM2 cultured in the planar plate.

DMSO (solvent)

With the solvent, duration was slightly extended. The action did not have a significant effect on the drug effect (Fig. 15-5).

When the action of the IKr blocker E-4031 whose most typical action is extension of action potential duration and the similar effects are focused on, the action of the extension of duration by E-4031 at high dose is less likely to be observed in CM2 cultured in the aligned plate than in CM2 cultured in the planar plate. Thus, it is thought that side effects are more likely to appear in CM2 cultured in the planar plate and less likely to appear in CM2 cultured in the aligned plate.

In most test systems, the duration of Ca transient before drug application was long in CM2 cultured in the aligned plate and short in CM2 cultured in the planar plate, and it is suggested that this is the same for the action potential duration. This extension of duration is generally correlated with the maturation of cardiomyocytes, and it is clear that functional changes (promotion of differentiation in the aligned plate) are occurring even on day 6 in CM2 cultured in the aligned plate and CM2 cultured in the planar plate.

(10) Drug safety test-2

The capacity-dependent action of two drugs that are often used to induce side effects on myocardium was investigated in a more mature state with iCell cardiomyocytes CM2 cultured for 15 days.

In the procedures described above, CM2 was seeded at a concentration of $6 \times 10^4$ cells/well in the aligned plate (96 well plate, ND Cell Aligner) and the planar plate (96 well plate). A maintenance culture was performed in a 37°C $CO_2$ incubator, and the drug was administered on day 15 with setting the day of seeding as day 0.

The drug used was isoproterenol and milrinone. The drug was dissolved in DMSO and administered. First, the measurement as dose0 was performed without adding drugs. Immediately after the measurement, the drug dissolved in DMSO was added to each well so as to be the concentration of each dose, and the measurement was performed after a certain time. The type, mechanism of action, and concentration of the drugs are shown in the table below.

[Table 7]

Table 7

| Agent | Mechanism of action | drug concentration (μM) | | |
|---|---|---|---|---|
| | | dose1 | Dose2 | Dose3 |
| Isoproterenol | β receptor agonist | 0.03 | 0.3 | 3 |
| Milrinone | Phosphodiesterase 3 inhibitor | 30 | 100 | 300 |

For the cell responsiveness after drug administration, a motion test method using a live cell imaging device (SI8000, manufactured by Sony Corporation) was performed to investigate the effect on the beating rate, the contractile velocity, and the relaxation velocity.

(10-1) Measurement by motion test method using live cell imaging device

The measurement was performed with the following procedure.

CM2 on day 15 of maintenance culture was used, and video data was first obtained as dose0 using SI8000 (manufactured by Sony Corporation) without adding drugs. Immediately after the measurement, each drug at the concentration of each dose was added to the same well plate, and video data was obtained after 120 minutes for isoproterenol and after 15 minutes for milrinone. The addition test of each drug was performed at n = 5.

The beating rate (BR), the contractile velocity (CV), and the relaxation velocity (RV) were analyzed from the video data of SI8000, and the effects of the drugs on the motion of the cells were compared.

The measurement data was corrected with the average value of dose0 of no adding drugs (each measurement value/measurement value of dose0), and the relative value with setting the measurement value of dose0 as 1 was determined.

For the significant difference test, a T test was performed by comparing the results of the planar plate and the aligned plate at each drug addition concentration to the results of dose0. In addition, a T test was performed between the planar plate and the aligned plate in the drug addition group. For the comparison group with a P value of 0.01 or less, the graph was marked.

Isoproterenol ($\beta$ receptor agonist)

The addition of isoproterenol increased the beating rate (BR) and the relaxation velocity (RV) in both CM2 cultured in the planar plate and CM2 cultured in the aligned plate. The relaxation velocity was increased at dose2 and dose3 in the aligned plate compared to those in the planar plate.

The contractile velocity (CV) was increased in CM2 cultured in the aligned plate, but tended to reduce in CM2 cultured in the planar plates. CV was increased predominantly at dose2 in the aligned plate compared to before the drug addition, and increased significantly at dose2 and dose3 in the aligned plate compared to those in the planar plate (Fig. 16-1).

Milrinone (phosphodiesterase 3 inhibitor)

The addition of milrinone increased the beating rate (BR), the contractile velocity (CV), and the relaxation velocity (RV) in CM2 cultured in the aligned plate. In CM2 cultured in the planar plate, the drug administration did not change the beating rate, but tended to reduce the contractile velocity and the relaxation velocity.

The beating rate (BR) of CM2 cultured in the aligned plate was predominantly increased in all doses compared to before the drug addition. The relaxation velocity (RV) was predominantly increased at dose3 compared to before the drug addition. In addition, RV was significantly increased compared to that in the planar plate. The contractile velocity (CV) was significantly increased at dose3 compared to that in the planar plate (Fig. 16-2).

DMSO (solvent)

No action having a significant effect on the drug effect was observed with the solvent.

As shown in the above (10), from the results of investigating the capacity-dependent action of two drugs that are often used to induce side effects on myocardium in a more mature state with iCell cardiomyocytes CM2 cultured for 15 days, a significant increase of the contractile velocity was observed by using the aligned plate, and it was thus shown that the use of the aligned plate enables detection of the positive inotropic action which had been considered difficult to verify about the effect in iCell cardiomyocytes.

(11) Drug safety test-3 (anti-cancer drug)

The capacity-dependent action of anti-cancer drug that induce side effects on myocardium (toxic myocarditis, hypersensitive myocarditis) was investigated using iCell cardiomyocyte CM2 cultured for 15 days.

In the same procedure as in the above (10), CM2 was seeded at a concentration of $6 \times 10^4$ cells/well in the aligned plate (96 well plate, ND Cell Aligner) and the planar plate (96 well plate). A maintenance culture was performed in a 37°C $CO_2$ incubator, and the drug was administered on day 15 with setting the day of seeding as day 0.

The drug used was doxorubicin (an anthracycline-based anti-cancer drug). The drug was dissolved in DMSO and administered. First, as a pre-measurement, a measurement was performed without adding the drug. Immediately after the measurement, the drug dissolved in DMSO was added to each well so that the concentration was 3 $\mu$M, and the measurement was performed after 24 hours and 48 hours. The measurement was performed in the same manner as the (10-1) measurement by motion test method using live cell imaging device described above.

For the measurement data, the measurement value at the concentration of 3 $\mu$M was corrected with the average value of the pre-measurement without adding the drug (measurement value at 3 $\mu$M/measurement value of pre-measurement), and the change rate with setting the measurement value of the pre-measurement as 1 was determined.

For the significant difference test, a T test was performed by comparing the results of the drug addition group in the planar plate and the aligned plate at each time with those of the DMSO addition group at each time. In addition, a T test was performed between the planar plate and the aligned plate in the drug addition group. For the comparison group with a P value of 0.01 or less, the graph was marked.

DMSO used as the solvent was added so that the final concentration was 0.01%. The effect of DMSO was confirmed by measuring 0.01% DMSO addition group after 24 hours and 48 hours. The measurement data were processed in the same manner as the drug addition group.

Doxorubicin (anti-cancer drug)

The addition of doxorubicin increased the beating rate (BR) in both CM2 cultured in the planar plate and CM2 cultured in the aligned plate. The measurement result after 48 hours was more increased than the measurement result after 24 hours, and the beating rate increased over time. In particular, 3 $\mu$M addition of doxorubicin significantly increased the beating rate in CM2 cultured in the aligned plate compared to that in the planar plate. The addition of DMSO tended to reduce the beating rate in both the planar plate and the aligned plate (Fig. 17).

The increase in CV (contractile velocity) was observed in CM2 cultured in the aligned plate. In the measurement after 24 hours, CV was significantly increased by 3 $\mu$M addition compared to the DMSO addition group, and in the measurements after 24 and 48 hours, CV was significantly increased in CM2 cultured in the aligned plate compared to that in the planar plate. It was observed that the contractile velocity tended to reduce in the planar plate by adding DMSO, but did not change in the aligned plate compared to before the drug addition (Fig. 17).

The relaxation velocity (RV) was increased by the addition of doxorubicin in both CM2 cultured in the planar plate and CM2 cultured in the aligned plate. In the measurement after 48 hours, RV was significantly increased in CM2 cultured in the aligned plate compared to that in the planar plate. RV tended to increase by the addition of DMSO in the aligned plate, but did not change in the planar plate compared to before the drug addition (Fig. 17).

As shown in (11) above, from the results of investigating the capacity-dependent action using the anti-cancer drug doxorubicin, a significant increase in the beating rate (BR), the contractile velocity (CV) and the relaxation velocity (RV) was observed by using the aligned plate, and it was shown that the use of the aligned plate enables detection of toxicity to the myocardium.

(12) Measurement of oxygen consumption rate

In the above procedure, CM2 was seeded at a concentration of $6 \times 10^4$ cells/well in the aligned plate (96 well plate, ND Cell Aligner) and the planar plate (96 well plate). A maintenance culture was performed in a 37°C $CO_2$ incubator, and the oxygen consumption rate was measured on day 27 with setting the day of seeding as day 0.

The measurement was performed using an oxygen consumption rate plate assay kit (manufactured by DOJINDO LABORATORIES, E297).

To the well plate in which CM2 was cultured, an oxygen probe diluted with medium was added and allowed to stand for 30 minutes in a plate reader incubated at 37°C in advance. Next, a sample solution containing a $\beta$-oxidation inhibitor Etomoxir that inhibits a fatty acid metabolism enzyme at the final concentration 50 $\mu$M was added, and meneral oil was immediately added dropwise to block the supply of oxygen.

After allowing the well plate to stand for 5 minutes in a plate reader incubated at 37°C, the fluorescence intensity was measured over time. The fluorescent plate reader was measured under conditions of an excitation wavelength of 495 nm/fluorescence wavelength of 650 nm, and the data acquisition was performed for at least 168 minutes every 10 minutes.

The measurement was performed with 6 wells without addition of Etomoxir as control and 3 wells with addition of 50 $\mu$M Etomoxir.

The oxygen concentration (pmol) was calculated with the calculation sheet attached to the oxygen consumption rate plate assay kit, and the oxygen consumption rate (pmol/min) was calculated from the oxygen concentration reduction amount for 60 minutes of 10 to 70 minutes after the start of the measurement.

As a result of the measurement, in the planar plate, OCR without addition of Etomoxir was 79.1 pmol/min and OCR with addition of Etomoxir was 77.8 pmol/min, and the addition of Etomoxir led to OCR of 98.3% compared to OCR before addition.

On the other hand, in the aligned plate, OCR without addition of Etomoxir was 63.6 pmol/min and OCR with addition of Etomoxir was 7.0 pmol/min, and the addition of Etomoxir reduced OCR to 11.0% compared to OCR before addition.

The results are shown in Fig. 16. In the longitudinal axis in Fig. 16, OCR without adding Etomoxir is set to 1.0.

From the results described above, it is thought that the cultured cell sheet of the present embodiment has advanced in maturation compared to conventional cultured cell sheets, and as a result, the use of the cultured cell sheet of the present embodiment enables evaluation of a compound or drug to be evaluated in the state more closely to in vivo.

**Claims**

1. A cultured cell sheet comprising cardiomyocytes, wherein

    the cardiomyocytes are arranged with alignment, and
    a degree of alignment determined according to the following formula (1) is 23% or more:

    Degree of alignment (%) = (number of rod-shaped structures included within $\pm15°$ of mode)/(total number of rod-shaped structures) $\times$ 100 $\quad$ (1)

    wherein in an image obtained by microscopy of the cultured cell sheet immunostained with an anti-$\alpha$-actinin antibody, where a screen horizontal direction is regarded as 0°, the degree of alignment is a frequency of rod-shaped structures included within $\pm15°$ of a mode of angles which are measured in a longitudinal direction of the rod-shaped structures detected with an $\alpha$-actinin antibody within a measurement range of 71.6 $\mu$m $\times$ 71.6 $\mu$m.

2. The cultured cell sheet according to claim 1, wherein in live cell imaging, number of vectors (degree of alignment) showing an angle of $\pm5°$ from an angle of a mode of motion vectors is 12% or more.

3. The cultured cell sheet according to claim 1, wherein the cardiomyocytes are stem cell-derived cardiomyocytes.

4. The cultured cell sheet according to claim 1, which satisfies at least one of the following requirements A1 and A2:

    Requirement A1: a ratio of an expression level of a myosin light chain 3 (MYL3) gene to an expression level of an actin beta (ACTB) gene, MYL3/ACTB, is 12.0 or more; and
    Requirement A2: a ratio of an expression level of a myosin heavy chain 7 (MYH7) gene to an expression level of a myosin heavy chain 6 (MYH6) gene, MYH7/MYH6, is 6.0 or more.

5. The cultured cell sheet according to claim 1, which satisfies at least one of the following requirements B1 and B2:

    Requirement B1: a ratio of an expression level of creatine kinase M-type (CKM) gene to an expression level of actin beta (ACTB) gene, CKM/ACTB, is 1.80 or more; and
    Requirement B2: a ratio of an expression level of a lactate dehydrogenase A subunit (LDHA) gene to an expression level of ACTB gene, LDHA/ACTB, is 0.80 or more.

6. The cultured cell sheet according to claim 1, wherein an area of mitochondria per cell is 200 $\mu$m$^2$ or more.

7. The cultured cell sheet according to claim 1, which satisfies the following formula (2):

$$1.0 \leq \mathrm{CV/RV} \leq 2.8 \quad (2)$$

    wherein CV (m/sec) and RV (m/sec) denote contractile velocity and relaxation velocity, respectively, detected in live cell imaging.

8. The cultured cell sheet according to claim 1, which satisfies the following formula (3):

$$\mathrm{Duration/(Interval)}^{1/2} \geq 0.68 \quad (3)$$

    wherein in calcium imaging analysis, when a waveform peak height of a calcium transient is 100%, Duration (sec) denotes a waveform width at a height of 20%, and Interval (sec) denotes an interval between peaks.

9. The cultured cell sheet according to claim 1, wherein a cell constituting the cultured cell sheet has an action potential duration at 80% repolarization of 600 msec or more and a maximum diastolic potential of -60 mV or less in a patch-clamp test in a solution at 25°C.

10. The cultured cell sheet according to claim 1, which is for regenerative medicine.

11. A method for producing the cultured cell sheet according to any one of claims 1 to 10, comprising:

culturing cardiomyocytes by using a cell sheet-forming member comprising a surface for forming a cultured cell sheet, wherein
the surface comprises a plurality of flat portions and a plurality of recession and protrusion portions;
each of the flat portions has a shape extending in a first direction, and a plurality of the flat portions are arrayed in a second direction intersecting the first direction over an entirety of the surface;
each of the recession and protrusion portions comprises a plurality of stepped structures filling a gap between the flat portions adjacent to each other, and a pitch of the stepped structures is 100 nm or more and 10 $\mu$m or less;
the stepped structures each comprise a protrusion portion;
the recession and protrusion portions each comprise a plurality of the protrusion portions in a bottom surface of a recession portion sandwiched between the flat portions adjacent to each other; and
a difference between a height of a distal end surface of each of the recession and protrusion portions and a height of each of the flat portions is 0.5 $\mu$m or less in a thickness direction of the cell sheet-forming member.

12. A method for evaluating a compound or drug, comprising allowing a compound or drug to be evaluated to act on the cultured cell sheet according to any one of claims 1 to 10.

13. The method for evaluating a compound or drug according to claim 12, comprising evaluating at least one change selected from the group consisting of a change in a physiological property and a change in motor function of the cultured cell sheet.

14. The method for evaluating a compound or drug according to claim 12, wherein the compound or drug to be evaluated is at least one existing compound selected from the group consisting of an INa blocker, an Ikr blocker, an Iks blocker, an Ica blocker, a 5-HT4 receptor agonist, an alpha-receptor blocker, a beta-receptor blocker, an alpha-receptor agonist, a beta-receptor agonist, a toxic deleterious substance, an anti-cancer drug, and another bioactive substance, or a candidate compound thereof.

15. A method for evaluating quality of a cultured cell sheet comprising cardiomyocytes, comprising:
evaluating any one of the following (i) to (viii):

(i) a degree of alignment determined according to the following formula (1) is 23% or more:

$$\text{Degree of alignment (\%) = (number of rod-shaped structures included within } \pm15° \text{ of mode)/(total number of rod-shaped structures)} \times 100 \quad (1)$$

wherein in an image obtained by microscopy of the cultured cell sheet immunostained with an anti-$\alpha$-actinin antibody, where a screen horizontal direction is regarded as 0°, the degree of alignment is a frequency of rod-shaped structures included within $\pm15°$ of a mode of angles which are measured in a longitudinal direction of the rod-shaped structures detected with an $\alpha$-actinin antibody within a measurement range of 71.6 $\mu$m $\times$ 71.6 $\mu$m,
(ii) the number of vectors (degree of alignment) showing an angle of $\pm5°$ from an angle of a mode of motion vectors is 12% or more in live cell imaging,
(iii) the cultured cell sheet satisfies at least one of the following requirements A1 and A2:

Requirement A1: a ratio of an expression level of myosin light chain 3 (MYL3) gene to an expression level of actin beta (ACTB) gene, MYL3/ACTB, is 12.0 or more; and
Requirement A2: a ratio of an expression level of a myosin heavy chain 7 (MYH7) gene to an expression level of a myosin heavy chain 6 (MYH6) gene, MYH7/MYH6, is 6.0 or more,

(iv) the cultured cell sheet satisfies at least one of the following requirements B1 and B2:

Requirement B1: a ratio of an expression level of creatine kinase M-type (CKM) gene to an expression level of actin beta (ACTB) gene, CKM/ACTB, is 1.80 or more; and

Requirement B2: a ratio of an expression level of a lactate dehydrogenase A subunit (LDHA) gene to an expression level of ACTB gene, LDHA/ACTB, is 0.80 or more,

(v) an area of mitochondria per cell is 200 $\mu m^2$ or more,
(vi) the cultured cell sheet satisfies the following formula (2):

$$1.0 \le CV/RV \le 2.8 \quad (2)$$

wherein CV (m/sec) and RV (m/sec) denote contractile velocity and relaxation velocity, respectively, detected in live cell imaging.
(vii) the cultured cell sheet satisfies the following formula (3):

$$Duration/(Interval)^{1/2} \ge 0.68 \quad (3)$$

wherein in calcium imaging analysis, when a waveform peak height of a calcium transient is 100%, Duration (sec) denotes a waveform width at a height of 20%, and Interval (sec) denotes an interval between peaks, and
(viii) a cell constituting the cultured cell sheet has an action potential duration at 80% repolarization of 600 msec or more and a maximum diastolic potential of -60 mV or less in a patch-clamp test in a solution at 25°C.

[Fig. 1]

(a)

100
120
111
110

(b)

141 140
130
141 140

(c)

130 140 130 140 130
141 141

(d)

130 140 130 140 130
141 141

142 142 142 142

[Fig. 2]

[Fig. 3]

[Fig. 4]

[Fig. 5]

[Fig. 6]

MYH7/MYH6

Number of days in culture (day)

[Fig. 7-1]

(A) CKM

(B) COX6A2

(C) CKMT2

(D) LDHA

[Fig. 7-2]

(A)

CKM/ACTB

(B)

COX6A2/ACTB

(C)

CKMT2/ACTB

(D)

LDHA/ACTB

[Fig. 7-3]

( A )

CACNA2D1

( B )

KCNJ2

( C )

KCNE1

( D )

SCN5A

[Fig. 7-4]

(A) CACNA2D1/ATP1A1

(B) KCNJ2/ATP1A1

(C) KCNE1/ATP1A1

(D) SCN5A/ATP1A1

[Fig. 7-5]

(A) LPL

(B) ACAT1

(C) HADHA

(D) HADHB

[Fig. 7-6]

(A) LPL/ACTB

(B) ACAT1/ACTB

(C) HADHA/ACTB

(D) HADHB/ACTB

[Fig. 7-7]

[Fig. 7-8]

(A) PPARGC1A/HIF1A — Number of days in culture (day)

(B) ESRRA/HIF1A — Number of days in culture (day)

(C) VEGFA/HIF1A — Number of days in culture (day)

(D) APLN/HIF1A — Number of days in culture (day)

[Fig. 7-9]

(A)

FABP3

(B)

ESM1

(C)

EMCN

(D)

BCL2

[Fig. 7-10]

(A) FABP3/HIF1A

(B) ESM1/HIF1A

(C) EMCN/HIF1A

(D) BCL2/HIF1A

[Fig. 8]

(A)

Mitochondrial area day 9

(B)

Mitochondrial activity day 9

[Fig. 9]

(A) Aligned plate

(B) Planar plate

20μm

20μm

[Fig. 10]

(A) Aligned plate

(B) Planar plate

[Fig. 11-1]

[Fig. 11-2]

## CV/RV

[Fig. 12]

(A)

(B)

[Fig. 13]

## Aligned

Maximum diastolic potential: -68.6 mV

Action potential duration at 80% repolarization: 791 msec

## Planar

Maximum diastolic potential: -57.9 mV

Action potential duration at 80% repolarization: 478 msec

[Fig. 14]

(A) E-4031

(B) Isoproterenol

[Fig. 15-1]

(A)
Duration (E4031)   *p<0.05

(B)
Interval (E4031)   *p<0.05

[Fig. 15-2]

(A)
Duration (Quinidine)   *p<0.05

(B)
Interval (Quinidine)

[Fig. 15-3]

(A)
Duration (Cisapride)   *p<0.05

(B)
Interval (Cisapride)   *p<0.05

[Fig. 15-4]

(A)
Duration (Isoproterenol)   *p<0.05

(B)
Interval (Isoproterenol)

EP 4 553 148 A1

[Fig. 15-5]

(A) Duration (DMSO)

(B) Interval (DMSO)

[Fig. 16-1]

(A) BR *p<0.01

(B) CV *p<0.01

(C) RV *p<0.01

[Fig. 16-2]

(A)

(B)

(C)

[Fig. 17]

[Fig. 18]

<table>
<tr><td colspan="2">**INTERNATIONAL SEARCH REPORT**</td><td>International application No.<br>**PCT/JP2023/024866**</td></tr>
</table>

| | |
|---|---|
| **A.** | **CLASSIFICATION OF SUBJECT MATTER** |

*C12N 5/071*(2010.01)i; *C12N 5/077*(2010.01)i; *C12Q 1/02*(2006.01)i; *C12Q 1/06*(2006.01)i; *C12M 1/00*(2006.01)i
FI: C12N5/077; C12M1/00 A; C12N5/071; C12Q1/02; C12Q1/06

According to International Patent Classification (IPC) or to both national classification and IPC

| | |
|---|---|
| **B.** | **FIELDS SEARCHED** |

Minimum documentation searched (classification system followed by classification symbols)

C12N5/071; C12N5/077; C12Q1/02; C12Q1/06; C12M1/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| X | US 2015/0125952 A1 (UNIVERSITY OF WASHINGTON THROUGHITS CENTER FOR COMMERCIALIZATION) 07 May 2015 (2015-05-07)<br>claims, paragraphs [0015], [0020], [0074], [0075], [0082], [0084]-[0086], [0098], [0672], [0694], examples, figures | 1-10, 12-14 (all), 15 (part of (i)) |
| Y | claims, paragraphs [0015], [0020], [0074], [0075], [0082], [0084]-[0086], [0098], [0672], [0694], examples, figures | 11 (all) |
| Y | claims, paragraphs [0015], [0020], [0074], [0075], [0082], [0084]-[0086], [0098], [0672], [0694], examples, figures | 15 (part of (ii)) |
| Y | TOKUNO, Hisako et al. Induction of Anisotropic Orientation and Enhancement of Gene Functional Expression of Human Pluripotent Stem Cell-Derived Cardiomyocytes Cultured on Nanofabricated Substrates Consisting of Micron Planar Lines and Nano Dot Structures. Research Square. 2021, pp. 1-19, [online], [retrieved on 24 July 2023], Retrieved from the Internet: <URL: https://assets.researchsquare.com/files/rs-727809/v1/12c913a1-435e-4974-ad20-628d1ba67df2.pdf?c=1631886684>, DOI:https://doi.org/10.21203/rs.3.rs-727809/v1 abstract, p. 4, table 1, p. 8, lines 7-9, fig. 1 | 11 (all) |
| Y | JP 2019-037220 A (OJI HOLDINGS CORPORATION) 14 March 2019 (2019-03-14)<br>claims, examples | 11 (all) |

☑ Further documents are listed in the continuation of Box C.　　☑ See patent family annex.

| | |
|---|---|
| * | Special categories of cited documents: |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **01 September 2023** | **12 September 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2023/024866**

**C.    DOCUMENTS CONSIDERED TO BE RELEVANT**

| Category* | Citation of document, with indication, where appropriate, of the relevant passages | Relevant to claim No. |
|---|---|---|
| Y | IZUMI-NAKASEKO, Hiroko et al. Optimizing the Direction and Order of the Motion Unveiled the Ability of Conventional Monolayers of Human Induced Pluripotent Stem Cell-Derived Cardiomyocytes to Show Frequency-Dependent Enhancement of Contraction and Relaxation Motion. Frontiers in Cell and Developmental Biology. 2020, vol. 8, Article 542562, pp. 1-14<br>abstract | 15 (part of (ii)) |
| Y | WO 2017/169397 A1 (SONY CORPORATION) 05 October 2017 (2017-10-05)<br>paragraph [0048] | 15 (part of (ii)) |
| Y<br><br>Y<br>Y | JP 2020-98199 A (UNIVERSITY OF MIYAZAKI) 25 June 2020 (2020-06-25)<br>claims, paragraph [0036]<br>claims, paragraph [0036]<br>claims, paragraph [0036] | 15 (part of (iii))<br><br>15 (part of (iv))<br>15 (part of (v)) |
| Y | WO 2021/112115 A1 (JICHI MEDICAL UNIVERSITY) 10 June 2021 (2021-06-10)<br>paragraph [0103] | 15 (part of (iii)) |
| Y<br><br>Y | WO 2019/078278 A1 (KYOTO UNIVERSITY) 25 April 2019 (2019-04-25)<br>paragraph [0042]<br>paragraph [0042] | 15 (part of (iii))<br><br>15 (part of (iv)) |
| Y | US 2020/0407687 A1 (UNIVERSITY HEALTH NETWORK) 31 December 2020 (2020-12-31)<br>paragraph [0075], fig. 3 | 15 (part of (iii)) |
| Y | DU, Wentao et al. Effects of target regulation of LDHA through the PDK1/Akt/mTOR pathway on myocardial apoptosis caused by ischemia/reperfusion injury in rats. Int. J. Clin. Exp. Med. 2017, vol. 10, no. 10, pp. 14194-14202<br>abstract | 15 (part of (iv)) |
| Y | JP 2018-27048 A (NIIGATA UNIVERSITY) 22 February 2018 (2018-02-22)<br>claims, examples | 15 (part of (v)) |
| X<br><br>Y | WO 2015/162810 A1 (OLYMPUS CORPORATION) 29 October 2015 (2015-10-29)<br>claims, paragraphs [0019], [0022]<br>claims, paragraphs [0019], [0022] | 15 (part of (vi)),<br>15 (part of (viii))<br>15 (part of (vii)) |
| Y | JP 2019-526255 A (THE UNIVERSITY OF QUEENSLAND) 19 September 2019 (2019-09-19)<br>paragraphs [0106], [0128], [0129] | 15 (part of (vii)) |
| A | WO 2020/158481 A1 (OJI HOLDINGS CORPORATION) 06 August 2020 (2020-08-06)<br>entire text | 11 (all) |
| A | WO 2020/158482 A1 (OJI HOLDINGS CORPORATION) 06 August 2020 (2020-08-06)<br>entire text | 11 (all) |

Form PCT/ISA/210 (second sheet) (January 2015)

**INTERNATIONAL SEARCH REPORT**

International application No.

**PCT/JP2023/024866**

| Box No. III      Observations where unity of invention is lacking (Continuation of item 3 of first sheet) |
| --- |

This International Searching Authority found multiple inventions in this international application, as follows:

The claims are classified into the following eight inventions.

(Invention 1) Claims 1-14 and part of claim 15 (pertaining to (i))
Claims 1-14 have the special technical feature of
a "culture cell sheet comprising cardiomyocytes, wherein
the cardiomyocytes are arranged to have orientation, and
the degree of orientation determined according to formula (1) indicated below is 23% or more:
(1): degree of orientation (%)=(number of rod-shaped structures included within ±15° of the mode)/(total number of rod-shaped structures)×100,
wherein the degree of orientation is determined, in an image obtained by microscopy of the culture cell sheet immunostained with an anti-α-actinin antibody, where the horizontal direction of the image is regarded as 0°, from the frequency of rod-shaped structures included within ±15° of the mode of angles in the longitudinal direction of rod-shaped structures detected with the α-actinin antibody within the measurement range (71.6 μm×71.6 μm)", and are thus classified as invention 1.
The invention understood by selecting (i) in claim 15 has a link to the invention of claim 1, and is therefore classified as invention 1.

(Invention 2) Part of claim 15 (pertaining to (ii))
The invention understood by selecting (ii) in claim 15 cannot be said to share a same or corresponding special technical feature with claim 1 classified as invention 1.
The invention understood by selecting (ii) in claim 15 is not dependent on claim 1 and is not substantially identical to or similarly closely related to any of the claims classified as invention 1.
Consequently, the invention understood by selecting (ii) in claim 15 cannot be classified as invention 1, and thus is classified as invention 2.

(Invention 3) Part of claim 15 (pertaining to (iii))
The invention understood by selecting (iii) in claim 15 cannot be classified as invention 1 similarly to invention 2.
The invention understood by selecting (iii) in claim 15 cannot be said to share a same or corresponding special technical feature with the invention understood by selecting (ii) in claim 15 classified as invention 2.
Further, the invention understood by selecting (iii) in claim 15 is not substantially identical to or similarly closely related to the invention understood by selecting (ii) in claim 15 classified as invention 2.
Consequently, the invention understood by selecting (iii) in claim 15 cannot be classified as invention 2, and thus is classified as invention 3.

(Invention 4)-(Invention 8) Parts of claims 15 (respectively pertaining to (iv)-(viii))
The inventions understood by selecting (iv)-(viii) in claim 15 are, similarly to invention 3, classified as inventions 4-8, respectively, in the order the inventions are described.

Form PCT/ISA/210 (continuation of first sheet) (January 2015)

| INTERNATIONAL SEARCH REPORT | International application No. |
|---|---|
| | **PCT/JP2023/024866** |

**Box No. III   Observations where unity of invention is lacking (Continuation of item 3 of first sheet)**

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

Form PCT/ISA/210 (continuation of first sheet) (January 2015)

**INTERNATIONAL SEARCH REPORT**
Information on patent family members

International application No.

**PCT/JP2023/024866**

| Patent document cited in search report | | | Publication date (day/month/year) | Patent family member(s) | | Publication date (day/month/year) |
|---|---|---|---|---|---|---|
| US | 2015/0125952 | A1 | 07 May 2015 | WO 2013/151755 A1 claims, paragraphs [015], [020], [074], [075], [082], [084]-[086], [098], [0672], [0694], examples, figures | | |
| JP | 2019-037220 | A | 14 March 2019 | US 2021/0130762 A1 claims, examples | | |
| | | | | EP 3674390 A1 | | |
| | | | | WO 2019/039485 A1 | | |
| WO | 2017/169397 | A1 | 05 October 2017 | JP 2017-175965 A paragraph [0048] | | |
| JP | 2020-98199 | A | 25 June 2020 | (Family: none) | | |
| WO | 2021/112115 | A1 | 10 June 2021 | (Family: none) | | |
| WO | 2019/078278 | A1 | 25 April 2019 | US 2020/0239847 A1 paragraph [0070] | | |
| | | | | EP 3699265 A1 | | |
| US | 2020/0407687 | A1 | 31 December 2020 | (Family: none) | | |
| JP | 2018-27048 | A | 22 February 2018 | (Family: none) | | |
| WO | 2015/162810 | A1 | 29 October 2015 | US 2017/0037483 A1 claims, paragraphs [0067], [0070] | | |
| JP | 2019-526255 | A | 19 September 2019 | WO 2018/035574 A1 page 34, lines 4-13, page 41, lines 3-28 | | |
| | | | | US 2019/0203179 A1 | | |
| WO | 2020/158481 | A1 | 06 August 2020 | (Family: none) | | |
| WO | 2020/158482 | A1 | 06 August 2020 | (Family: none) | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020022460 A

**Non-patent literature cited in the description**

- *Circ. Res.*, 10 April 2020, vol. 126 (8), 1086-1106
- *Nat Rev Cardiol.*, June 2020, vol. 17 (6), 341-359
- **OKAI et al.** Video-based assessment of drug-induced effects on contractile motion properties using human induced pluripotent stem cell-derived cardiomyocytes. *Journal of Pharmacological and Toxicological Methods*, September 2020, vol. 105, 106893
- **ARANY et al.** *HIF-independent regulation of VEGF and angiogenesis by the transcriptional coactivator PGC-1a*, 21 February 2008, vol. 451
- Methods in Molecular Biology, vol. 2320
- *Circulation*, 2013, vol. 127, 575-584
- *Bioinformatics*, 10 March 2020, vol. 36 (6), 1955-1956